# EUROPEAN PATENT SPECIFICATION

(11) **EP 4 371 976 B1**
(45) Date of publication and mention of the grant of the patent: **24.06.2026**
(21) Application number: 22383119.9
(22) Date of filing: 21.11.2022
(51) Int. Cl.: C07D 233/61, C07C 323/59, A61K 31/197, A61P 1/00

(54) **IONIZABLE LIPIDS AND LIPID NANOPARTICLES CONTAINING THEREOF**
IONISIERBARE LIPIDE UND DIESE ENTHALTENDE LIPIDNANOPARTIKEL
LIPIDES IONISABLES ET NANOPARTICULES LIPIDIQUES LES CONTENANT

(43) Date of publication of application: 22.05.2024
(73) Proprietor: Certest Biotec, S.L., 50840 San Mateo De Gallego (Zaragoza) (ES)
(72) Inventor: GIMÉNEZ WARREN, Javier, 50840 SAN MATEO DE GÁLLEGO (ES); HEREDERO GARCÍA, Juan, 50840 SAN MATEO DE GÁLLEGO (ES); MARTÍNEZ OLIVÁN, Juan Enrique, 50840 SAN MATEO DE GÁLLEGO (ES); PEÑA MORENO, Álvaro, 50840 SAN MATEO DE GÁLLEGO (ES); DE MIGUEL SAMANIEGO, Diego, 50840 SAN MATEO DE GÁLLEGO (ES); TORO CÓRDOVA, Alfonso, 50840 SAN MATEO DE GÁLLEGO (ES)
(74) Representative: ZBM Patents - Zea, Barlocci & Markvardsen

(56) References cited:
- WO-A1-2021/055835
- WO-A1-2022/204288
- FR-A1- 3 104 943
- HASSETT KIMBERLY J. ET AL: "Optimization of Lipid Nanoparticles for Intramuscular Administration of mRNA Vaccines", MOLECULAR THERAPY-NUCLEIC ACIDS, vol. 15, 15 April 2019 (2019-04-15), US, pages 1 - 11, XP055815007, ISSN: 2162-2531, Retrieved from the Internet <URL:https://www.cell.com/molecular-therapy-family/nucleic-acids/pdf/S2162-2531(19)30017-4.pdf> DOI: 10.1016/j.omtn.2019.01.013
- MOLLA MIJANUR R. ET AL: "One-Pot Parallel Synthesis of Lipid Library via Thiolactone Ring Opening and Screening for Gene Delivery", BIOCONJUGATE CHEMISTRY, vol. 29, no. 4, 20 March 2018 (2018-03-20), US, pages 992 - 999, XP093034759, ISSN: 1043-1802, Retrieved from the Internet <URL:https://pubs.acs.org/doi/pdf/10.1021/acs.bioconjchem.8b00007> DOI: 10.1021/acs.bioconjchem.8b00007
- CHURUSOVA SVETLANA G. ET AL: "Palladium(II) Pincer Complexes of Functionalized Amides with S-Modified Cysteine and Homocysteine Residues: Cytotoxic Activity and Different Aspects of Their Biological Effect on Living Cells", INORGANIC CHEMISTRY, vol. 60, no. 13, 5 July 2021 (2021-07-05), Easton , US, pages 9880 - 9898, XP093062600, ISSN: 0020-1669, DOI: 10.1021/acs.inorgchem.1c01138

## Description

### Technical Field

The present disclosure relates to ionizable lipids and to lipid nanoparticles (LNPs) comprising said ionizable lipids. These LNPs can be used as non-viral vectors for the delivery of active ingredients, including polynucleotides, to cells.

### Background Art

Treatment of different conditions such as infectious diseases is rapidly changing nowadays, especially since new polynucleotide delivery systems are being designed and their efficiency optimized to be a useful alternative to traditional therapies.

Gene delivery systems intended to contain encapsulated polynucleotides, such as RNA for systemic delivery, must be safe and non-toxic, be in the nanometer scale, provide protection against degradation to the polynucleotides, remain intact in the system for a given period of time in order to reach their target, and be easily degraded once they have released the load.

The use of RNA in gene therapy and vaccination is generally considered safer than the use of DNA, since RNA does not involve the risk of being stably integrated into the genome of the transfected cell. In addition, RNA degrades more easily *in vivo,* and therefore the risk of generating undesired anti-RNA antibodies that would reduce therapy efficacy and could produce very serious side effects is lower. Two of the main limitations of RNA-based therapies are the low transfection rates and the limited protein production efficiency. To compensate these, the dosage is increased in order to obtain the desirable therapeutic effects. Consequently, a higher dose also means elevated costs and some undesirable side effects observed.

One of the more potent intracellular delivery technologies for encapsulation and delivery of active molecules, such as for example genetic material (e.g. mRNA) in vaccines, is lipid nanoparticles (LNPs). Currently, LNPs used in commercial mRNA vaccines usually comprise four types of lipids in their composition, that is, a cationic or an ionizable lipid, a structural lipid which is a sterol such as cholesterol, a PEG-modified lipid, and a non-cationic lipid such as a phospholipid. In particular, ionizable lipids are crucial in LNPs.

Ionizable lipid properties have a great impact on the protection of the genetic material encapsulated inside the LNP, as they allow the structure and physicochemical properties of the genetic material to be maintained until the LNP reaches the target (e.g. a tissue, a cell) where the genetic material is to be released.

Ionizable lipids usually present the following general structure:
Lipophilic moiety----linking group----bridge---- Hydrophilic moiety

In the state of the art there are many examples where the hydrophilic moiety comprises an ionizable amine, a functional group where a change in pH influences its formal charge. Besides, it has also been disclosed that the presence of ester groups, easily hydrolysable by enzymes, facilitates the degradation of the ionizable lipid once the genetic material has been released into the tissue/cell of interest, what improves its biocompatibility and biodegradability. The proximity of ester groups to the hydrophilic moiety has also been described to have a great impact on the potency of the lipid.

As an example, Moderna uses the SM-102 lipid in its SARS-CoV-2 vaccine formulation (Spikevax) and Pfizer has licensed ALC-0315 from Acuitas for theirs (Comirnaty).

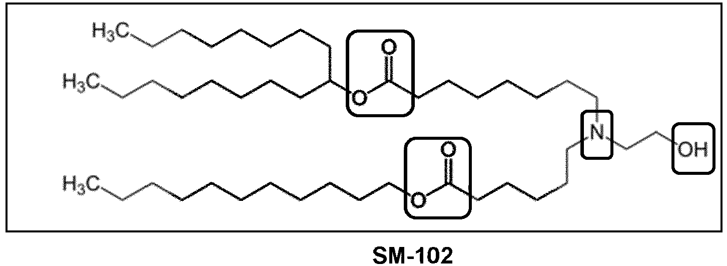

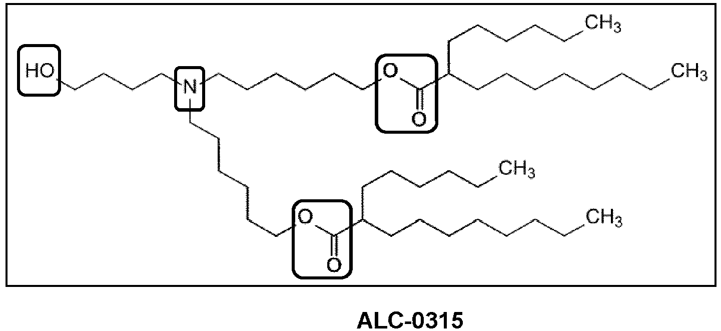

Molla MR, *et al.* (cf. Molla MR, et al. "One-Pot Parallel Synthesis of Lipid Library via Thiolactone Ring Opening and Screening for Gene Delivery". Bioconjug. Chem. 2018, vol. 29(4), pp. 992-999. doi: 10.1021/acs.bioconjchem.8b00007) discloses a combinatorial library of lipidoids with hydrophobic tails containing reducible disulfide groups and allowing to obtain stable liposomes. Hassett et al., "Optimization of Lipid Nanoparticles for Intramuscular Administration of mRNA Vaccines", Molecular Therapy Nucleic Acids, vol. 15, 15 April 2019, pages 1-11 discloses LNPs for use in vaccines.

Despite continuous improvements in such LNPs delivery systems, efficient and specific delivery of targeted agents is still problematic. Thus, there is still a need for alternative ionizable lipids and/or LNPs which overcome some of the disadvantages of the ones of the prior art, in particular, showing good stability, high transfection efficiency, and safety.

### Summary of Invention

The present inventors have developed a new ionizable lipid that allows obtaining lipid nanoparticles (LNPs) that can be effectively used as non-viral vectors for delivery of active ingredients, including polynucleotides, to cells. Particularly, LNPs comprising the ionizable lipid of the present disclosure show enhanced/improved transfection rates.

The present inventors found that ionizable lipids of Formula (I): 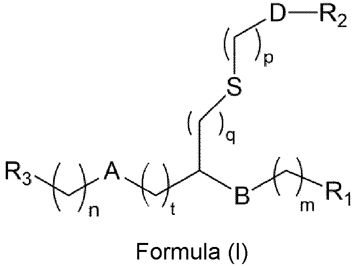 comprising at least one amide moiety, at least one ester moiety, at least one thioether moiety, at least one stereogenic center and a polar head R3, and two substituents R1 and R2, wherein R1, R2 and R3 are as defined in the present disclosure, are particularly useful in the preparation of LNPs encapsulating an active agent. Moreover, the present inventors have found that transfection rates in vivo of LNP prepared with the ionizable lipids of Formula (I) and comprising an active agent are unexpectedly high compared to other commercial or standard compositions known in the art.

Thus, from the data provided in the Examples, it is apparent that the ionizable lipids of the present disclosure provide a new tool to overcome some of the limitations of known LNPs.

The higher transfection efficiency of the LNPs of the present disclosure may allow reducing the therapeutic dose of polynucleotide, such as RNA, required in gene therapy and vaccination, which may help to reduce the associated secondary effects. At the same time, the LNPs of the present disclosure show good thermal and mechanical properties, and have demonstrated that their transfection efficiency *in vivo* remains even three months after being stored at 4 °C without further precautions, meaning a long shelf life under normal refrigeration conditions, and which could be extended at lower temperatures (e.g. at domestic freezer temperatures between -25 °C to -15°C or could be frozen at lower temperatures of up to -90 °C).

Therefore, a first aspect of the present disclosure relates to an ionizable lipid of formula (I): 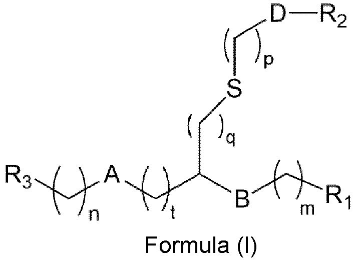
or a pharmaceutically acceptable salt thereof, or a stereoisomer of any one of them,
wherein
   A is 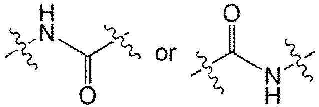
   B is 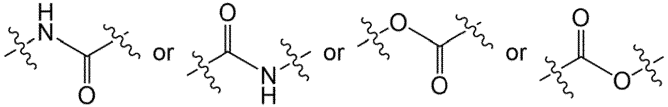
   D is 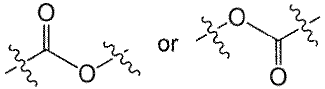
n, m and p are independently 0, 1, 2, 3, 4, 5, or 6;
q is selected from 1 and 2,
t is selected from 0 and 1,
and wherein q + t = 2;
R₁ and R₂ are independently a linear or branched C1-C30 alkyl, C2-C30 alkenyl, C2-C30 alkynyl, wherein R₁ and R₂ are optionally substituted with one or more substituents selected from the group consisting of -OH, -COOR₄, and -C(=O)S-R₄, wherein R₄ is C1-C6 alkyl, C2-C6 alkenyl. or C2-C6 alkynyl;
and wherein
R₃ is a heterocycle comprising at least one N atom, or alternatively R₃ is: 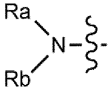
wherein Ra and Rb are independently linear or branched C1-C6 alkyl, optionally substituted with a hydroxyl group;
provided that the compounds methyl-S-(2-methoxy-2-oxoethyl)-N-(pyridin-2-ylcarbonyl)-homocysteinate 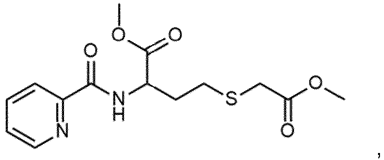 and
methyl N-[(4-chloropyridin-2-yl)carbonyl]-S-(2-methoxy-2oxoethyl)homocysteinate 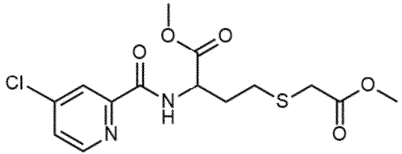 are excluded.

Another aspect of the invention relates to a LNP comprising an ionizable lipid of formula (I) as defined herein.

In another aspect, the LNP of the present disclosure can further comprise a pharmaceutically active agent and, as such, be formulated in a pharmaceutical with excipients and carriers.

Thus, another aspect of the present invention relates to a pharmaceutical composition comprising the LNP comprising a pharmaceutically active agent as defined herein and a pharmaceutically acceptable excipient or carrier.

The LNP comprising a pharmaceutically active agent or the pharmaceutical composition of the present disclosure may be used in medicinal applications.

Hence, another aspect of the invention relates to the LNP comprising a pharmaceutically active agent or the pharmaceutical composition of the present disclosure for use in medicine, particularly for use in a method for treating a disease or disorder in a subject in need thereof; or for use in a method of inducing an immune response in a subject, for use in a method for the therapeutic immunization of a subject, for use as a vaccine, or for use in gene therapy. The method comprises administering to the subject a therapeutically effective amount of the nanoparticle composition or of the pharmaceutical composition.

Another aspect of the invention relates to the use of the LNP as defined herein as an encapsulation agent for an active ingredient.

### Brief Description of Drawings

Figure 1, related to Example 5, depicts protein expression in mice administered with LNPs prepared with the following lipids of the invention: VC-LC-0163, VC-LC-0289, VC-LC-0374, VC-LC-0389, VC-LC-0553, VC-LC-0554, VC-LC-0353, VC-LC-0355, containing RNA as the active ingredient. The lipids tested and depicted comprise the same R3 substituent (polar head) in all cases but one, and different combinations of R2 and R3 substituents, showing good transfection results, as demonstrated by the total flux measurements for each lipid in the image and in Table 7(A-C).
Figure 2, related to comparative Example 6, depicts protein expression *in vivo* in mice injected with LNPs comprising VC-LC-0236 (2A) as the ionizable lipid in the LNP composition and VC-LC-0588 (2B) as the ionizable lipid in the LNP composition.
Figure 3, related to Example 7, depicts protein expression in mice injected with LNPs of Table 11, having different composition rates for ionizable lipid VC-LC-0431, DOPE, cholesterol and DMG-PEG2000.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more." Unless indicated otherwise, definite articles used herein, such as "the" also include the plural of the noun.

The term "and/or" means that any one of the options to which it relates are possible or the at least two options take place at the same time.

The term "moiety" refers to a specific segment or functional group of a molecule or compound.

As used herein, the term "subject" refers to any mammal, including both human and non-human mammals.

As used herein, the term "C1-C# alkyl" refers to saturated linear or branched hydrocarbon which contains from 1 to # carbon atoms, and which is optionally substituted. Non-limiting examples of alkyl groups include, without being limited to, methyl, ethyl, n-propyl, isopropyl, n-butyl, sec-butyl, tert-butyl, pentyl, neopentyl, n-hexyl, decyl, isodecyl, undecyl, dodecyl, tetradecyl, and hexadecyl.

The term "C2-C# alkenyl" refers to an unsaturated linear or branched hydrocarbon chain which comprises from 2 to # carbon atoms and at least one or more double bonds, and which is optionally substituted. Examples of alkenyl groups may include without limitation ethenyl (i.e. vinyl), allyl, propenyl, butenyl, pentenyl and hexenyl, dodecenyl, tetradecenyl and hexadecenyl.

The term "C2-C# alkynyl" refers to an unsaturated linear or branched hydrocarbon chain which comprises from 2 to # carbon atoms and at least one or more triple bonds, and which is optionally substituted. Examples of alkynyl groups include, without being limited to, ethynyl, prop-1-ynyl, prop-2-ynyl, 1- methylprop-2-ynyl, but-1-ynyl, but-2-ynyl, but-3-ynyl.

The term "heterocycle comprising at least one N atom" refers to an optionally monosubstituted or multi-substituted cyclic system including one or more rings, where at least one ring includes at least one nitrogen.

The term "optionally substituted" means that the number of substituents can be equal to or different from zero. Unless otherwise indicated, it is possible that optionally substituted groups are substituted with as many optional substituents as can be accommodated by replacing a hydrogen atom with a non-hydrogen substituent on any available carbon or nitrogen atom.

The term "polynucleotide" is interchangeably used with "nucleic acid" and refers to a polymer of nucleotides, either ribonucleotides or deoxyribonucleotides. A polynucleotide formed by ribonucleotides may be referred to as "RNA polynucleotide", "ribonucleic acid" or simply "RNA"; and a polynucleotide formed by deoxyribonucleotides may be referred to as "DNA polynucleotide", "deoxyribonucleic acid" or simply "DNA". The polynucleotide may be single- or double-stranded, optionally incorporating synthetic, non-natural, or altered nucleotides capable of incorporation into DNA or RNA. "Artificial polynucleotide" refers to a polynucleotide with a sequence that does not occur in nature or that has been altered by human intervention.

As used herein, the term "messenger RNA", abbreviated as "mRNA", refer to any RNA polynucleotide which encodes a polypeptide of interest and which is capable of being translated to produce the encoded polypeptide of interest *in vitro, in vivo,* or *ex vivo.* Typically, an mRNA is single stranded and comprises an ORF in its structure.

As used herein "open reading frame" or "ORF" refers to a sequence of several nucleotide triplets that encodes a polypeptide, that is, that can be translated into a polypeptide sequence.

As used herein, "DNA construct" refers to an artificial polynucleotide including a sequence of interest operatively linked to an expression promoter, said promoter controlling expression of the sequence of interest.

As used herein, "expression vector" refers to a vector used to introduce a specific nucleic acid, typically a DNA construct, into a target cell for expression of the nucleic acid by the cell. Examples of suitable expression promoters and expression vectors include those conventionally used in molecular biology and known to the skilled person.

The term "polypeptide" refers to any peptide or protein comprising two or more amino acids joined to each other by peptide bonds or modified peptide bonds (i.e., peptide isosteres). "Polypeptide" refers to both short chains, commonly referred as peptides, oligopeptides, or oligomers, and to longer chains generally referred to as proteins.

The expression "therapeutically effective amount" as used herein, refers to the amount of a compound that, when administered, is sufficient to prevent the development of, or alleviate to some extent, one or more of the symptoms of the disease to which is addressed. The particular dose of compound administered according to this invention will of course be determined by the particular circumstances surrounding the case, including the compound administered, the route of administration, the particular condition being treated, the particular circumstances of the individual subject to be treated, and similar considerations. The term "pharmaceutical" encompasses also the concept of "veterinary composition". Thus, they relate to compositions that are therapeutically effective when administered by any desired or applicable route to any animal, including humans.

According to the present invention, the term "antigen" is a compound that can be recognized by immunoglobulin receptors of B cells, or by the T-cell receptor when complexed with MHC. Preferably, an "antigen" is a polypeptide.

The term "nanoparticle" as used herein, refers to a particle with at least two dimensions at the nanometer scale, particularly with all three dimensions at the nanoscale, where the nanoscale is in the range of about 1 nm to about 500 nm. Particularly, when the nanoparticle is substantially rod-shaped with a substantially circular cross-section, such as a nanowire or a nanotube, the "nanoparticle" refers to a particle with at least two dimensions at the nanoscale, these two dimensions being the cross-section of the nanoparticle. As used herein, "size" or "mean size" in the context of nanoparticle compositions refers to the mean diameter of a nanoparticle composition. The term "lipid nanoparticle" as used herein, refers to a nanoparticle whose external envelope is totally or partially made of lipids.

As used herein, the "polydispersity index. (PDI)" is a ratio that describes the homogeneity of the particle size distribution of a system. A small value, e.g., less than 0.3, indicates a narrow particle size distribution.

As used herein, the term "zeta potential" is the electrokinetic potential of a lipid, e.g., in a particle composition. Z potential is also defined as the potential difference between the dispersion medium and the stationary layer of fluid attached to the dispersed particle. It is generally accepted as a quantification of the magnitude of the charge, and it is often the only available path for characterization of double-layer properties.

As used herein, "apparent pKa" refers to an experimentally determined value resulting from the average ratio of all the ionized to deionized groups in a nanoparticle. Apparent pKa is different to the intrinsic pKa of any individual molecule, and it is an important parameter for the performance of nanoparticles encapsulating RNAs. The apparent pKa of nanoparticles can be measured by different techniques known in the art. For example, acid-base titration and 2-(p-toluidino)-6-naphtalene sulfonic acid (TNS) fluorescent methods are widely used in the art. Nanoparticles with an optimum pKa carry negligible charges at physiological pH, which prevent nonspecific binding and toxicity in the body. The optimum pKa of nanoparticles plays an important role in the endosomal escape mechanism and in the release of RNAs in the cytosol to exert therapeutic effect.

### Ionizable lipids

As mentioned above, a first aspect of the invention refers to an ionizable lipid of formula (I): 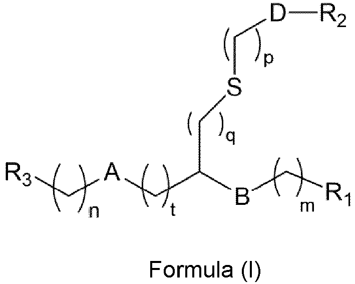 or a pharmaceutically acceptable salt thereof, or a stereoisomer of any one of them, wherein A, B, D, n, m, p, q, t, R₁, R₂, and R₃ are as defined herein; provided that the compounds methyl-S-(2-methoxy-2-oxoethyl)-N-(pyridin-2-ylcarbonyl)-homocysteinate and methyl N-[(4-chloropyridin-2-yl)carbonyl]-S-(2-methoxy-2oxoethyl)homocysteinate are excluded.

The term "pharmaceutically acceptable salts" used herein encompasses any salt formed from pharmaceutically acceptable non-toxic acids including inorganic or organic acids. There is no limitation regarding the salts, except that if used for therapeutic purposes, they must be pharmaceutically acceptable. The preparation of pharmaceutically acceptable salts of the ionizable lipids of the present disclosure can be carried out by methods known in the art. For instance, they can be prepared from the parent compound, which contains a basic or acidic moiety, by conventional chemical methods. Generally, such salts are, for example, prepared by reacting the free acid or base forms of the ionizable lipids of the present disclosure with a stoichiometric amount of the appropriate pharmaceutically acceptable base or acid in water or in an organic solvent or in a mixture of them.

Examples of pharmaceutically acceptable salts include acid addition salts formed with inorganic acids e.g. hydrochloric, hydrobromic, sulfuric, nitric, or phosphoric acid; and organic acids e.g. succinic, maleic, acetic, fumaric, citric, tartaric, benzoic, malic, lactic, formic, propionic, glycolic, camphorsulfuric, mandelic, benzenesulfonic, p-toluenesulfonic, oxalic, methanesulfonic or naphthalenesulfonic acid; and base addition salts formed with alkali metals and alkaline earth metals and organic bases such as N,N-dibenzylethylenediamine, chloroprocaine, choline, diethanolamine, ethylenediamine, meglumine, lysine, and procaine. The ionizable lipids of the present disclosure and their salts may differ in some physical properties, but they are equivalent for the purposes of the present invention.

The ionizable lipids of the present disclosure have asymmetric centers and, therefore, can give rise to various stereoisomers. As used herein, the term "stereoisomer" refers to all isomers of individual ionizable lipids that differ only in the orientation of their atoms in space. The term stereoisomer includes enantiomers, racemates, racemic mixtures, geometric isomers (cis/trans or syn/anti or E/Z), and diastereomers. The present invention relates to each of these stereoisomers and also mixtures thereof.

The preparation processes described herein can be modified to give enantiopure compounds as well as mixtures of stereoisomers. It is possible to prepare specific stereoisomers or specific mixtures by various processes including the use of stereospecific reagents or by introducing chiral centers into the compounds during its preparation process. In addition, it is possible to separate stereoisomers once the compound has been prepared by standard resolution techniques known to the skilled person.

In all embodiments of the invention referring to the ionizable lipids of the present disclosure, the pharmaceutically acceptable salts, or stereoisomer of the ionizable lipids or of their pharmaceutically acceptable salts are always contemplated even if they are not specifically mentioned.

The ionizable lipids of the present disclosure also include isotopes of the structure depicted. "Isotopes" refers to atoms having the same atomic number but different mass numbers resulting from a different number of neutrons in the nuclei. For example, isotopes include, without being limited to, tritium, deuterium, ¹³C or ¹⁴C, or ¹⁵N. Further, a compound or salt of the present disclosure can be prepared in combination with solvent or water molecules to form solvates and hydrates by routine methods.

The ionizable lipids of the present disclosure are characterized by their retention time, mass spectrometry, size distribution, polydispersity index, and Z-potential. These parameters can be measured by methods well-known in the art. Some of them are indicated in more detail in the examples below.

According to one embodiment, R₃ is a 5-membered ring or a 6-membered ring comprising one N atom and, optionally, a second heteroatom selected preferably from N and O.

In another embodiment, optionally in combination with one or more features of the various embodiments described above, R3 is selected from the following structures:

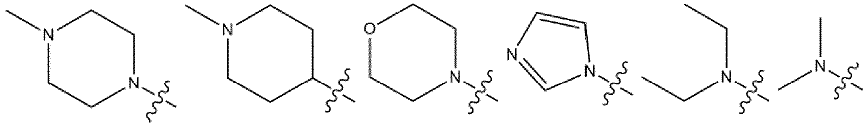

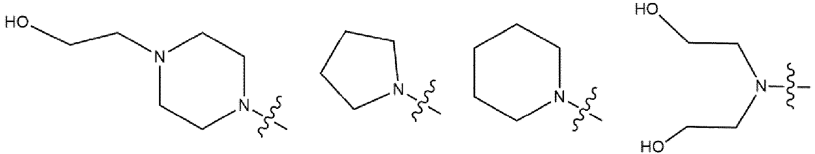

In another embodiment, optionally in combination with one or more features of the various embodiments described above, n is selected from 0, 1, 2 or 3; p is selected from 0, 1, 2, or 3; m is selected from 0, 1, 2 or 3; t is 0 or 1; q is 1 or 2, and t + q = 2.

In another embodiment, n is 2 or 3, m = t = 0, p = q = 2,
A=B= 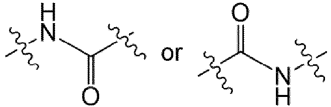
wherein
R₁ is a C10-C20 branched alkyl;
R₂ is a C2-C30 linear or branched alkyl or a C6-C24 linear alkenyl or alkynyl; and
R₃ is: 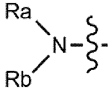
wherein Ra = Rb = C1-C4 alkyl.

In another embodiment, n = 2, and R₂ is C2-C30 linear or branched alkyl, and wherein R3 is: 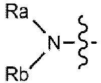 wherein Ra = Rb = C1-C4 alcohol terminated alkyl.

In another embodiment, n is 2 or 3, m = t = 0, p = q = 2,
A is 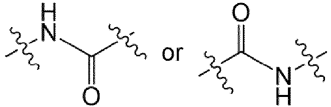
R₁ is C10-C20 branched alkyl, R₂ is C3-C30 linear or branched alkyl, and wherein R₃ is an imidazole ring.

In another embodiment, n = 3, m = t = 0, p = q = 2,
A is 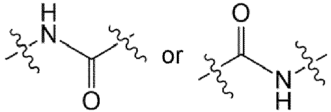
R₁ is C10-C20 branched alkyl, R₂ is C4-C20 linear or branched alkyl, and R₃ is a pyrrolidine ring.

In another embodiment, n = 2, m = t = 0, p = q = 2,
A is 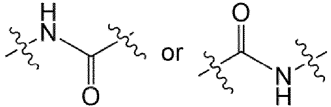
R1 is C10-C20 linear or branched alkyl, R₂ is C4-C20 linear or branched alkyl, and wherein R₃ is N-4-methylpiperazine.

In another embodiment, n is selected from 2 or 3,
m = t = 0, and p = q = 2,
A=B= 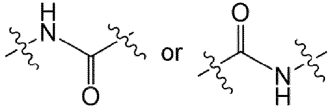
R₁ is C10-C22 linear or branched alkyl, alkenyl or alkynyl; R₂ is C3-C30 linear or branched alkyl or C3-C30 linear alkenyl or alkynyl; and wherein R₃ is a morpholino ring.

In another embodiment, n is selected from 2 or 3, m = 0, p = 2,
q = t = 1,
A is 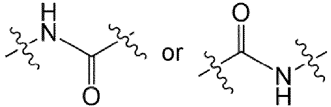
B = D, R₁ is C10-C22 linear branched alkyl; R₂ is C3-C30 linear or branched alkyl or C3-C30 linear alkenyl or alkynyl, and R₃ is a morpholino ring.

Examples of ionizable lipids of formula (I) include the compounds depicted in Table 1 below. **Table 1**

| **Example number** | **Ref. number** | **Structure** | **Retention time (min)** | **Theoretical** / **experimental MS** |
|---|---|---|---|---|
| 1 | VC-LC-0099 | 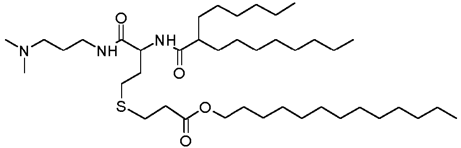 | 16,67 | 712.60/712.73 |
| 2 | VC-LC-0101 | 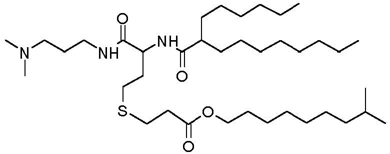 | 15.42 | 670.55/670.72 |
| 3 | VC-LC-0103 | 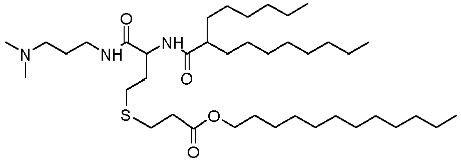 | 16.14 | 698.58/698.74 |
| 4 | VC-LC-0104 | 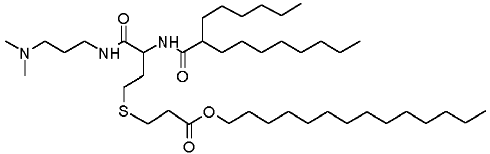 | 16.83 | 726.61/726.79 |
| 5 | VC-LC-0106 | 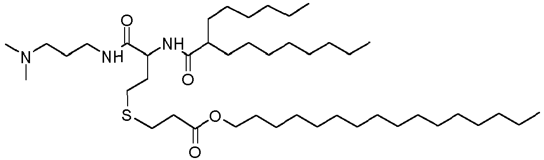 | 17.43 | 754.64/754.78 |
| 6 | VC-LC-0107 | 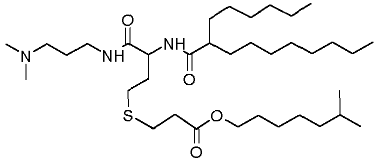 | 14.49 | 642.52/642.62 |
| 7 | VC-LC-0141 | 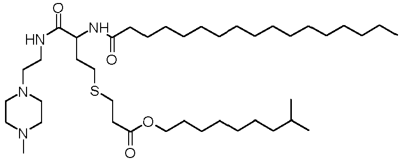 | 15.15 | 725.59/725.79 |
| 8 | VC-LC-0163 | 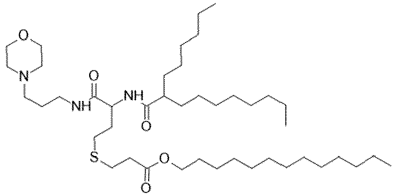 | 15.77 | 754.61/753.83 |
| 9 | VC-LC-0165 | 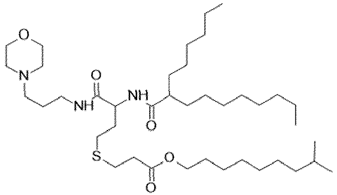 | 15.22 | 712.16/712.76 |
| 10 | VC-LC-0166 | 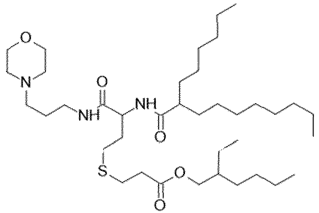 | 14.52 | 684.53/684.7 |
| 11 | VC-LC-0167 | 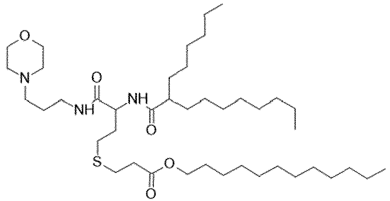 | 16.06 | 740.59/740.8 |
| 12 | VC-LC-0168 | 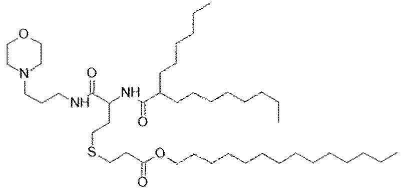 | 16.74 | 768.62/768.78 |
| 13 | VC-LC-0169 | 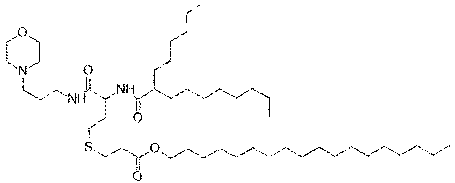 | 18.04 | 824.69/824.84 |
| 14 | VC-LC-0170 | 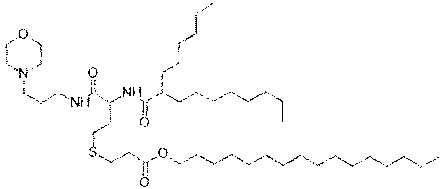 | 17.44 | 796.65/796.82 |
| 15 | VC-LC-0171 | 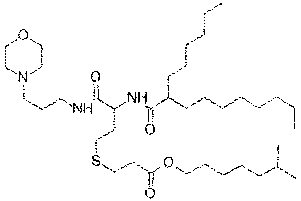 | 14.51 | 684.53/684.69 |
| 16 | VC-LC-0173 | 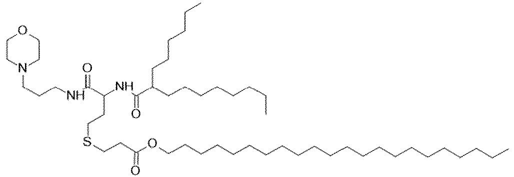 | 18.95 | 880.75/880.92 |
| 17 | VC-LC-0175 | 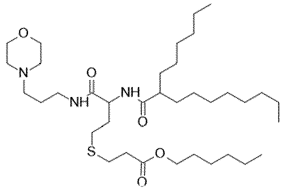 | 13.75 | 656.50/656.65 |
| 18 | VC-LC-0176 | 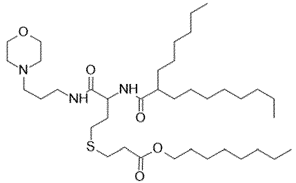 | 14.64 | 684.53/684.70 |
| 19 | VC-LC-0178 | 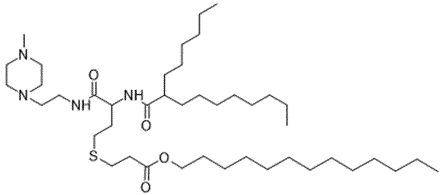 | 15.85 | 753.62/753.78 |
| 20 | VC-LC-0180 | 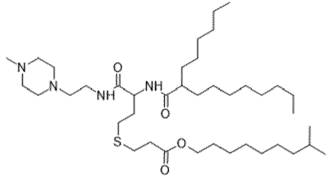 | 14.93 | 711.58/711.77 |
| 21 | VC-LC-0181 | 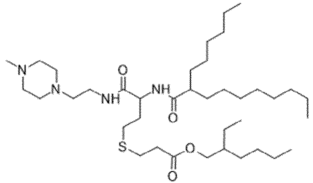 | 14.13 | 683.55/683.71 |
| 22 | VC-LC-0182 | 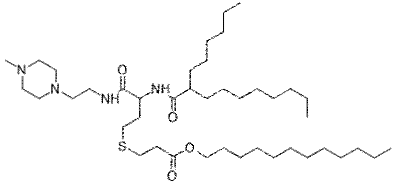 | 15.74 | 739.61/739.81 |
| 23 | VC-LC-0183 | 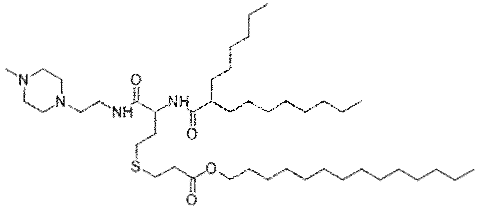 | 16.39 | 767.64/767.79 |
| 24 | VC-LC-0185 | 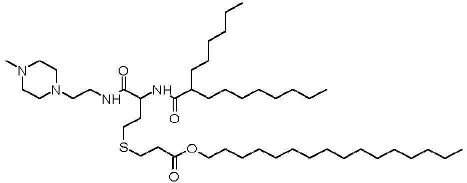 | 17.08 | 795.67/795.85 |
| 25 | VC-LC-0186 | 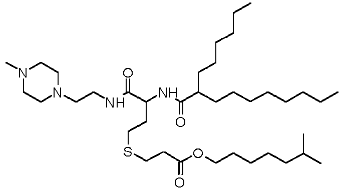 | 14.11 | 683.55/683.71 |
| 26 | VC-LC-0191 | 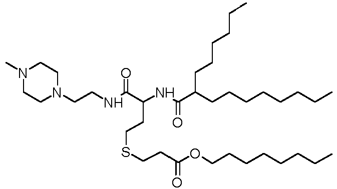 | 14.13 | 683.55/683.71 |
| 27 | VC-LC-0194 | 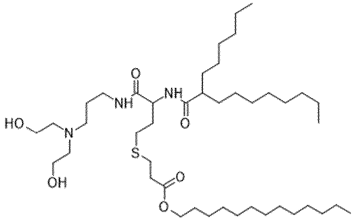 | 15.83 | 772.62/772.84 |
| 28 | VC-LC-0196 | 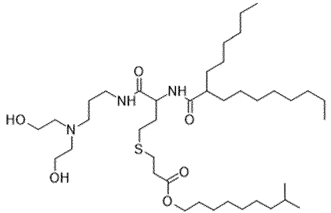 | 14.71 | 730.58/730.80 |
| 29 | VC-LC-0197 | 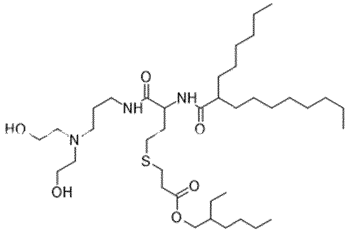 | 14.12 | 702.54/702.73 |
| 30 | VC-LC-0198 | 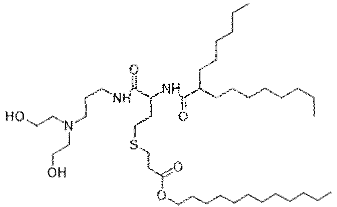 | 15.67 | 758.60/758.81 |
| 31 | VC-LC-0199 | 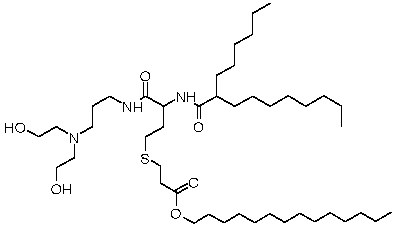 | 16.43 | 786.63/786.84 |
| 32 | VC-LC-0200 | 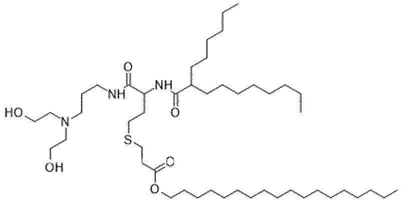 | 17.79 | 842.70/842.89 |
| 33 | VC-LC-0201 | 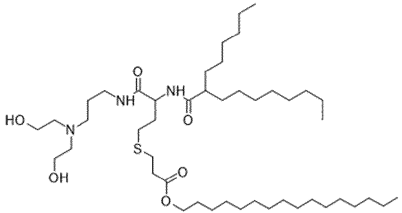 | 17.09 | 814.67/814.86 |
| 34 | VC-LC-0202 | 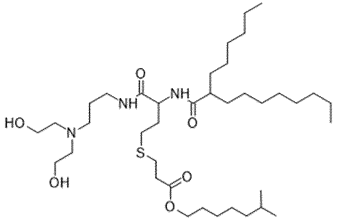 | 14.05 | 702.54/702.74 |
| 35 | VC-LC-0204 | 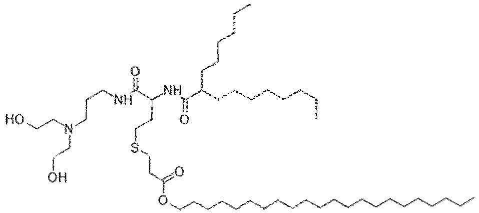 | 18.58 | 898.76/899.01 |
| 36 | VC-LC-0206 | 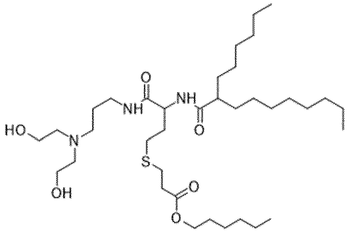 | 13.38 | 674.51/674.69 |
| 37 | VC-LC-0207 | 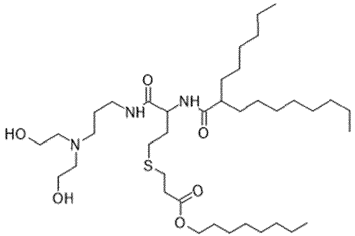 | 14.10 | 702.54/702.71 |
| 38 | VC-LC-0209 | 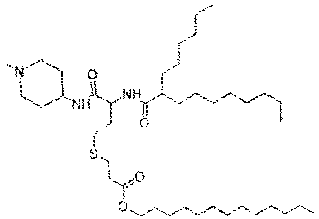 | 16.11 | 724.60/724.81 |
| 39 | VC-LC-0211 | 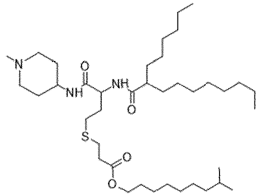 | 15.04 | 682.55/682.73 |
| 40 | VC-LC-0212 | 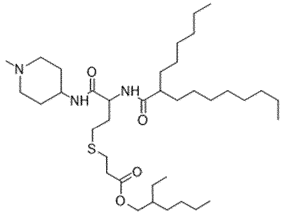 | 14.30 | 654.52/654.67 |
| 41 | VC-LC-0213 | 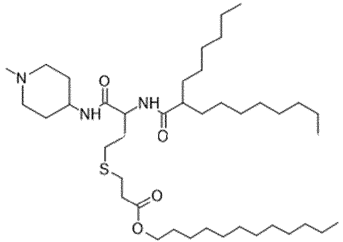 | 15.03 | 710.58/710.79 |
| 42 | VC-LC-0214 | 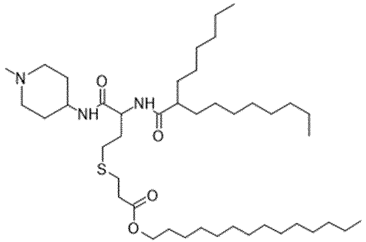 | 16.62 | 738.61/738.83 |
| 43 | VC-LC-0215 | 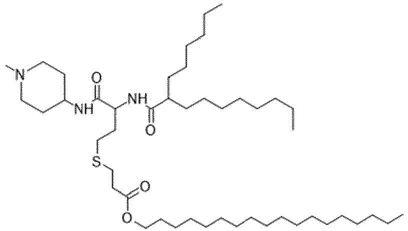 | 18.01 | 794.68/794.88 |
| 44 | VC-LC-0216 | 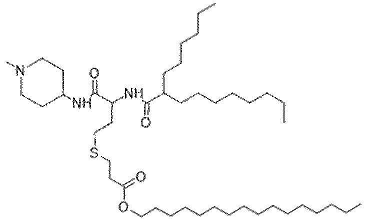 | 17.39 | 766.64/766.87 |
| 45 | VC-LC-0217 | 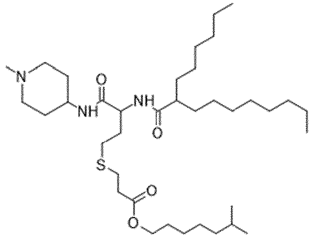 | 14.29 | 654.52/654.69 |
| 46 | VC-LC-0241 | 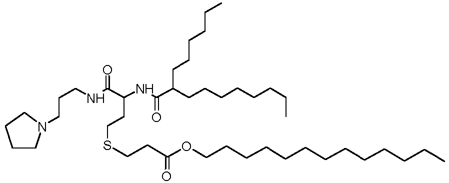 | 16.25 | 738.61/738.84 |
| 47 | VC-LC-0243 | 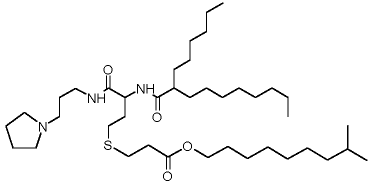 | 15.19 | 696.54/696.79 |
| 48 | VC-LC-0244 | 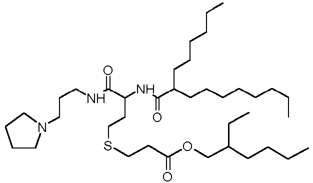 | 14.51 | 668.53/668.72 |
| 49 | VC-LC-0245 | 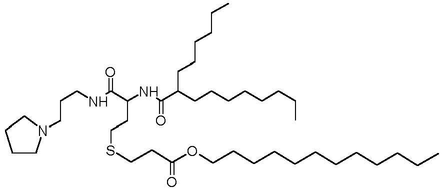 | 16.02 | 724.60/724.81 |
| 50 | VC-LC-0248 | 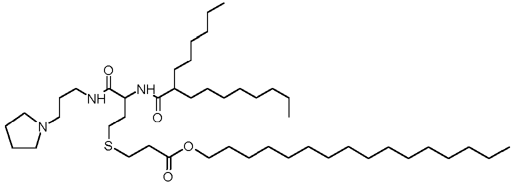 | 17.43 | 780.66/780.88 |
| 51 | VC-LC-0254 | 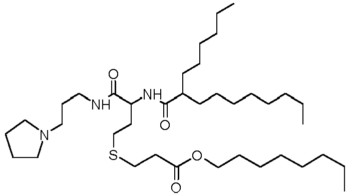 | 14.58 | 668.53/668.74 |
| 52 | VC-LC-0256 | 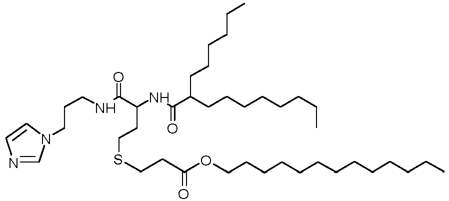 | 15.97 | 735.58/735.83 |
| 53 | VC-LC-0258 | 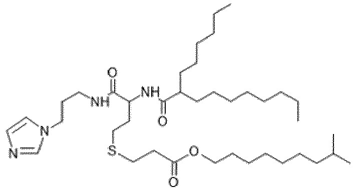 | 14.91 | 693.53/693.75 |
| 54 | VC-LC-0259 | 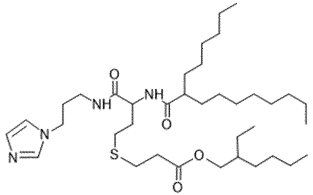 | 14.22 | 665.50/665.71 |
| 55 | VC-LC-0260 | 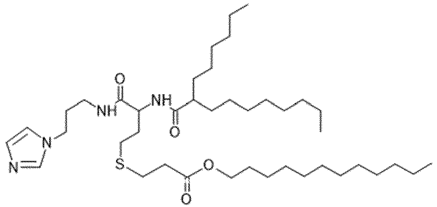 | 15.81 | 721.56/721.80 |
| 56 | VC-LC-0261 | 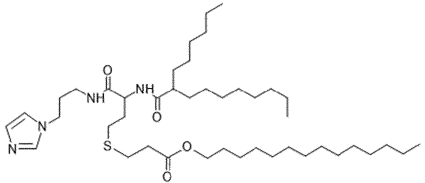 | 16.49 | 749.59/749.82 |
| 57 | VC-LC-0262 | 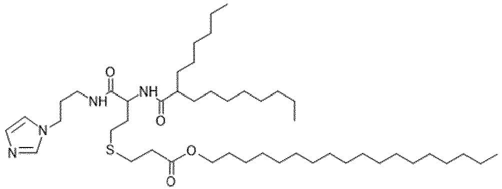 | 17.82 | 805.65/805.87 |
| 58 | VC-LC-0263 | 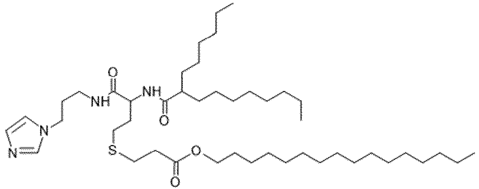 | 17.11 | 777.62/777.85 |
| 59 | VC-LC-0264 | 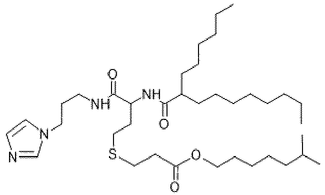 | 14.20 | 665.50/665.72 |
| 60 | VC-LC-0268 | 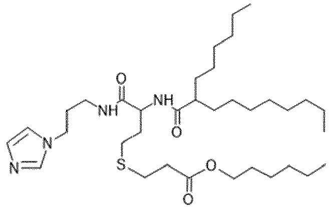 | 13.44 | 637.47/637.64 |
| 61 | VC-LC-0269 | 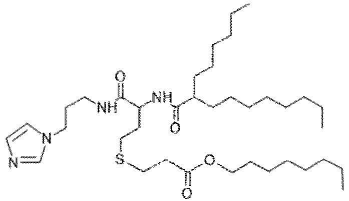 | 14.23 | 665.50/665.70 |
| 62 | VC-LC-0289 | 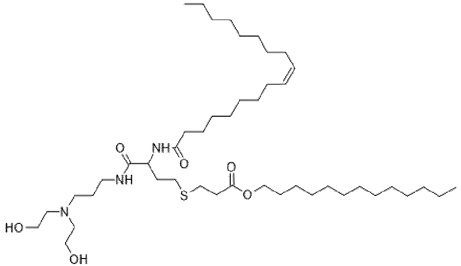 | 16.35 | 798.63/798.80 |
| 63 | VC-LC-0290 | 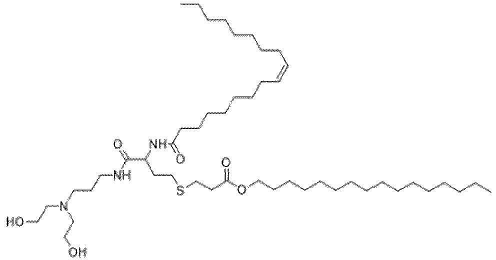 | 17.16 | 840.68/840.89 |
| 64 | VC-LC-0292 | 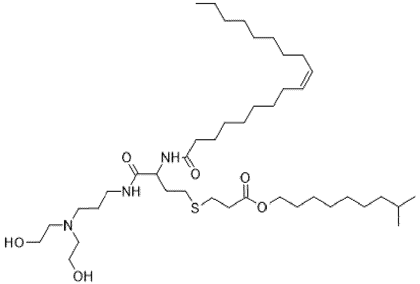 | 15.45 | 756.59/756.84 |
| 65 | VC-LC-0293 | 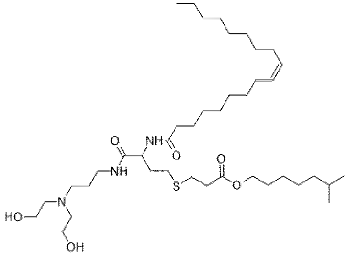 | 14.67 | 728.56/728.82 |
| 66 | VC-LC-0294 | 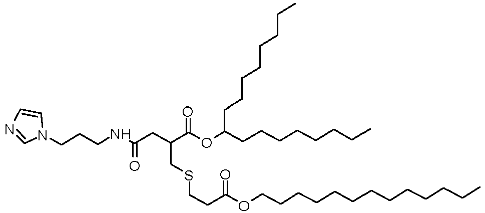 | 16.93 | 764.59/764.80 |
| 67 | VC-LC-0296 | 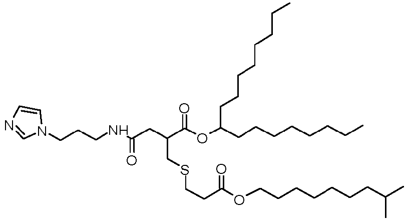 | 16.10 | 722.55/722.13 |
| 68 | VC-LC-0297 | 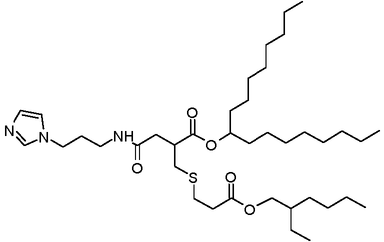 | 15.67 | 694.51/694.71 |
| 69 | VC-LC-0298 | 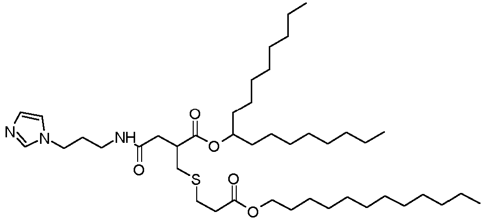 | 16.86 | 750.58/750.78 |
| 70 | VC-LC-0299 | 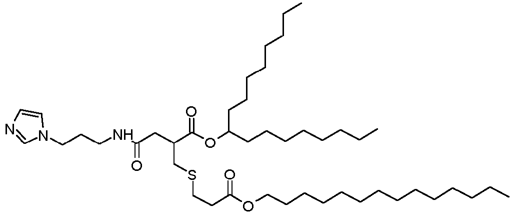 | 17.33 | 778.61/778.82 |
| 71 | VC-LC-0300 | 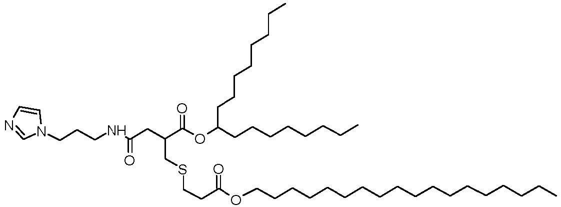 | 18.45 | 834.67/834.86 |
| 72 | VC-LC-0301 | 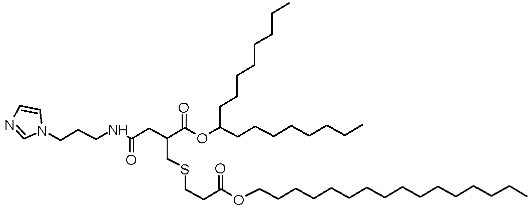 | 18.09 | 806.27/806.82 |
| 73 | VC-LC-0302 | 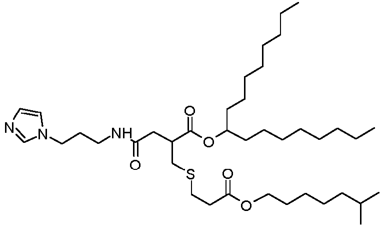 | 15.59 | 694.51/694.71 |
| 74 | VC-LC-0304 | 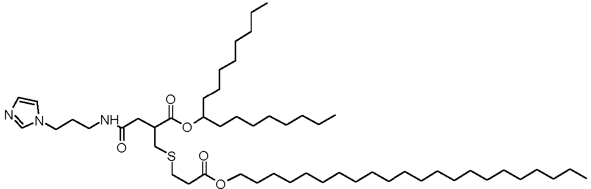 | 19.26 | 890.73/890.91 |
| 75 | VC-LC-0306 | 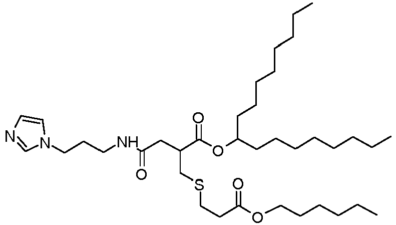 | 14.88 | 666.48/666.67 |
| 76 | VC-LC-0307 | 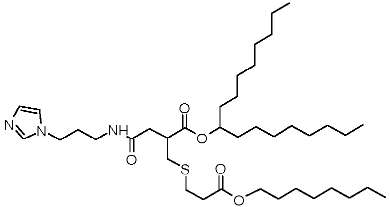 | 15.66 | 694.51/694.73 |
| 77 | VC-LC-0314 | 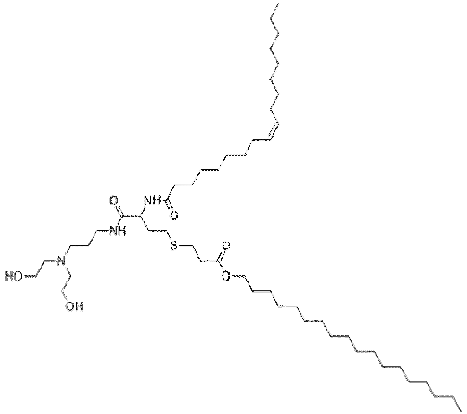 | 17.68 | 868.71/868.93 |
| 78 | VC-LC-0322 | 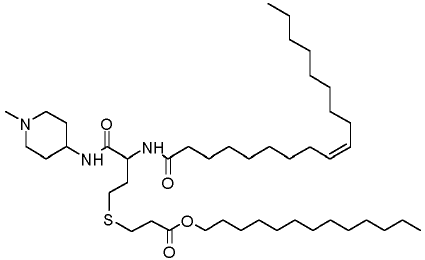 | 16.25 | 750.61/750.82 |
| 79 | VC-LC-0326 | 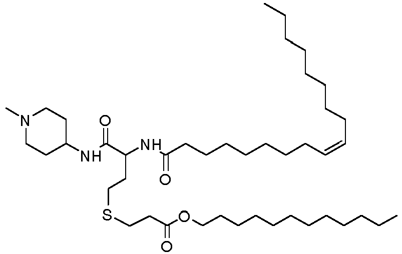 | 16.12 | 736.60/736.85 |
| 80 | VC-LC-0328 | 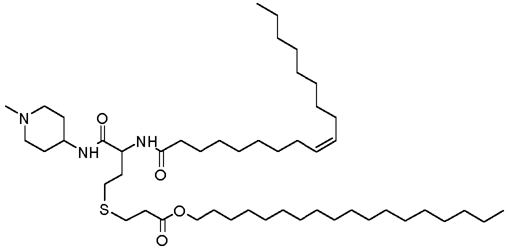 | 17.90 | 820.69/820.88 |
| 81 | VC-LC-0329 | 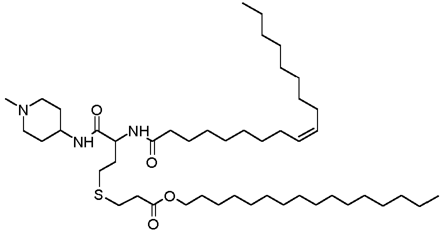 | 17.46 | 792.66/792.88 |
| 82 | VC-LC-0338 | 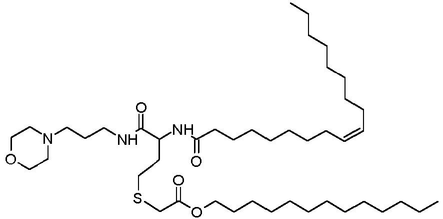 | 16.35 | 780.63/780.88 |
| 83 | VC-LC-0353 | 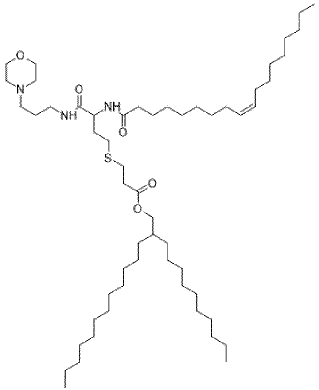 | 19.19 | 934.80/935.06 |
| 84 | VC-LC-0354 | 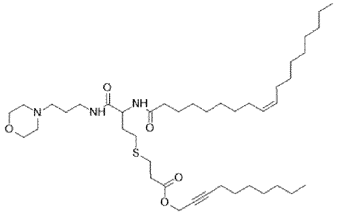 | 14.99 | 734.55/734.79 |
| 85 | VC-LC-0355 | 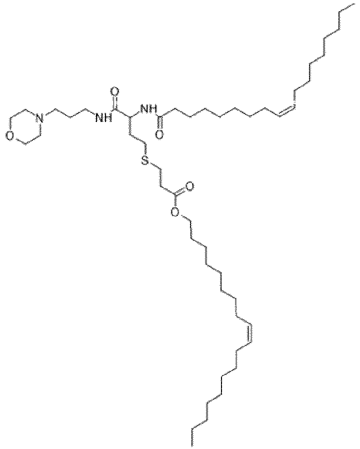 | 17.57 | 848.69/848.93 |
| 86 | VC-LC-0356 | 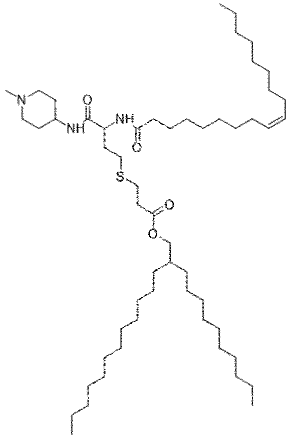 | 19.22 | 904.78/905.05 |
| 87 | VC-LC-0361 | 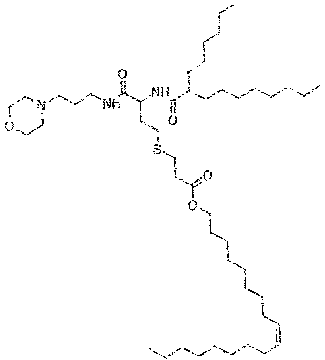 | 17.58 | 822.67/822.86 |
| 88 | VC-LC-0362 | 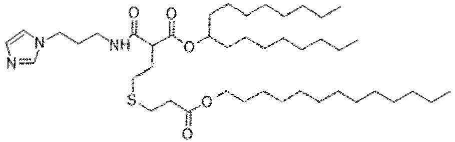 | 17.16 | 764.59/764.81 |
| 99 | VC-LC-0363 | 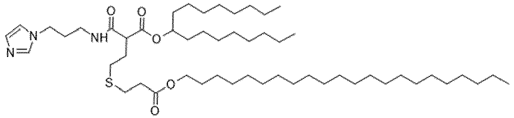 | 19.36 | 890.73/890.94 |
| 90 | VC-LC-0364 | 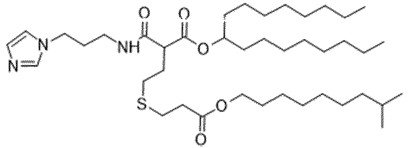 | 16.33 | 722.55/722.78 |
| 91 | VC-LC-0365 | 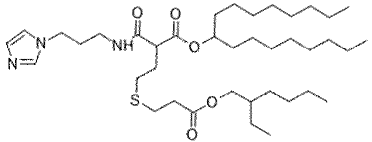 | 15.66 | 694.51/694.72 |
| 92 | VC-LC-0366 | 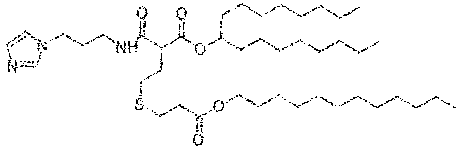 | 17.02 | 750.58/750.77 |
| 93 | VC-LC-0367 | 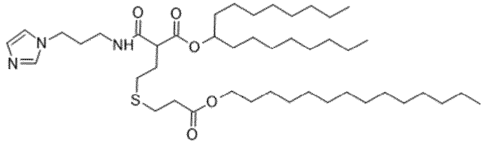 | 17.57 | 778.61/778.82 |
| 94 | VC-LC-0368 | 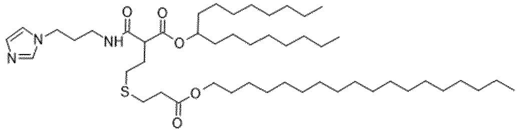 | 18.52 | 834.67/834.86 |
| 95 | VC-LC-0369 | 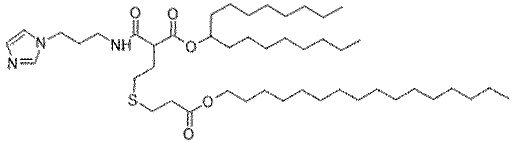 | 18.16 | 806.64/806.83 |
| 96 | VC-LC-0370 | 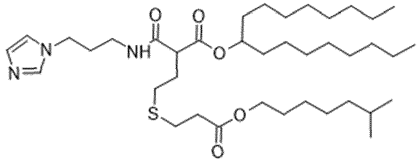 | 15.75 | 694.51/694.75 |
| 97 | VC-LC-0374 | 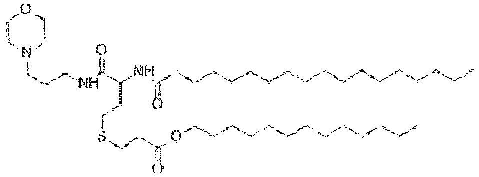 | 16.87 | 782.64/782.81 |
| 98 | VC-LC-0380 | 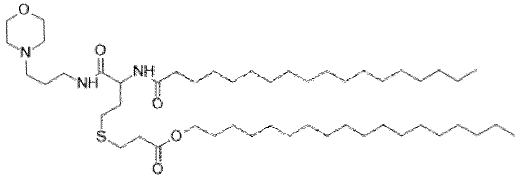 | 18.47 | 852.72/852.89 |
| 99 | VC-LC-0389 | 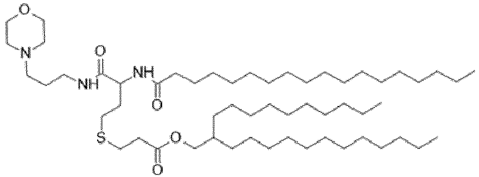 | 19.37 | 936.81/937.05 |
| 100 | VC-LC-0390 | 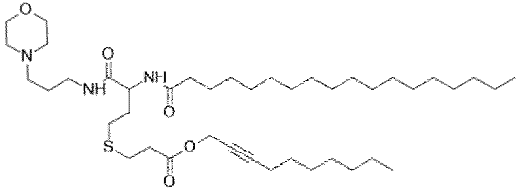 | 15.43 | 736.56/736.81 |
| 101 | VC-LC-0392 | 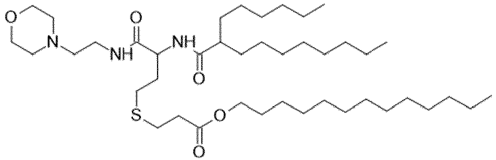 | 15.94 | 740.59/740.80 |
| 102 | VC-LC-0407 | 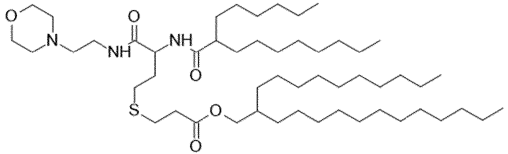 | 18.99 | 894.76/894.99 |
| 103 | VC-LC-0408 | 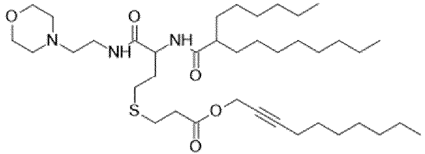 | 14.51 | 694.51/694.74 |
| 104 | VC-LC-0428 | 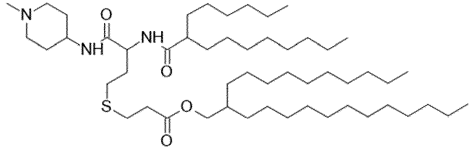 | 18.96 | 878.46/878.95 |
| 105 | VC-LC-0429 | 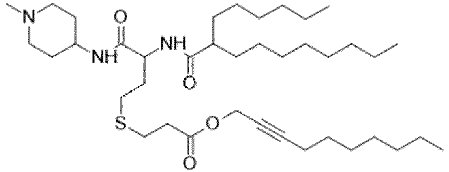 | 14.26 | 678.52/678.70 |
| 106 | VC-LC-0430 | 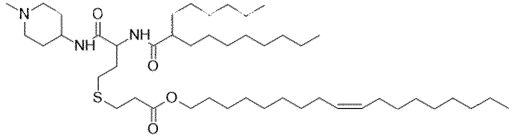 | 17.22 | 792.66/792.79 |
| 107 | VC-LC-0431 | 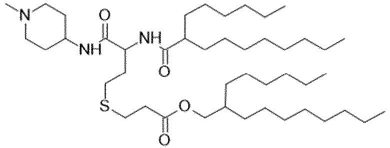 | 17.04 | 766.64/766.84 |
| 108 | VC-LC-0432 | 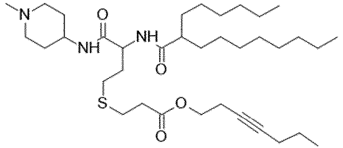 | 12.89 | 636.47/636.65 |
| 109 | VC-LC-0433 | 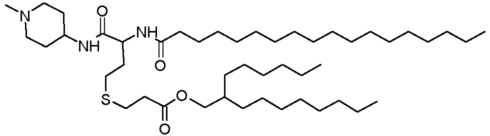 | 17.82 | 794.68/794.88 |
| 110 | VC-LC-0434 | 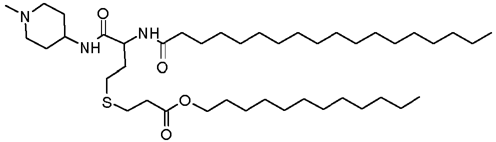 | 16.84 | 738.61/738.87 |
| 111 | VC-LC-0435 | 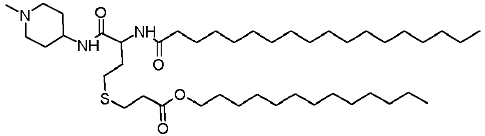 | 16.88 | 752.63/752.85 |
| 112 | VC-LC-0437 | 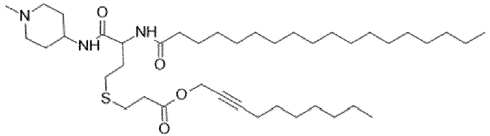 | 15.54 | 706.55/706.78 |
| 113 | VC-LC-0438 | 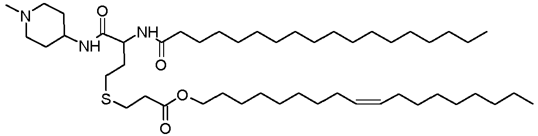 | 18.04 | 820.69/820.90 |
| 114 | VC-LC-0439 | 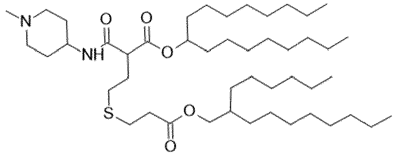 | 18.18 | 795.66/795.81 |
| 115 | VC-LC-0440 | 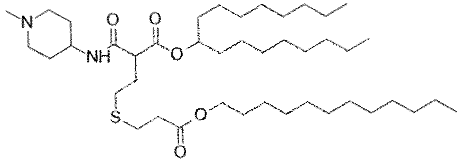 | 17.07 | 739.60/739.81 |
| 116 | VC-LC-0441 | 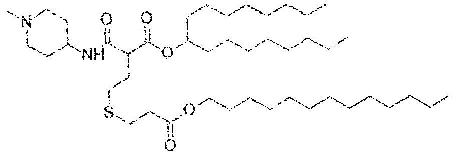 | 17.24 | 753.61/753.81 |
| 117 | VC-LC-0442 | 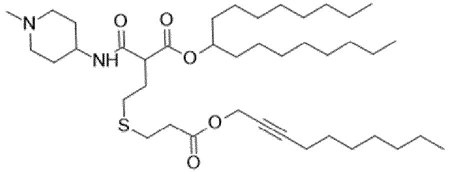 | 15.84 | 707.53/707.75 |
| 118 | VC-LC-0443 | 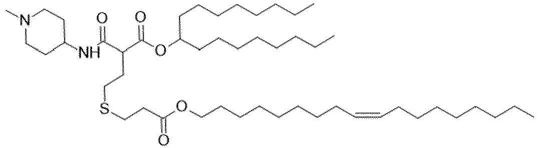 | 18.25 | 821.67/821.89 |
| 119 | VC-LC-0444 | 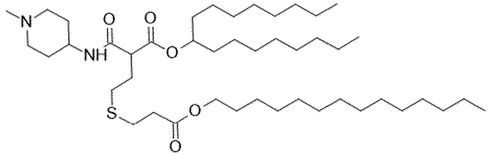 | 17.65 | 767.63/767.85 |
| 120 | VC-LC-0445 | 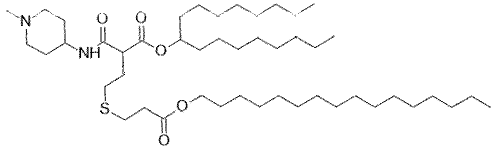 | 18.16 | 795.66/795.89 |
| 121 | VC-LC-0473 | 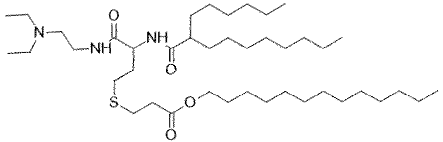 | 16.09 | 726.61/726.78 |
| 122 | VC-LC-0474 | 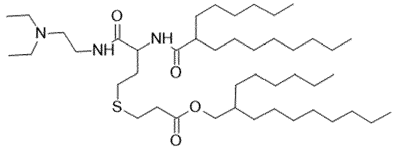 | 17.11 | 768.66/768.82 |
| 123 | VC-LC-0475 | 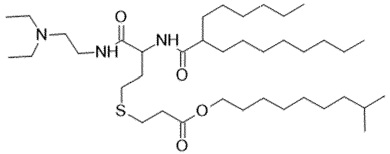 | 15.14 | 684.57/684.76 |
| 124 | VC-LC-0477 | 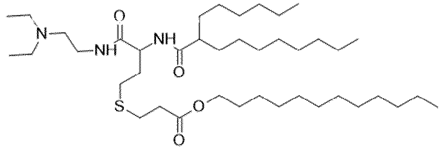 | 15.90 | 712.16/712.76 |
| 125 | VC-LC-0478 | 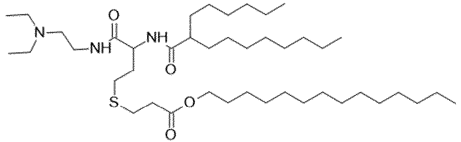 | 16.58 | 740.63/740.84 |
| 126 | VC-LC-0480 | 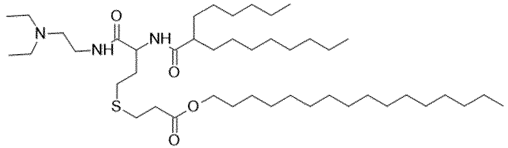 | 17.36 | 768.66/768.87 |
| 127 | VC-LC-0487 | 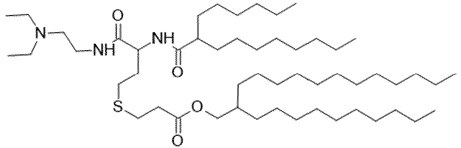 | 19.03 | 880.78/880.99 |
| 128 | VC-LC-0488 | 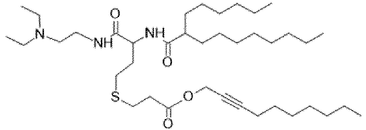 | 14.61 | 680.53/680.72 |
| 129 | VC-LC-0489 | 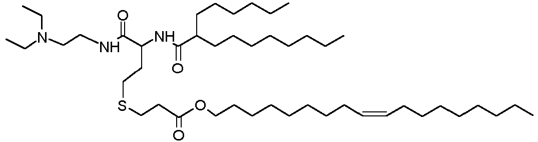 | 17.31 | 794.68/794.88 |
| 130 | VC-LC-0490 | 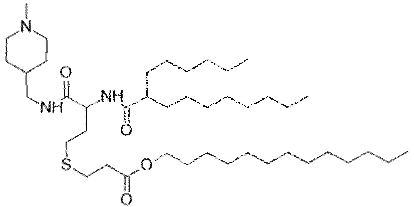 | 15.71 | 738.61/738.85 |
| 131 | VC-LC-0491 | 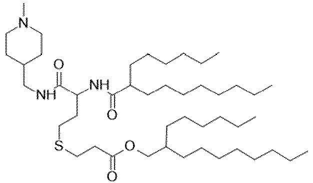 | 16.76 | 780.66/780.86 |
| 132 | VC-LC-0492 | 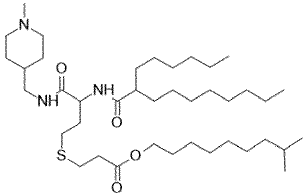 | 14.73 | 696.57/696.78 |
| 133 | VC-LC-0493 | 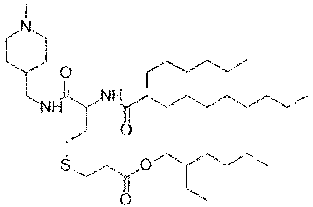 | 14.16 | 668.53/668.73 |
| 134 | VC-LC-0494 | 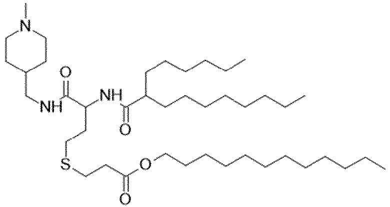 | 15.60 | 724.60/724.82 |
| 135 | VC-LC-0498 | 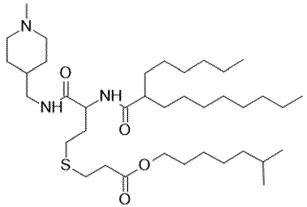 | 14.00 | 668.53/668.67 |
| 136 | VC-LC-0504 | 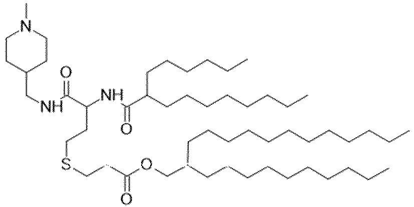 | 18.90 | 892.79/892.98 |
| 137 | VC-LC-0505 | 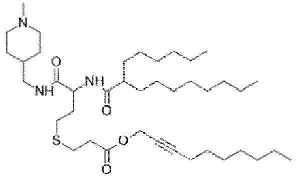 | 14.25 | 692.54/692.75 |
| 138 | VC-LC-0507 | 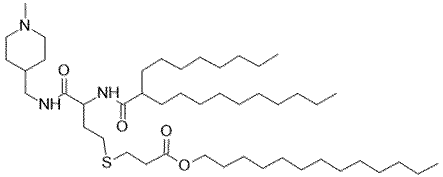 | 17.02 | 794.68/794.88 |
| 139 | VC-LC-0508 | 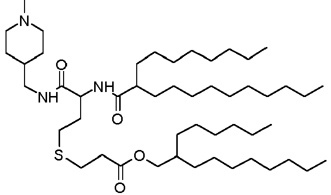 | 17.95 | 836.73/836.90 |
| 140 | VC-LC-0509 | 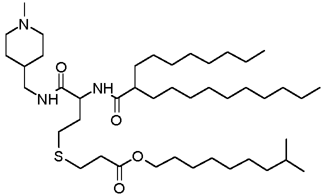 | 16.19 | 752.63/752.83 |
| 141 | VC-LC-0510 | 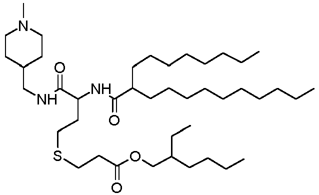 | 15.59 | 724.60/724.81 |
| 142 | VC-LC-0511 | 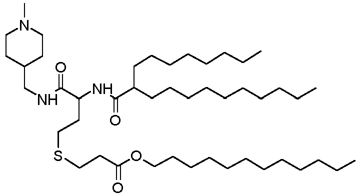 | 17.00 | 780.66/780.87 |
| 143 | VC-LC-0515 | 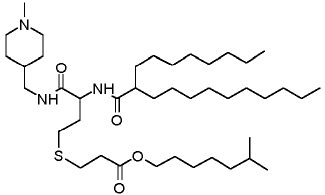 | 15.53 | 724.60/724.80 |
| 144 | VC-LC-0518 | 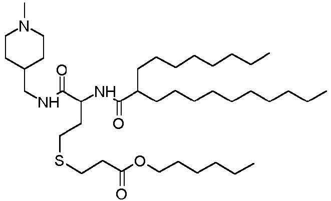 | 14.95 | 696.57/696.78 |
| 145 | VC-LC-0519 | 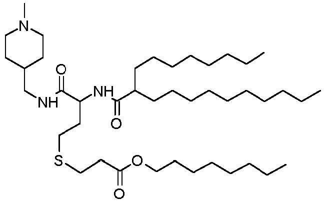 | 15.63 | 724.60/724.79 |
| 146 | VC-LC-0521 | 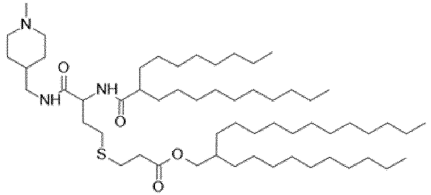 | 19.50 | 948.85/949 |
| 147 | VC-LC-0523 | 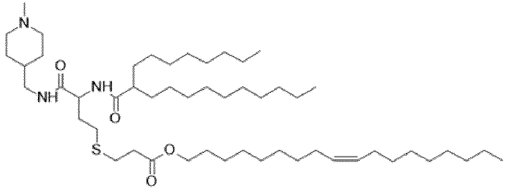 | 18.13 | 862.74/862.90 |
| 148 | VC-LC-0524 | 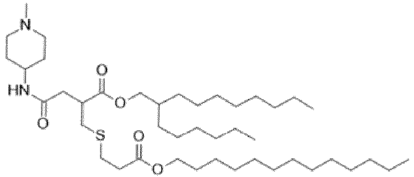 | 16.92 | 739.60/739.79 |
| 149 | VC-LC-0525 | 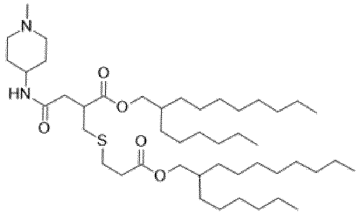 | 17.85 | 781.65/781.83 |
| 150 | VC-LC-0526 | 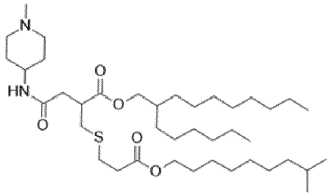 | 15.98 | 697.55/697.72 |
| 151 | VC-LC-0528 | 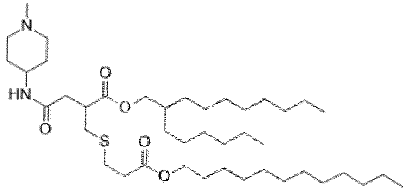 | 16.73 | 725.58/725.78 |
| 152 | VC-LC-0530 | 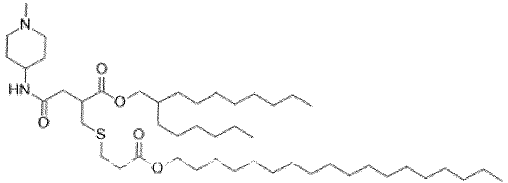 | 18.48 | 809.67/809.82 |
| 153 | VC-LC-0531 | 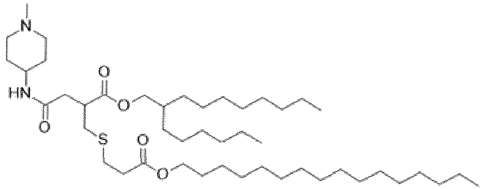 | 18.01 | 781.65/781.81 |
| 154 | VC-LC-0532 | 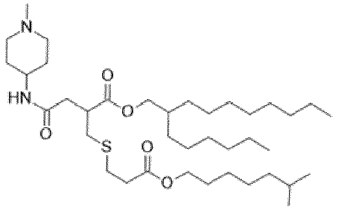 | 15.35 | 669.52/669.72 |
| 155 | VC-LC-0533 | 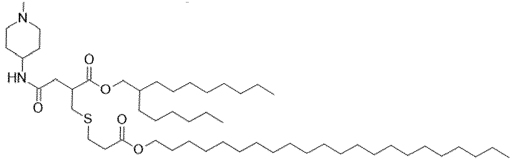 | 19.30 | 865.74/865.96 |
| 156 | VC-LC-0535 | 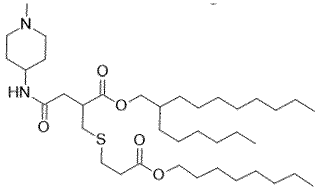 | 14.96 | 669.05/669.67 |
| 157 | VC-LC-0537 | 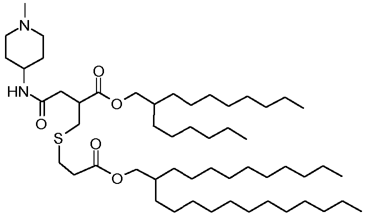 | 19.55 | 893.48/893.98 |
| 158 | VC-LC-0538 | 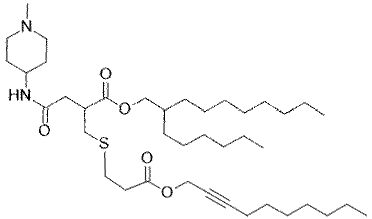 | 15.35 | 693.08/693.70 |
| 159 | VC-LC-0539 | 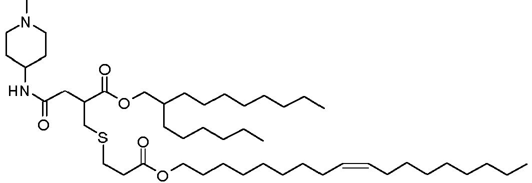 | 18.03 | 807.30/807.83 |
| 160 | VC-LC-0540 | 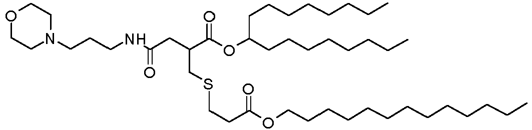 | 17.16 | 783.24/783.80 |
| 161 | VC-LC-0541 | 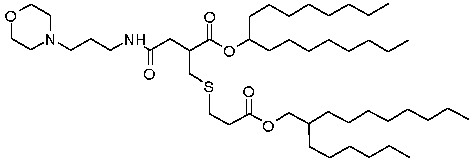 | 18.09 | 825.32/825.85 |
| 162 | VC-LC-0542 | 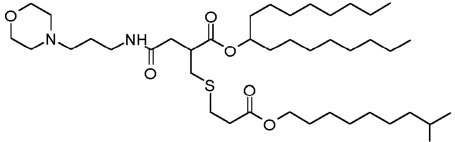 | 16.30 | 741.16/741.80 |
| 163 | VC-LC-0543 | 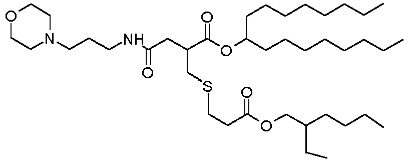 | 15.74 | 713.11/ 713.73 |
| 164 | VC-LC-0544 | 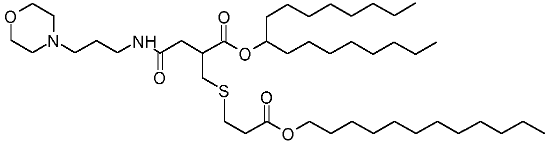 | 16.99 | 769.21/769.79 |
| 165 | VC-LC-0546 | 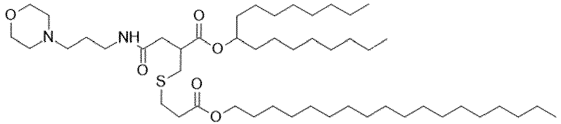 | 18.60 | 853.37/853.84 |
| 166 | VC-LC-0547 | 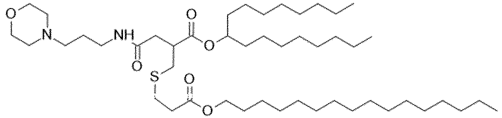 | 18.15 | 825.32/825.83 |
| 167 | VC-LC-0548 | 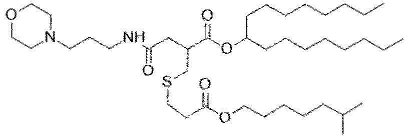 | 15.66 | 713.55/713.75 |
| 168 | VC-LC-0549 | 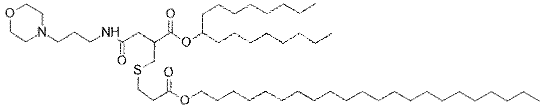 | 19.38 | 909.77/909.97 |
| 169 | VC-LC-0550 | 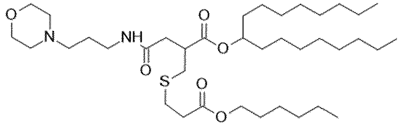 | 15.04 | 685.52/685.72 |
| 170 | VC-LC-0551 | 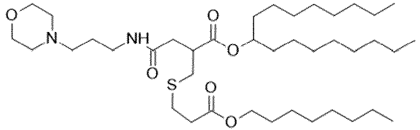 | 15.75 | 713.55/713.74 |
| 171 | VC-LC-0553 | 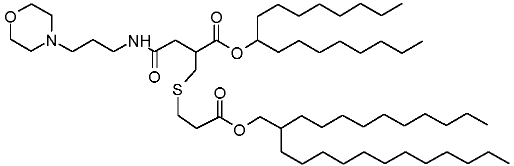 | 19.60 | 937.80/938.02 |
| 172 | VC-LC-0554 | 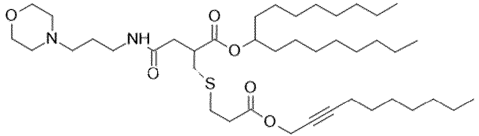 | 15.87 | 737.55/737.76 |
| 173 | VC-LC-0556 | 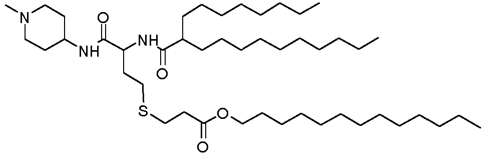 | 17.10 | 780.66/780.85 |
| 174 | VC-LC-0557 | 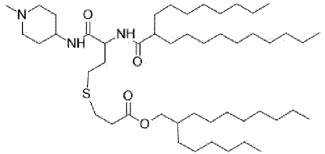 | 18.06 | 822.71/822.87 |
| 175 | VC-LC-0558 | 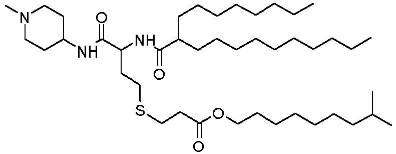 | 16.25 | 738.62/738.80 |
| 176 | VC-LC-0559 | 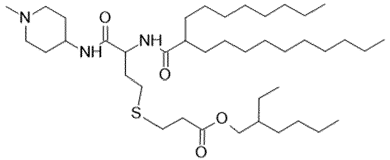 | 15.68 | 710.59/710.77 |
| 177 | VC-LC-0566 | 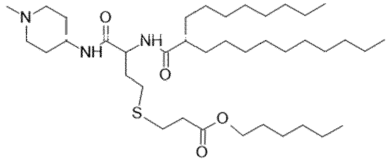 | 14.935 | 682.55/682.75 |
| 178 | VC-LC-0577 | 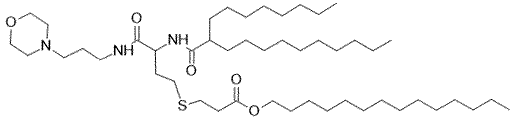 | 17.57 | 824.69/824.87 |
| 179 | VC-LC-0587 | 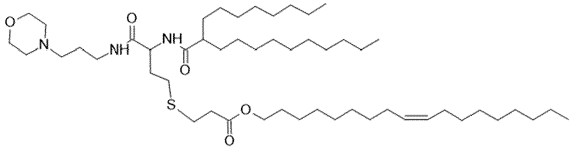 | 18.13 | 878.74/878.89 |
| 180 | VC-LC-0605 | 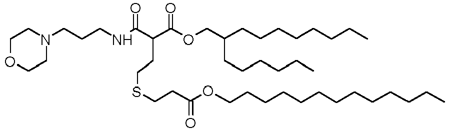 | 16.99 | 769.61/769.79 |
| 181 | VC-LC-0606 | 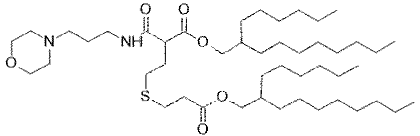 | 17.88 | 811.66/811.84 |
| 182 | VC-LC-0607 | 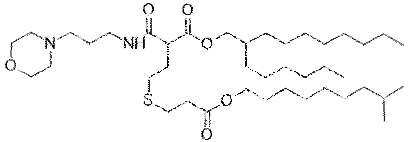 | 16.11 | 727.56/727.77 |
| 183 | VC-LC-0608 | 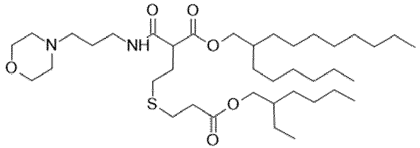 | 15.28 | 699.53/699.72 |
| 184 | VC-LC-0609 | 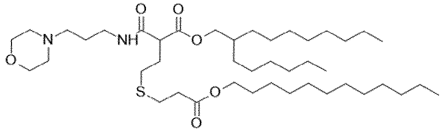 | 16.80 | 755.60/755.78 |
| 185 | VC-LC-0610 | 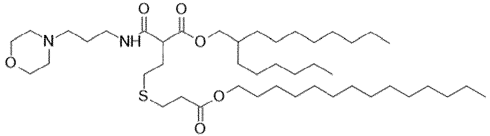 | 17.44 | 783.63/783.80 |

In another embodiment, the ionizable lipid of Formula (I) is a compound selected from the group consisting of: VC-LC-0099, VC-LC-0101, VC-LC-0163, VC-LC-0168, VC-LC-0169, VC-LC-0170, VC-LC-0178, VC-LC-0183, VC-LC-0194, VC-LC-0196, VC-LC-0198, VC-LC-0199, VC-LC-0200, VC-LC-0201, VC-LC-0202, VC-LC-0207, VC-LC-0209, VC-LC-0211, VC-LC-0213, VC-LC-0214, VC-LC-0215, VC-LC-0216, VC-LC-0241, VC-LC-0256, VC-LC-0258, VC-LC-0261, VC-LC-0262, VC-LC-0263, VC-LC-0268, VC-LC-0269, VC-LC-0289, VC-LC-0294, VC-LC-0296, VC-LC-0297, VC-LC-0298, VC-LC-0299, VC-LC-0300, VC-LC-0301, VC-LC-0302, VC-LC-0304, VC-LC-0306, VC-LC-0307, VC-LC-0353, VC-LC-0355, VC-LC-0356, VC-LC-0362, VC-LC-0366, VC-LC-0367, VC-LC-0369, VC-LC-0370, VC-LC-0389, VC-LC-0428, VC-LC-0429, VC-LC-0430, VC-LC-0431, VC-LC-0439, VC-LC-0440, VC-LC-0441, VC-LC-0442, VC-LC-0443, VC-LC-0444, VC-LC-0473, VC-LC-0474, VC-LC-0475, VC-LC-0477, VC-LC-0478, VC-LC-0480, VC-LC-0487, VC-LC-0488, VC-LC-0489, VC-LC-0490, VC-LC-0491, VC-LC-0492, VC-LC-0504, VC-LC-0505, VC-LC-0507, VC-LC-0508, VC-LC-0509, VC-LC-0510, VC-LC-0515, VC-LC-0521, VC-LC-0524, VC-LC-0525, VC-LC-0531, VC-LC-0539, VC-LC-0540, VC-LC-0541, VC-LC-0542, VC-LC-0543, VC-LC-0544, VC-LC-0546, VC-LC-0547, VC-LC-0548, VC-LC-0549, VC-LC-0550, VC-LC-0551, VC-LC-0553, VC-LC-0554, VC-LC-0556, VC-LC-0557, VC-LC-0558, VC-LC-0559, VC-LC-0606, VC-LC-0607, VC-LC-0608, VC-LC-0609, VC-LC-0610 described herein; or a pharmaceutically acceptable salt thereof, or a stereoisomer of any one of them.

### Preparation processes

Processes for the preparation of the ionizable lipids of Formula (I) are also part of the invention.

All reactants and solvents needed for the preparation of the compounds of the present disclosure are commercially available.

As a way of example, the ionizable lipid of Formula (I) can be prepared according to any of the following synthetic schemes. Any person skilled in the art will know which reactants are required to obtain any particular ionizable lipid according to the present disclosure following the synthetic methods depicted in the schemes below or an analogous method thereof.

Ionizable lipids of Formula (I) wherein B is an amide moiety and t=0, q=2, such as, for example, compound of Formula (la), can be prepared by the synthetic method, or an analogous one, depicted in scheme 1 below.

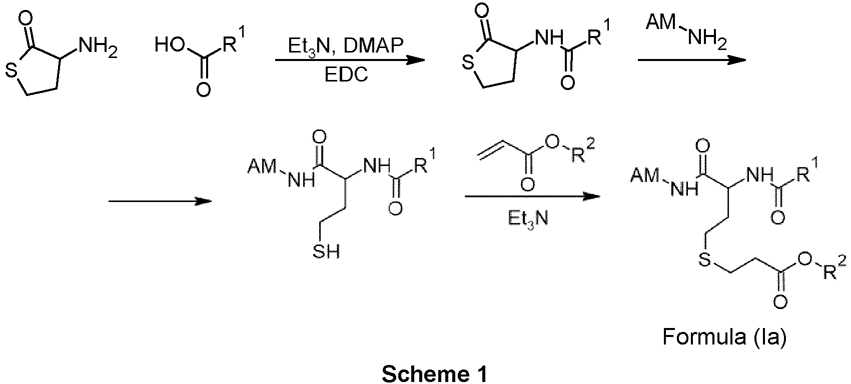

Examples of acids (R¹-COOH), amines (AM-NH₂), and acrylates (CH₂=CH-(CO)-O-R₂) that can be used for the preparation of compound of Formula (I) according to scheme 1 are listed in Tables 2, 3 and 4 below, respectively.

**Table 2**

| ACIDS | |
|---|---|
| R¹-COOH | |
| ACIDS 1-9 | 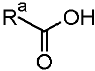 R^{a} = C₉ - C₁₇ |
| ACID 10 | 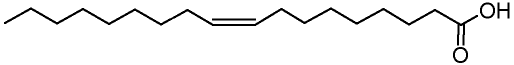 |
| ACID 11 | 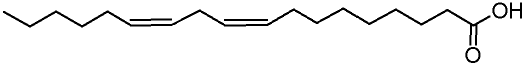 |
| ACID 12 | 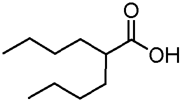 |
| ACID 13 | 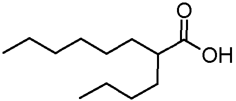 |
| ACID 14 | 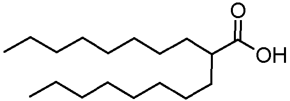 |
| ACID 15 | 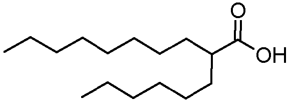 |
| ACID 16 | 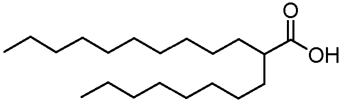 |
| ACID 17 | 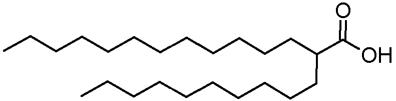 |

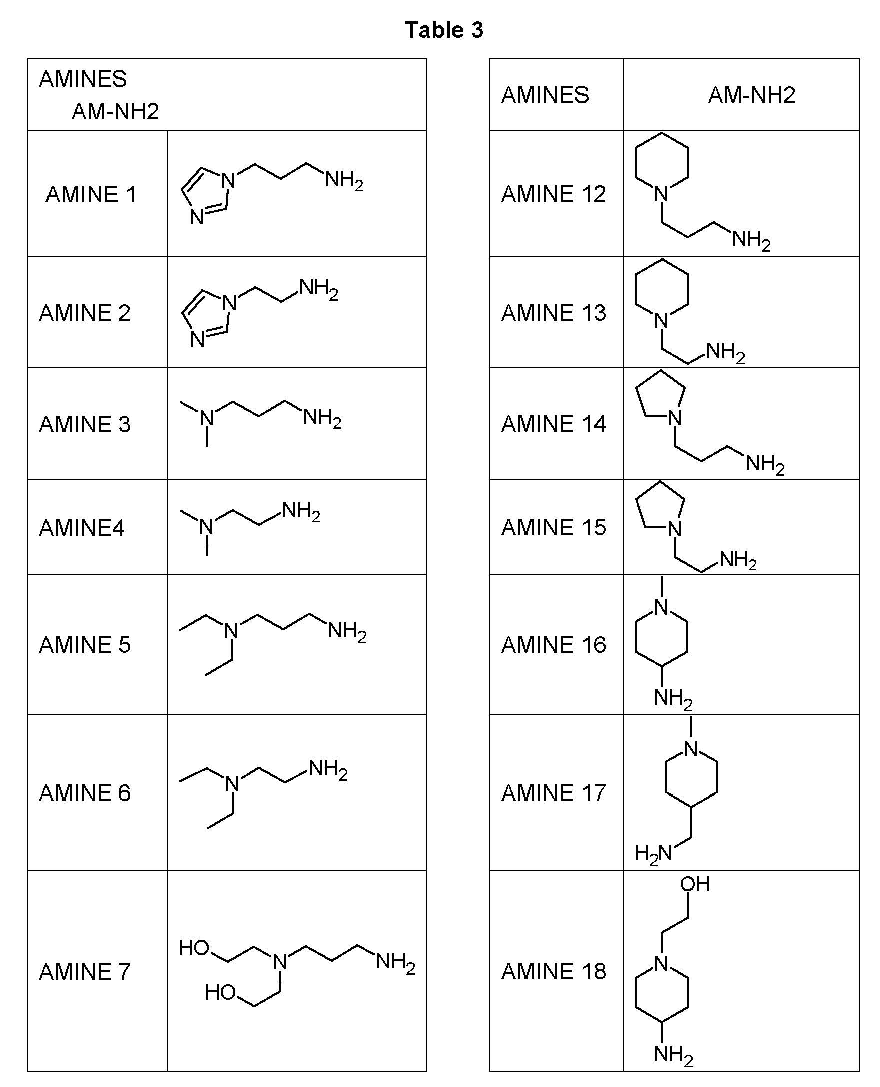

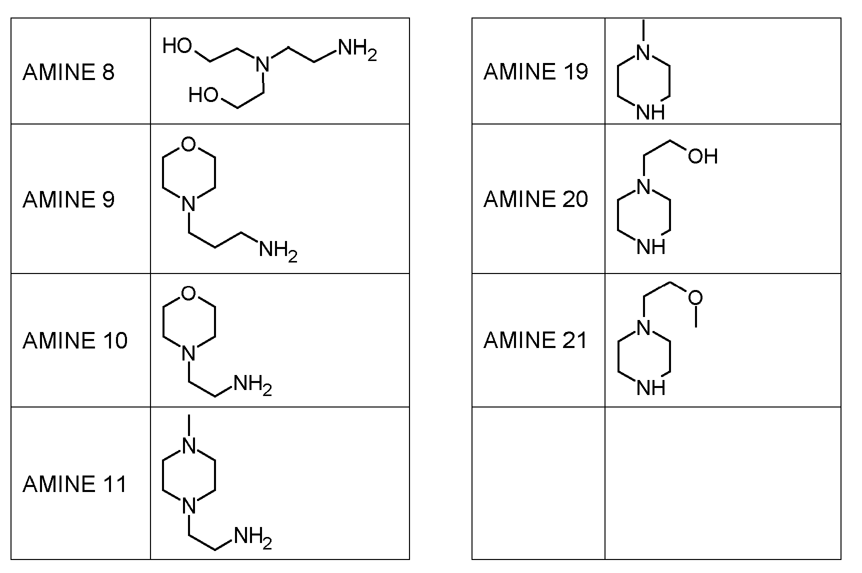

**Table 4**

| | |
|---|---|
| ACRYLATES | |
| 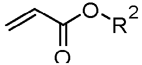 | |
| ACRYLATE 1 | R² = C₆ - C₃₀ |
| ACRYLATE 2 | R² = 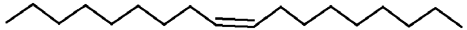 |
| ACRYLATE 3 | R² = 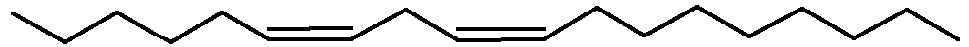 |
| ACRYLATE 4 | R² = CH₃-(CH₂)ₐC≡C-(CH₂)ₐ-, where a and b are 2-7 |

Ionizable lipids of Formula (I) wherein B is an amide moiety and t=q=1, such as, for example, compound of Formula (Ib) or analogous thereof, can be prepared by the synthetic method depicted in scheme 2 below.

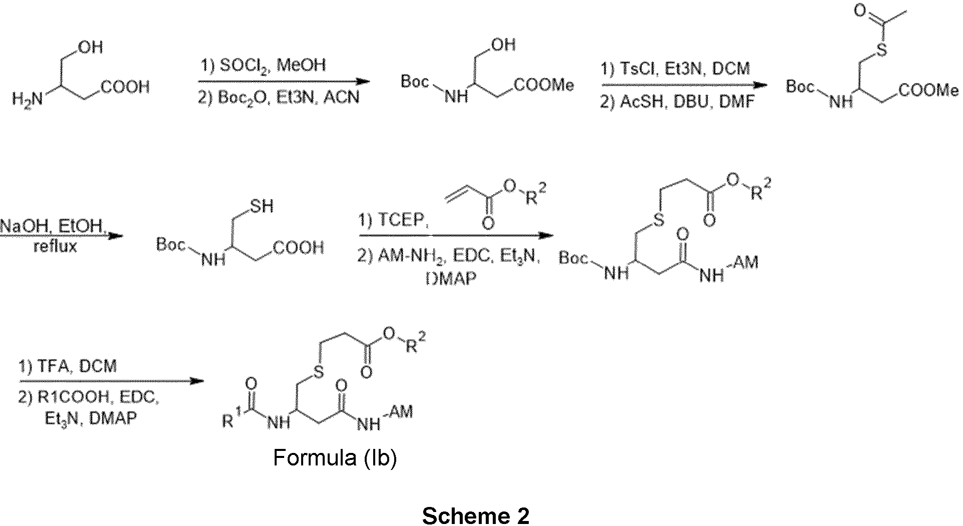

Ionizable lipids of Formula (I) wherein B is an ester moiety, such as, for example, compounds of Formula (Ic) or analogous thereof, or compounds of Formula (Id) or an analogous compound thereof, can be prepared by the synthetic method, or an analogous one, depicted in schemes 3 or 4 below.

Lipid analogues with amide-ester substitution, that is, compounds of Formula (Ic) (wherein B=C, t=0, q=2), and of Formula (Id) (wherein B=C, t=q=1) can be prepared according to the synthetic schemes 3 and 4 below:

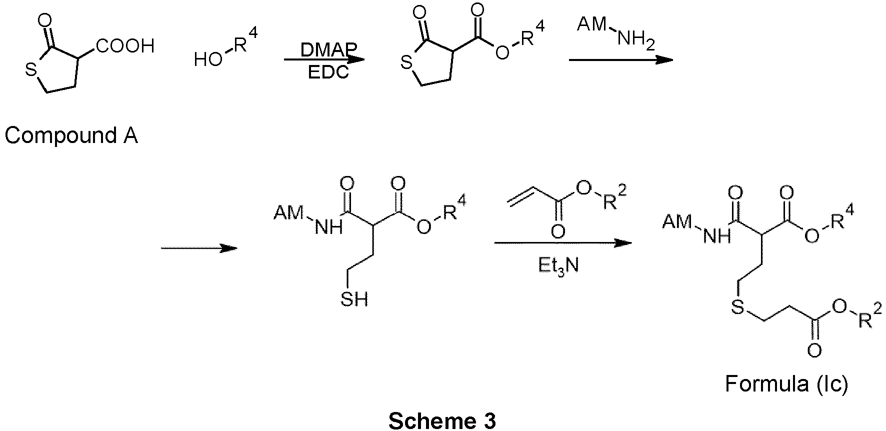

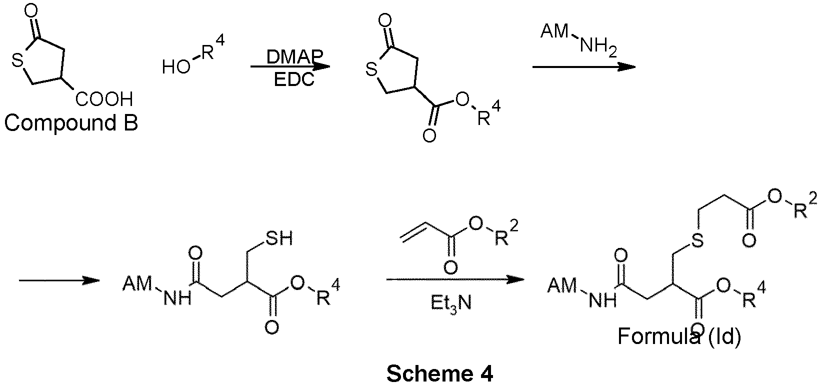

Depending on whether the reaction is performed starting from 2-oxotetrahydrothiophene-3-carboxylic acid (Compound A) or from 5-oxotetrahydrothiophene-3-carboxylic acid (Compound B), two different families of compounds (i.e., compound of Formula (Ic) or compound of Formula (Id), respectively) can be obtained. 2-Oxotetrahydrothiophene-3-carboxylic acid and 5-oxotetrahydrothiophene-3-carboxylic acid can be prepared as described in the literature (cf. Garbiras, B.J.; Marburg, S. "Preparation of Carboxythiolactones and Their Active Derivatives". Synthesis, 1999, vol. 2, pp. 270-274).

The possible alcohols R⁴-OH used for the preparation of compounds of Formula (Ic) and of Formula (Id) according to schemes 3 and 4 are listed in Table 5 below. The amines AM-NH₂ and the acrylate CH₂=CH(CO)OR₂ are the ones listed in Tables 3 and 4 above.

**Table 5**

| **ALCOHOLS** | |
|---|---|
| R⁴-OH | |
| ALCOHOLS 1-11 | 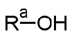 R^{a} = C₁₀ - C₂₀ |
| ALCOHOL 12 | 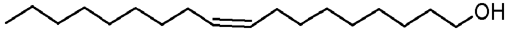 |
| ALCOHOL 13 | 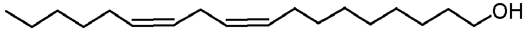 |
| ALCOHOL 14 | R⁴ = CH₃-(CH₂)ₐC≡C-(CH₂)_{b}-, wherein a and b are 2-7 |

### Lipid nanoparticles

As mentioned above, the ionizable lipid of the present disclosure can form LNPs in solution.

Therefore, the present invention also relates to a LNP comprising an ionizable lipid of formula (I) 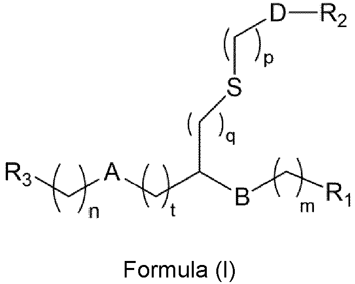
or a pharmaceutically acceptable salt thereof, or a stereoisomer of any one of them,
wherein
A is 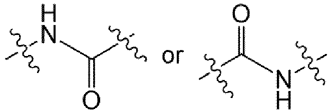
B is 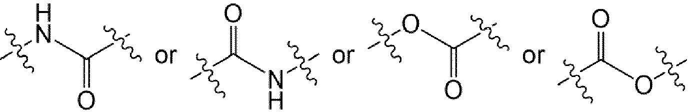
D is 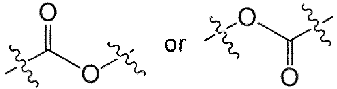
n, m and p are independently 0, 1, 2, 3, 4, 5, or 6;
q is selected from 1 and 2,
t is selected from 0 and 1,
and wherein q + t = 2;
R₁ and R₂ are independently a linear or branched C1-C30 alkyl, C2-C30 alkenyl, C2-C30 alkynyl, wherein R1 and R2 are optionally substituted with one or more substituents selected from the group consisting of -OH, -COOR₄, and (-C(=O)SR₄), wherein R₄ is C1-C6 alkyl, C2-C6 alkenyl or C2-C6 alkynyl,
and wherein
R₃ is a heterocycle comprising at least one N atom, or alternatively R₃ is: 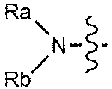
wherein Ra and Rb are independently linear or branched C1-C6 alkyl, optionally substituted with a hydroxyl group.

All embodiments indicated for the ionizable lipid of formula (I) also apply to LNP.

Typically, LNPs have a core-shell structure comprising an inner core and an external shell. LNP formulations with several lipidic components of different nature, such as an ionizable lipid, a sterol, a PEGylated lipid, and a non-cationic lipid (also referred to as "helper lipid") such as a phospholipid, comprise several phases that separate into a hydrophobic core region formed by the ionizable lipid and cholesterol, and a surrounding shell formed by the helper lipid, cholesterol and PEGylated lipids covering the surface. More particularly, the ionizable lipid of formula (I) forms part of the internal shell of the LNP and, optionally, a pharmaceutically active agent is encapsulated or loaded in the inner core.

In an embodiment, optionally in combination with one or more features of the various embodiments described above, the LNP further comprises at least one lipid selected from the group consisting of a non-cationic lipid, a sterol or a steroid precursor, and a PEG-modified lipid.

In another embodiment, optionally in combination with one or more features of the various embodiments described above, the LNP comprises the ionizable lipid of the present disclosure and a non-cationic lipid. Examples of non-cationic lipids include, without being limited to, 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1-stearoyl-2-oleoyl-sn-glycero-3-phosphocoline (SOPC),1,2-dipalmitoyl-sn-glycero-3-phosphocholine (DPPC), 1,2-distearoyl-sn-glycero-3 -phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl- sn -glycero-3-phosphocholine (18:0 Diether PC), 1-oleoyl-2-cholesterylhemisuccinoyl- sn -glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine,1,2-dioleoyl-sn-glycero-3-phosphoetha nolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), sphingomyelin, and mixtures thereof. In an embodiment, the non-cationic lipid is DSPC. In a particular embodiment, the non-cationic lipid is DOPE. In another particular embodiment, the lipid-containing particle comprises DSPC and DOPE.

In another embodiment, optionally in combination with one or more features of the various embodiments described above, the LNP further comprises a sterol or a sterol precursor.

Examples of sterols include, without being limited to, cholesterol, fecosterol, sitosterol, ergosterol, campesterol, stigmasterol, brassicasterol, tomatidine, ursolic acid, alpha-tocopherol, and mixtures thereof. In a particular embodiment, optionally in combination with one or more features of the various embodiments described above, the sterol is cholesterol. Examples of sterol precursors include, without being limited to, a triterpene, a triterpenoid, or a steroid precursor of this kind. Non-limiting examples of triterpenes, triterpenoids and other steroid precursors include squalene, achilleol, polypodatetrane, lanostane, cucurbitacin, hopane, oleanane, chamaecydin, lupine, and mixtures thereof.

In another embodiment, optionally in combination with one or more features of the various embodiments described above, the LNP further comprises a PEG-modified lipid. The term "PEG-modified lipid refers to a lipid comprising a polyethylene moiety. Examples of PEG-modified lipids include, without being limited to, a PEG-modified phosphatidylethanolamine, a PEG-modified phosphatidic acid, a PEG-modified phosphatidylcholine, a PEG-modified ceramide, a PEG-modified dialkylamine, a PEG-modified diacylglycerol, a PEG-modified dialkylglycerol, and mixtures thereof. In some embodiments, the PEG-modified lipid is PEG-DMG, PEG-c-DOMG (also referred to as PEG-DOMG), PEG-DSG, PEG-DPG, or a combination thereof.

In an embodiment, optionally in combination with one or more features of the various embodiments described above, the LNP comprises a lipid component comprising or consisting of the ionizable lipid as disclosed herein, a non-cationic lipid, a sterol, and a PEG-modified lipid.

In a particular embodiment, optionally in combination with one or more features of the various embodiments described above, the LNP comprises a lipid component comprising the ionizable lipid as disclosed herein and at least one of distearolyphosphatidycholine (DSPC), cholesterol, and DMG-PEG2000. In a more particular embodiment, the lipid component comprises or consists of the ionizable lipid as disclosed herein, DSPC, cholesterol, and DMG-PEG2000.

In another embodiment, optionally in combination with one or more features of the various embodiments described above, the LNP comprises from 25 to 60 mol% ionizable lipid, from 0.1 to 10 mol% PEG-modified lipid; from 10 to 45 mol% non-cationic lipid; and from 10 to 40 mol% sterol. Particularly, the LNP comprises from 32 to 50 mol% ionizable lipid, from 1 to 8 mol% PEG-modified lipid; from 12.5 to 42 mol% non-cationic lipid; and from 15 to 38.5 mol% sterol. More particularly, the LNP comprises from 35 to 47 mol% ionizable lipid, from 1 to 4 mol% PEG-modified lipid, from 30 to 38 mol% non-cationic lipid, and from 15 to 25 mol% sterol.

As used herein, "mol%" refers to a component's molar percentage relative to the total mols of all lipid components in the LNP (i.e., total mols of ionizable lipid, PEG-modified lipid; non-cationic lipid; and sterol).

In another embodiment, optionally in combination with one or more features of the various embodiments described above, the molar ratio of ionizable lipid to the non-cationic lipid ranges from 6:1 to 1:2, or from 2:1 to 1:1.

In another embodiment, optionally in combination with one or more features of the various embodiments described above, the molar ratio of ionizable lipid to the sterol ranges from 5:1 to 1:2, or from 2:1 to 1:1.

In another embodiment, optionally in combination with one or more features of the various embodiments described above, the molar ratio of ionizable lipid to the PEG-modified lipid ranges from 120:1 to 2:1, or from 100:1 to 10:1.

### Active agents

As mentioned above, the LNPs of the invention may comprise one or more pharmaceutically active agents.

As used herein, the term "pharmaceutically active agent" refers to an agent that has pharmacological activity and is used for curing, mitigating, treating or preventing a disease in a subject, in particular a human.

For the purposes of the present invention, pharmaceutically active agents include low molecular weight drugs, polynucleotides, peptides, antibodies, proteins, and combinations thereof.

As used herein, the term "polynucleotide" refers to a natural or an artificial deoxyribonucleic acid (DNA) or ribonucleic acid (RNA). The polynucleotide may comprise at least one chemical modification selected from the group consisting of pseudouridine, N1-methylpseudouridine (also referred to as 1-methylpseudouridine or m1Ψ), N6-methyladenosine (also referred to as m6A), 2-thiouridine (also referred to as s2U), 4'-thiouridine, 5-methylcytosine (also referred to 5mC), 2-thio-1-methyl-1-deaza-pseudouridine, 2-thio-1-methyl-pseudouridine, 2-thio-5-aza-uridine, 2-thio-dihydropseudouridine, 2-thio-dihydrouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydropseudouridine, 5-methyluridine (also referred to as m5U), 5-methoxyuridine, 2'-O-methyl uridine, and combinations thereof. In particular, the chemical modification is N1-methylpseudouridine, 5-methoxyuridine or a combination thereof; particularly the chemical modification is N1-methylpseudouridine.

The polynucleotide is partially modified with at least 10%, at least 20%, at least 30%, at 5 least 40%, at least 50%, at least 60%, at least 70%, at least 80%, at least 90%, or at least 99%, of N1-methylpseudouridine or fully modified with N1-methylpseudouridine, 5-methoxyuridine or a combination thereof.

In a particular embodiment, optionally in combination with any of the embodiments provided above, the active agent is selected from the group consisting of a polynucleotide, a DNA construct comprising a promoter operatively linked to a sequence encoding a polynucleotide, and an expression vector comprising a DNA construct comprising a promoter operatively linked to a sequence encoding a polynucleotide.

In a particular embodiment, optionally in combination with any of the embodiments provided above, the polynucleotide is a ribonucleic acid (RNA).

In particular, the RNA is selected from the group consisting of a short interfering RNA (siRNA), a self-replicating RNA (srRNA), circular RNA (circRNA), a self-amplifying RNA (saRNA), an asymmetrical interfering RNA (aiRNA), a microRNA (miRNA), a small interfering RNA (siRNA), a small RNA (sRNA), a Dicer-substrate RNA (dsRNA), a small hairpin RNA (shRNA), a messenger RNA (mRNA), and mixtures thereof.

In a particular embodiment, optionally in combination with any of the embodiments provided above, the RNA is an mRNA.

The skilled person knows how to produce the polynucleotide, the DNA construct, or the expression vector by routine methods well known in the art, for example, by chemical synthesis or by molecular biology techniques, without exercising any inventive skill.

In a particular embodiment, optionally in combination with any of the embodiments provided above, the ionizable lipid to RNA ratio (N/P; were N represent the moles of amine present in the ionizable lipid and P represents the moles of phosphate present in the polynucleotide backbone) in the LNP ranges from 20:1 to 2:1, particularly from 10:1 to 3:1.

In a particular embodiment, optionally in combination with any of the embodiments provided above, the polynucleotide is an isolated artificial polynucleotide.

In an embodiment, optionally in combination with one or more features of the various embodiments described above, the LNP comprises an ionizable lipid as defined herein, a non-cationic lipid, a sterol, a PEG-modified lipid, and a polynucleotide.

The LNPs containing one or more polynucleotides may be prepared by standard methods, for instance, microfluidic mixing as disclosed in Hassett, K. J. et al., ("Optimization of Lipid Nanoparticles for Intramuscular Administration of mRNA Vaccines", 2019, Mol. Ther. Nucleic Acid, vol. 15, pp. 1-11), or by manual/bulk mixing as disclosed in Wang X., Liu S., Sun Y., et al. ("Preparation of selective organ-targeting (SORT) lipid nanoparticles (LNPs) using multiple technical methods for tissue-specific mRNA delivery", 2022, Nat. Protoc., doi:10.1038/s41596-022-00755-x). Both methods are known in the art and the skilled person would know how to proceed in each specific case.

Typically, the process for the preparation of LNPs comprises: i) preparing a first alcoholic mixture comprising the ionizable lipid of the present disclosure and, optionally, at least one lipid selected from the group consisting of a non-cationic lipid, a sterol, and a PEG-modified lipid in a suitable alcohol such as for example ethanol; ii) preparing a second aqueous composition comprising a polynucleotides and an acidification buffer; and iii) mixing i) with ii) in a microfluidic mixer. The microfluidic mixer allows thorough and rapid mixing of the lipid phase and the polynucleotide phase in a microscale device. Depending on the process parameters, and in particular on the total flow rate, the skilled person will be able to modulate the size of the LNPs.

In a particular embodiment, optionally in combination with any of the embodiments provided above, the polynucleotide encodes a polypeptide, particularly, wherein the polypeptide is an antigen. More particularly, the antigen is selected from the group consisting of a viral protein, a bacterial protein, and a tumor-associated antigen.

In a particular embodiment, optionally in combination with any of the embodiments provided above, the polypeptide is an antibody or a fragment thereof. In a more particular embodiment, the antibody or a fragment thereof is a therapeutic antibody or a fragment thereof.

In another embodiment, optionally in combination with any of the embodiments provided above, the antigen is a SARS-CoV-2 antigen, particularly a SARS-CoV-2 spike antigen.

Preparation methods for the above lipids, LNPs and pharmaceutical compositions are described herein and/or known in the art. The skilled in the art would know, depending on the intended use of the composition, which LNP use to encapsulate each pharmaceutically active agent. In particular, methods for the synthesis of the compositions formed by the LNP encapsulating RNA are well-known by the skilled person in the art and duly established in the protocols for molecular biology. Particular conditions are indicated in the examples.

As above indicated, another aspect of the invention relates to a pharmaceutical composition comprising the LNP as defined herein and a pharmaceutically acceptable excipient or carrier.

The expression "pharmaceutically acceptable excipient or carrier" refers to pharmaceutically acceptable materials, compositions, or vehicles. Each component must be pharmaceutically acceptable in the sense of being compatible with the other ingredients of the pharmaceutical composition. It must also be suitable for use in contact with the tissue or organ of humans and non-human animals without excessive toxicity, irritation, allergic response, immunogenicity or other problems or complications commensurate with a reasonable benefit/risk ratio.

Examples of suitable pharmaceutically acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

The relative amounts of the pharmaceutically active ingredient, the pharmaceutically acceptable excipient, and/or any additional ingredients in a pharmaceutical composition of the invention will vary, depending upon the identity, size, and/or condition of the subject treated and further depending upon the route by which the composition is to be administered.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Excipients such as colouring agents, coating agents, sweetening, and flavouring agents can be present in the composition, according to the judgment of the formulator.

In an embodiment, optionally in combination with any of the embodiments provided above, the pharmaceutical composition disclosed herein is administered orally, intranasally, intravenously, intraperitoneally, intramuscularly, intradermally, subcutaneously, topically, or by intra-articular administration.

The pharmaceutical compositions of the present disclosure may be prepared by methodology well known in the pharmaceutical art. For example, a pharmaceutical composition intended to be administered by injection can be prepared by combining the lipid nanoparticles of the invention with sterile, distilled water or other carrier so as to form a solution. Some excipients or carriers can be added to ease the formation of a homogeneous solution or suspension. As mentioned above, the LNP comprising a pharmaceutically active agent, or the pharmaceutical composition of the present disclosure may be used in therapeutic applications. In particular, they may be used as non-viral vectors of general use for biomedical applications, such as vaccines or gene therapy, being effective for transfection of genetic material into eukaryotic cells.

Thus, an aspect of the invention relates to a LNP or a pharmaceutical composition as defined herein for use in a method of treating a disease or disorder in a subject in need thereof, the method comprising administering to the subject a therapeutically effective amount of the nanoparticle composition or of the pharmaceutical composition as defined herein.

Another aspect of the invention relates to a LNP or a pharmaceutical composition as defined herein for use in a method of inducing an immune response in a subject, for use in a method for the therapeutic immunization of a subject, for use as a vaccine, or for use in gene therapy. Particularly, the subject is a human.

In an embodiment, optionally in combination with any of the embodiments provided above, the disease or disorder is selected from the group consisting of infectious diseases, cancer and proliferative diseases, genetic diseases, autoimmune diseases, diabetes, neurodegenerative diseases, cardio- and reno-vascular diseases, and metabolic diseases.

When the pharmaceutically active agent is a polynucleotide, the LNP or the pharmaceutical composition described herein can be used in vaccine therapy, in the enhancement of the efficacy of a conventional vaccine and/or as a novel vaccine form for use against infectious pathogens, such as viruses, bacteria, fungi, protozoa, prions, and helminths (worms); or for use in treating diseases such as cancer and proliferative diseases.

In an embodiment, optionally in combination with any of the embodiments provided above, the pharmaceutical composition is a vaccine. In a more particular embodiment, the pharmaceutical composition is a vaccine and further comprises an adjuvant. The skilled person would know, based on its common general knowledge, which excipients, carriers, and adjuvants to include in the vaccine depending on the intended use.

Throughout the description and claims the word "comprise" and variations of the word, are not intended to exclude other technical features, additives, components, or steps. Furthermore, the word "comprise" encompasses the case of "consisting of".

The following examples and drawings are provided by way of illustration, and they are not intended to be limiting of the present invention. Furthermore, the present invention covers all possible combinations of particular and preferred embodiments described herein.

### Examples

Reagents were purchased from Sigma-Aldrich, TCI Chemicals, Fluorochem, or VWR. All possible wise combinations shown previously were prepared following a three-step reaction protocol.

### Example 1 - Synthesis of lipid VC-LC-0163

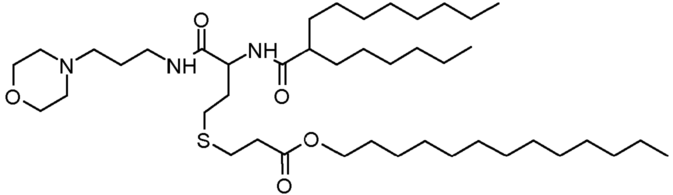

### Step 1.

DL-homocysteine hydrochloride (776 mg, 5 mmol) was dissolved in 13 mL of anhydrous methylene chloride at room temperature. Then triethylamine (588 uL, 4.25 mmol) was added followed by N-(3-dimethylaminopropyl)-N'-ethylcarbodiimide hydrochloride (870 mg, 4.51 mmol), dimethylaminopyridine (104 mg, 0.85 mmol) and 2-hexyldecanoic acid (1.30 g, 4.25 mmol). The reaction mixture was stirred at room temperature overnight, after which it was checked for completion by TLC chromatography. The resulting product was purified by flash chromatography using a gradient of hexane /ethyl acetate (100/0 to 0/100). The fractions containing the product were combined and evaporated under reduced pressure yielding a yellowish oil (1.39 g, 89%).

### Step 2.

The product from step 1 (250 mg, 0.7 mmol) was dissolved in anhydrous tetrahydrofuran (1.5 mL) and was added to a solution of 3-morpholinopropylamine (1.03 mL, 7.03 mmol) in anhydrous tetrahydrofuran (1.0 mL) under nitrogen atmosphere. The solution was then stirred at room temperature for 30 minutes, after which the solvent was evaporated under reduced pressure. The dry mixture was redissolved in ethyl acetate (20 mL) and washed with a solution of 0.1 M HCl (aq) (20 mL, 3 consecutive washes), then distilled water (20 mL, 3 consecutive washes) and finally with saturated brine (20 mL, 3 consecutive washes; NaCl:water prepared by adding 35 g of NaCl in 100 mL of water). The combined organic fractions were dried with MgSO₄ (anh) and subsequently evaporated under reduced pressure to yield a yellowish oil (351 mg, quantitative yield).

### Step 3.

To a solution of the product from step 2 (50 mg, 0.1 mmol) in a 1:1 mixture of tetrahydrofuran:ethanol (400 µL), triethylamine (14 µL, 0.1 mmol) followed by tridecyl acrylate (29 uL, 0.1 mmol) were added. The reaction mixture was stirred overnight at room temperature after which after which it was checked for completion by TLC chromatography. The resulting product was purified by column chromatography using a gradient of dichloromethane/dichloromethane:methanol: Ammonium hydroxide (80:20:1) (100/0 to 0/100). The fractions containing the product were combined and evaporated under reduced pressure yielding VC-LC-0163 as a yellowish oil (64 mg, 78%).

VC-LC-0163 was characterised by HPLC-LSD-MS (HPLC Water Alliance ELSD 2424 and column X bridge BEH C8, 4.6 mm, 50 mm, 2.5 µm, gradient 5% to 95% B (A: Water with 0.01% TFA; B: Acetonitrile with 0.01% TFA) for 20 min, rate = 1 mL/min).

The injection volume is 2 µL and the temperature in the column is 65 °C.

Successful detection of the analyte was performed by an evaporative light dispersion detector and by a simple quadrupole mass detector in positive mode with a spectrophotometer Waters Acquity QDa.

Retention time: 15.76 minutes, MS (ES): experimental m/z [M+H]+ 754.82 {theoretical m/z [M+H]+ 754,61}.

### Example 2 - Synthesis of lipid VC-LC-0605

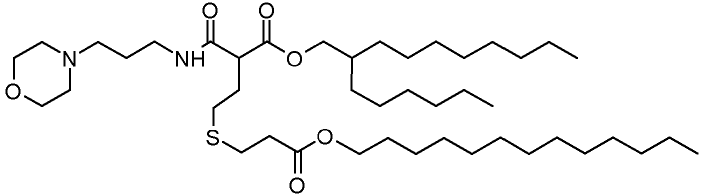

### Step 1.

2-Oxotetrahydrothiophene-3-carboxylic acid (730 mg, 5 mmol) was dissolved in 13 mL of anhydrous methylene chloride at room temperature. Then N-(3-dimethylaminopropyl)-N'-ethylcarbodiimide hydrochloride (870 mg, 4.51 mmol) was added followed by, dimethylaminopyridine (104 mg, 0.85 mmol) and 2-hexyl-1-decanol (1.03 g, 4.25 mmol). The reaction was stirred at room temperature overnight, after which it was checked for completion by TLC chromatography. The resulting product was purified by flash chromatography using a gradient of hexane /ethyl acetate (100/0 to 0/100). The fractions containing the product were combined and evaporated under reduced pressure yielding a yellowish oil (1.33 g, 84%).

In order to obtain VC-LC-0605, steps 2 and 3 as in Example 1 were carried out (41% yield).

VC-LC-0605 was characterised by HPLC-LSD-MS (HPLC Water Alliance ELSD 2424 and column X bridge BEH C8, 4.6 mm, 50 mm, 2.5 µm, gradient 5% to 95% B (A: Water with 0.01% TFA; B: Acetonitrile with 0.01% TFA) for 20 min, rate = 1 mL/min.

The injection volume is 2 µL and the temperature in the column is 65 °C.

Successful detection of the analyte was performed by an evaporative light dispersion detector and by a simple quadrupole mass detector in positive mode with an spectrophotometer Waters Acquity QDa.

Retention time: 16.99 minutes, MS (ES): experimental m/z (MH+) 769.61 {theoretical m/z [M+H]+ 769,79}.

### Example 3 - Synthesis of lipid VC-LC-0540

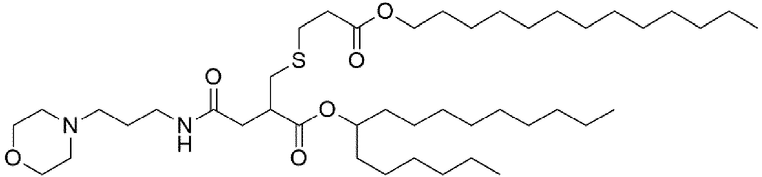

In order to obtain lipid VC-LC-0540 the process of Example 2 was carried out but starting from 5-oxotetrahydrothiophene-3-carboxylic acid instead of 2-oxotetrahydrothiophene-3-carboxylic acid (35% yield).

VC-LC-0540 was characterised by HPLC-LSD-MS (HPLC Water Alliance ELSD 2424 and column X bridge BEH C8, 4.6 mm, 50 mm, 2.5 µm, gradient 5% to 95% acetonitrile B (A: Water with 0.01% TFA; B: Acetonitrile with 0.01% TFA) for 20 min, rate = 1 mL/min.

The injection volume is 2 µL and the temperature in the column is 65 °C.

Successful detection of the analyte was performed by an evaporative light dispersion detector and by a simple quadrupole mass detector in positive mode with an spectrophotometer Waters Acquity QDa.

Retention time: 17.16 minutes, MS (ES): experimental m/z (MH+) 783.63 {theoretical m/z [M+H]+ 783,80}.

### Example 4 - Preparation and characterisation of LNPs comprising the compounds of the present disclosure.

### RNA encapsulation into LNPs

For in vivo administration, mRNA comprising in the 5' to 3' direction a sequence encoding for luciferase of SEQ ID NO:1, was encapsulated into LNPs following the procedure described in Hassett, K. J. et al., "Optimization of Lipid Nanoparticles for Intramuscular Administration of mRNA Vaccines", 2019, Mol. Ther. Nucleic Acid, vol. 15, pp. 1-11. Briefly, the purified mRNAs were first diluted in sodium citrate buffer at pH=4 at a final concentration of 266 µg/ml. Separately, a mixture of the ionizable lipid of the invention:DSPC (Merk 850365P):cholesterol (Sigma C3045):DMG-PEG2000 (Cayman 33945-1) were dissolved into ethanol at the respective molar percentages of 50:10:38.5:1.5, and with a lipid-nitrogen-to-phosphate ratio (N:P) ratio of 5,5:1.

The mRNA aqueous solution was then carefully added to the ethanol solution, and the resulted solution was homogenized by pipetting up and down for 4-5 times. The resulting LNPs were immediately diluted 1:1 with Tris buffer and dialyzed overnight against Tris buffer containing 15% sucrose. The resulting LNP solution was then collected, and the encapsulated mRNA was assessed by Quant-IT^{®} Ribogreen (Invitrogen R11490) following the manufacturer's instructions.

The LNP solution was then adjusted to a final concentration of mRNA of 100 µg/ml. Size distribution, polydispersity and Z-potential were measured by dynamic light scattering (DLS) using Malvern Zetasizer Advance Lab Blue Label. RNA encapsulation was assessed by Quant-IT^{®} Ribogreen following the manufacturer's instructions.

Finally, the LNP solution was passed through a 0.22 mm filter and the LNPs were stored at -80 °C until required.

Table 6 provides the results of several parameters for LNPs comprising the specific ionizable lipids shown therein in the following standard formulation:
ionizable lipid:non-cationic lipid:sterol:PEG-modified lipid → 50:10:38.5:1.5.

**Table 6**

| REFERENCE | Encaps. efficiency (%) | Particle size/ Ø (nm) | P.D.I. | Z Potential (± D.E.) [mV] | Apparent pKa |
|---|---|---|---|---|---|
| VC-LC-0099 | 98.48 | - | - | 0.00 | - |
| VC-LC-0101 | 96.01 | - | - | 0.00 | - |
| VC-LC-0103 | 100.00 | - | - | 0.00 | - |
| VC-LC-0104 | 100.00 | - | - | 0.00 | - |
| VC-LC-0106 | 98.70 | - | - | 0.00 | - |
| VC-LC-0107 | 100.00 | - | - | 0.00 | - |
| VC-LC-0141 | 95.10 | - | - | 0.00 | - |
| VC-LC-0163 | 77.59 | 191.2 | 0.23 | -21.28 | - |
| VC-LC-0165 | 45.16 | 181.9 | 0.21 | -26.69 | - |
| VC-LC-0166 | 42.53 | 164.8 | 0.11 | -24.55 | - |
| VC-LC-0167 | 34.31 | 200.6 | 0.22 | -22.30 | - |
| VC-LC-0168 | 58.76 | 198.1 | 0.16 | -23.72 | - |
| VC-LC-0169 | 33.80 | 158.2 | 0.14 | -25.92 | - |
| VC-LC-0170 | 56.36 | 266.4 | - | -22.79 | 5.58 |
| VC-LC-0171 | 53.65 | 228.8 | 0.24 | -23.07 | - |
| VC-LC-0173 | 52.08 | - | - | -30.51 | 5.33 |
| VC-LC-0175 | 23.97 | 120 | 0.09 | -15.84 | - |
| VC-LC-0176 | 47.36 | - | - | -17.58 | 5.77 |
| VC-LC-0178 | 65.40 | 196.3 | 0.20 | -6.81 | - |
| VC-LC-0180 | 87.95 | 197.5 | 0.19 | -4.28 | - |
| VC-LC-0181 | 85.48 | 206.7 | 0.35 | -6.47 | - |
| VC-LC-0182 | 91.18 | 217.1 | 0.22 | -2.72 | - |
| VC-LC-0183 | 92.40 | 186.8 | 0.15 | -1.64 | - |
| VC-LC-0185 | 70.97 | - | - | -1.54 | 5.85 |
| VC-LC-0186 | 86.70 | 158.7 | 0.18 | 3.38 | - |
| VC-LC-0191 | 87.02 | 135.2 | 0.11 | 4.77 | - |
| VC-LC-0194 | 80.78 | - | - | 0.00 | - |
| VC-LC-0196 | 92.20 | - | - | 0.00 | - |
| VC-LC-0197 | 90.54 | 262.2 | 0.22 | 1.39 | - |
| VC-LC-0198 | 90.20 | 227.4 | 0.29 | -2.88 | - |
| VC-LC-0199 | 96.01 | 194.1 | 0.16 | -1.59 | - |
| VC-LC-0200 | 92.38 | 184.2 | 0.10 | -2.57 | - |
| VC-LC-0201 | 86.70 | 259.7 | - | 2.80 | 6.76 |
| VC-LC-0202 | 89.61 | 171.7 | 0.13 | 0.80 | - |
| VC-LC-0204 | 79.19 | 244.2 | 0.35 | -4.77 | - |
| VC-LC-0206 | 82.26 | 179.9 | 0.17 | -2.86 | 7.30 |
| VC-LC-0207 | 93.61 | 185.7 | 0.2 | -0.83 | 7.18 |
| VC-LC-0209 | 48.12 | 148.7 | 0.11 | -4.98 | - |
| VC-LC-0211 | 67.87 | 181 | 0.14 | -4.59 | 6.68 |
| VC-LC-0212 | 83.35 | 179 | 0.26 | -20.85 | - |
| VC-LC-0213 | 64.58 | 221.3 | 0.19 | 4.48 | - |
| VC-LC-0214 | 87.16 | 174.1 | 0.11 | -0.90 | - |
| VC-LC-0215 | 58.34 | 143.5 | - | -13.70 | 6.25 |
| VC-LC-0216 | 52.13 | - | 0.35 | -20.53 | 6.36 |
| VC-LC-0217 | 72.79 | 156.7 | 0.14 | -2.41 | - |
| VC-LC-0241 | 62.91 | 177.4 | 0.11 | -2.52 | - |
| VC-LC-0243 | 93.35 | 197.6 | 0.18 | 2.43 | - |
| VC-LC-0244 | 53.57 | 175 | 0.16 | 1.09 | - |
| VC-LC-0245 | 94.64 | 177.3 | 0.16 | 5.96 | - |
| VC-LC-0248 | 89.76 | 248 | 0.35 | 4.52 | 7.24 |
| VC-LC-0254 | 80.00 | 168.7 | 0.20 | 7.01 | - |
| VC-LC-0256 | 92.30 | 184.3 | 0.12 | -15.91 | 5.91 |
| VC-LC-0258 | 73.31 | 166.3 | 0.13 | -9.74 | - |
| VC-LC-0259 | 41.89 | 138.8 | 0.14 | -8.47 | 6.21 |
| VC-LC-0260 | 44.25 | 161.7 | 0.18 | -17.24 | - |
| VC-LC-0261 | 58.07 | 187.3 | 0.13 | -12.63 | - |
| VC-LC-0262 | 33.79 | 143.8 | 0.11 | -10.53 | - |
| VC-LC-0263 | 60.70 | 188.6 | 0.13 | -17.30 | 5.83 |
| VC-LC-0264 | 38.96 | 147 | 0.15 | -8.30 | - |
| VC-LC-0268 | 29.52 | 173 | 0.14 | -10.85 | 6.18 |
| VC-LC-0269 | 36.60 | 176.7 | 0.15 | -10.28 | 5.84 |
| VC-LC-0289 | 89.26 | 207.1 | 0.2 | -0.34 | 6.25 |
| VC-LC-0290 | 56.36 | 266.4 | - | -22.79 | 5.58 |
| VC-LC-0292 | 94.63 | 177.7 | 0.18 | 7.79 | 7.06 |
| VC-LC-0293 | 92.48 | 178.6 | 0.13 | 12.29 | 7.36 |
| VC-LC-0294 | 65.74 | 160.7 | 0.09 | -10.37 | 5.77 |
| VC-LC-0296 | 48.51 | 175.8 | 0.13 | -7.19 | 5.62 |
| VC-LC-0297 | 46.94 | 193.3 | 0.18 | -11.77 | 5.80 |
| VC-LC-0298 | 59.92 | 190.5 | 0.16 | -13.51 | 6.06 |
| VC-LC-0299 | 57.64 | 193.3 | 0.15 | -13.45 | 6.09 |
| VC-LC-0300 | 36.51 | 156.9 | 0.17 | -9.75 | - |
| VC-LC-0301 | 46.00 | 131.6 | 0.13 | -10.54 | - |
| VC-LC-0302 | 40.58 | 112.3 | 0.13 | -9.30 | - |
| VC-LC-0304 | 54.05 | - | 0.26 | -23.20 | 6.02 |
| VC-LC-0306 | 45.62 | 184.2 | 0.09 | -12.78 | 5.70 |
| VC-LC-0307 | 43.89 | 178 | 0.14 | -15.15 | 6.11 |
| VC-LC-0314 | 90.53 | 201.7 | 0.16 | 2.76 | - |
| VC-LC-0322 | 97.01 | 220.5 | 0.17 | 2.31 | 6.61 |
| VC-LC-0326 | 99.06 | 181.9 | 0.25 | 7.00 | 6.00 |
| VC-LC-0328 | 99.45 | 216.5 | 0.2 | 7.09 | - |
| VC-LC-0329 | 97.13 | 211 | 0.22 | 7.99 | - |
| VC-LC-0338 | 62.80 | 146.3 | 0.14 | -5.38 | 6.64 |
| VC-LC-0353 | 57.26 | 175.8 | 0.1 | -8.97 | 6.29 |
| VC-LC-0354 | 68.28 | 234.3 | 0.13 | -0.57 | 7.01 |
| VC-LC-0355 | 81.58 | 242.2 | 0.3 | -4.71 | 6.59 |
| VC-LC-0356 | 68.27 | 218.9 | 0.09 | -10.96 | 5.84 |
| VC-LC-0361 | 41.05 | 169.3 | 0.18 | -12.18 | 5.29 |
| VC-LC-0362 | 73.55 | 121.1 | 0.14 | -13.94 | 6.00 |
| VC-LC-0363 | 63.87 | 143.9 | 0.18 | -17.92 | 6.36 |
| VC-LC-0364 | 78.29 | 116.8 | 0.13 | -14.33 | 6.38 |
| VC-LC-0365 | 66.86 | 124.1 | 0.24 | -17.43 | 6.33 |
| VC-LC-0366 | 89.18 | 122.6 | 0.16 | -13.24 | 6.33 |
| VC-LC-0367 | 47.47 | 134.6 | 0.19 | -15.48 | 6.05 |
| VC-LC-0368 | 89.08 | 140.9 | 0.1 | -15.81 | 5.98 |
| VC-LC-0369 | 92.05 | 137.6 | 0.15 | -10.72 | 6.02 |
| VC-LC-0370 | 62.48 | 134.7 | 0.21 | -7.08 | 6.23 |
| VC-LC-0374 | 70.37 | 174.7 | 0.16 | -9.62 | 6.44 |
| VC-LC-0380 | 71.74 | 170.6 | 0.25 | -17.08 | 5.31 |
| VC-LC-0389 | 76.40 | 218 | 0.26 | -9.68 | 6.10 |
| VC-LC-0390 | 83.80 | 192.8 | 0.24 | -1.14 | 6.10 |
| VC-LC-0392 | 40.58 | 136.1 | 0.19 | -11.14 | 4.72 |
| VC-LC-0407 | 60.22 | 167.1 | 0.1 | -12.36 | 4.66 |
| VC-LC-0408 | 41.23 | 168.1 | 0.25 | -15.07 | 4.83 |
| VC-LC-0428 | 65.37 | 185.3 | 0.11 | -8.34 | 5.84 |
| VC-LC-0429 | 46.96 | 155.6 | 0.14 | -12.49 | 6.48 |
| VC-LC-0430 | 84.48 | 216.2 | 0.23 | 1.65 | 7.00 |
| VC-LC-0431 | 60.4 | 196.6 | 0.11 | -0.28 | 5.77 |
| VC-LC-0432 | 46.27 | 126.9 | 0.29 | -16.42 | 6.44 |
| VC-LC-0433 | 96.77 | 180.8 | 0.13 | 4.87 | 7.75 |
| VC-LC-0434 | 99.09 | 214.6 | 0.2483 | 1.34 | 5.72 |
| VC-LC-0435 | 99.22 | 182.6 | 0.2 | 8.58 | 7.25 |
| VC-LC-0437 | 99.92 | 152 | 0.23 | 7.76 | 7.02 |
| VC-LC-0438 | 95.26 | 181.3 | 0.15 | 7.19 | 6.61 |
| VC-LC-0439 | 91.14 | 144.8 | 0.1 | -6.48 | 5.39 |
| VC-LC-0440 | 76.52 | 167.4 | 0.10 | -3.61 | 6.75 |
| VC-LC-0441 | 78.30 | 186.5 | 0.12 | -4.04 | 6.40 |
| VC-LC-0442 | 87.80 | 171.8 | 0.12 | -4.19 | 6.47 |
| VC-LC-0443 | 86.01 | 181.3 | 0.13 | -2.92 | 6.73 |
| VC-LC-0444 | 85.85 | 185.7 | 0.07 | -3.35 | 6.28 |
| VC-LC-0445 | 48.02 | - | - | -10.64 | 5.82 |
| VC-LC-0473 | 43.07 | 134.0 | 0.085 | -7.78 | 6.73 |
| VC-LC-0474 | 66.27 | 153.2 | 0.21 | -7.69 | 5.61 |
| VC-LC-0475 | 52.31 | 135 | 0.13 | -4.72 | 6.59 |
| VC-LC-0477 | 57.43 | 146.7 | 0.15 | -9.55 | 6.65 |
| VC-LC0478 | 65.16 | 156.2 | 0.17 | -7.01 | 6.82 |
| VC-LC-0480 | 73.32 | 154.5 | 0.2 | -6.33 | 6.37 |
| VC-LC-0487 | 62.43 | 156.3 | 0.17 | -9.59 | 6.53 |
| VC-LC-0488 | 62.45 | 149.1 | 0.16 | -4.15 | 6.79 |
| VC-LC-0489 | 60.57 | 143.8 | 0.13 | -5.88 | 6.03 |
| VC-LC-0490 | 97.16 | 136.1 | 0.18 | 0.83 | 6.86 |
| VC-LC-0491 | 72.06 | 160.8 | 0.17 | -3.40 | 7.07 |
| VC-LC-0492 | 98.15 | 150.9 | 0.16 | 0.83 | 7.47 |
| VC-LC-0493 | 98.56 | 128.8 | 0.12 | -0.57 | 6.46 |
| VC-LC-0494 | 98.99 | 169.8 | 0.22 | 5.52 | 6.86 |
| VC-LC-0498 | 99.46 | 152.3 | 0.15 | 1.06 | 7.47 |
| VC-LC-0504 | 94.39 | 168.7 | 0.074 | -1.96 | - |
| VC-LC-0505 | 99.88 | 168.6 | - | -2.94 | 6.74 |
| VC-LC-0507 | 95.15 | 151.1 | 0.13 | -2.76 | 6.56 |
| VC-LC-0508 | 91.49 | 152.6 | 0.13 | -2.43 | 7.48 |
| VC-LC-0509 | 98.62 | 170.1 | 0.23 | 0.25 | 7.60 |
| VC-LC-0510 | 97.61 | 144.9 | 0.15 | -2.73 | 7.39 |
| VC-LC-0511 | 98.97 | 155.6 | 0.14 | 4.06 | 6.85 |
| VC-LC-0515 | 98.61 | 141.7 | 0.12 | 1.90 | 7.26 |
| VC-LC-0518 | 97.70 | 149.1 | 0.17 | 3.90 | 6.67 |
| VC-LC-0519 | 97.15 | 137.9 | 0.16 | -1.93 | 6.69 |
| VC-LC-0521 | 52.01 | 153.1 | 0.071 | -4.50 | 6.06 |
| VC-LC-0523 | 95.57 | 169.3 | 0.17 | 5.29 | 7.32 |
| VC-LC-0524 | 97.01 | 129.9 | 0.15 | 3.83 | 7.85 |
| VC-LC-0525 | 59.89 | 151 | 0.18 | -5.11 | 6.67 |
| VC-LC-0526 | 97.90 | 124.7 | 0.16 | 3.68 | 7.92 |
| VC-LC-0528 | 98.83 | 137.4 | 0.2 | 4.55 | 7.01 |
| VC-LC-0530 | 97.41 | 164 | 0.26 | 7.65 | 7.88 |
| VC-LC-0531 | 98.54 | 158.6 | 0.19 | 9.15 | 8.06 |
| VC-LC-0532 | 99.89 | 137.8 | 0.15 | 8.05 | 8.11 |
| VC-LC-0533 | 98.35 | 178.1 | 0.19 | 5.74 | 8.0 |
| VC-LC-0535 | 96.61 | 144.7 | 0.11 | 4.45 | 7.87 |
| VC-LC-0537 | 69.42 | 165.1 | 0.14 | -8.81 | 5.23 |
| VC-LC-0538 | 96.86 | 153.4 | 0.16 | 5.13 | 8.25 |
| VC-LC-0539 | 85.33 | 176.7 | 0.13 | -1.24 | 6.90 |
| VC-LC-0540 | 43.65 | 140.3 | 0.11 | -8.47 | 6.21 |
| VC-LC-0541 | 42.80 | 154.7 | 0.11 | -6.83 | 6.39 |
| VC-LC-0542 | 33.76 | 156.3 | 0.14 | -10.60 | 6.43 |
| VC-LC-0543 | 31.75 | 155.7 | 0.14 | -10.98 | 6.61 |
| VC-LC-0544 | 36.86 | 140.3 | 0.12 | -11.02 | 6.53 |
| VC-LC-0546 | 39.62 | 164.2 | 0.14 | -10.43 | 6.64 |
| VC-LC-0547 | 31.52 | 168 | 0.18 | -13.52 | 6.82 |
| VC-LC-0548 | 30.22 | 145.5 | 0.17 | -8.82 | 6.64 |
| VC-LC-0549 | 36.38 | 194.9 | 0.27 | -16.95 | 5.03 |
| VC-LC-0550 | 26.96 | 173.7 | 0.16 | -14.52 | 6.53 |
| VC-LC-0551 | 29.06 | 155.3 | 0.15 | -11.66 | 6.51 |
| VC-LC-0553 | 36.11 | 196.2 | 0.22 | -8.66 | 6.39 |
| VC-LC-0554 | 29.19 | 158.4 | 0.15 | -9.33 | 6.45 |
| VC-LC-0556 | 54.53 | 156.7 | 0.14 | -7.57 | 5.83 |
| VC-LC-0557 | 77.19 | 142.5 | 0.11 | -6.12 | 5.81 |
| VC-LC-0558 | 50.58 | 155.2 | 0.11 | -6.62 | 5.95 |
| VC-LC-0559 | 65.25 | 138.8 | 0.14 | -5.71 | 6.40 |
| VC-LC-0566 | 55.25 | 149.0 | 0.13 | -3.35 | 6.57 |
| VC-LC-0577 | 55.61 | 182.2 | 0.13 | -15.06 | 5.69 |
| VC-LC-0587 | 38.50 | 150.3 | 0.12 | -6.62 | 5.54 |
| VC-LC-0605 | 52.30 | 136.3 | 0.24 | -11.79 | 5.68 |
| VC-LC-0606 | 51.46 | 168.0 | 0.18 | -9.73 | 5.64 |
| VC-LC-0607 | 49.38 | 148.0 | 0.17 | -11.81 | 5.89 |
| VC-LC-0608 | 50.40 | 179.2 | 0.20 | -11.14 | 5.88 |
| VC-LC-0609 | 54.21 | 168.6 | 0.16 | -20.42 | 5.77 |
| VC-LC-0610 | 55.68 | 168.1 | 0.13 | -21.04 | 5.93 |

As shown in Table 6, each of the compounds tested had an acceptable particle size for the purpose sought. The encapsulation efficiency, measured as the percentage of RNA entrapped in the LNP, ranged from 100% to approximately 27%. Nevertheless, this is only an indicative of how easily the components of the nanoparticle interact between them to form a LNP able to entrap RNA. It does not indicate the intracellular transfection efficiency, which is measured here as luminescence given as total flux of p·s⁻¹.

As an illustrative example, LNPs comprising compound VC-LC-0550 show an encapsulation efficiency of 26.96%, but the total flux measured is 3,4·10⁷ p·s⁻¹. This means that approximately 27% of the RNA was encapsulated when it was contacted with the components of the LNP. The value of the total flux observed indicated that the LNPs were not immediately degraded *in vivo* after injection, and that transfection of the cells was indeed very successful, hence the contents encapsulated inside the LNPs effectively reached the cytosol after the endosomal escape.

### Example 5. Protein expression in mice muscle administered with selected examples of LNPs prepared with the lipids of the invention and containing RNA as active ingredient.

### Administration of the mRNA (LNP) to mice

Female BALB/c mice (Charles River Laboratories), 8-10-week-old, weighting 18-23 g, were acclimatized to new conditions upon arrival to experimental facilities for 3-7 days. Housing conditions were room temperature 20-24 °C, humidity 50-70 %, and light intensity 60 lux with a light-dark cycle of 12 hours.

For Firefly Luciferase activity measurement in mice, LNPs produced as indicated above (ionizable lipid:helper lipid: sterol:PEG-modified lipid → 50:10:38.5:1.5) and containing 5 µg of the indicated mRNA in 50 µl final volume, were injected intramuscularly.

From 4 to 72 hours after RNA-LPN inoculation mice were anaesthetized by inhalation with 4% of isoflurane using a vaporizer. The maintenance of the anaesthesia was performed at 1.5% of isoflurane. Then, D-luciferin (Quimigen, 12507) was injected intraperitoneally at 150 mg/kg, normally ~200 µL of the stock at 15 mg/mL in PBS for a 20 g mouse. Luciferase images were acquired 10 minutes after luciferin inoculation using the IVIS Lumina XRMS Imaging System following manufacturer's instructions.

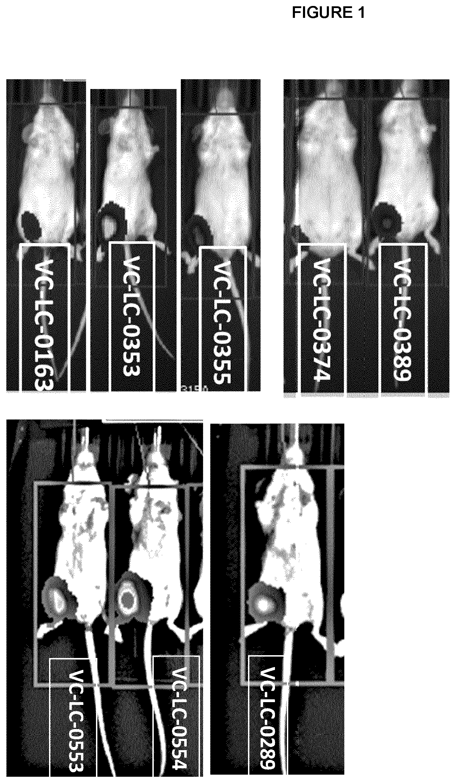

Table 7(A-C) below shows selected examples of LNPs comprising ionizable lipids of Formula (I) comprising the same polar head (morpholino) and different combinations of R2 and R3 in order to prove that the general structure of Formula (I) produces the desired effect independently of the alkyl residues. Figure 1 depicts protein expression in mice of compounds depicted in Table 7(A-C).

**Table 7A**

| Chain 1 (R1) | LINEAR SATURATED | |
|---|---|---|
| Chain 2 (R2) | Saturated | Branched |
| (R3) morpholine | 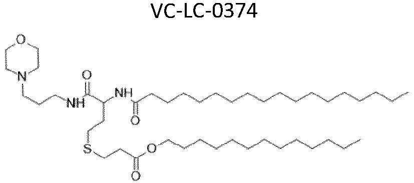 | 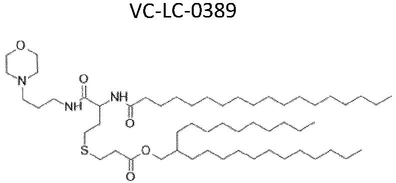 |
| | Total flux: 3,50E+06 | Total flux: 1,70E+07 |

**Table 7B**

| Chain 1 (R1) | BRANCHED | | |
|---|---|---|---|
| Chain 2 (R2) | Saturated | Branched | Unsaturated |
| (R3) morpholine | 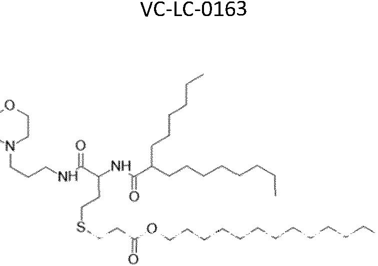 | 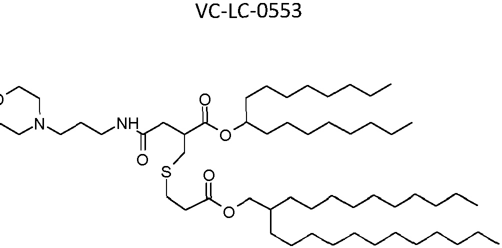 | 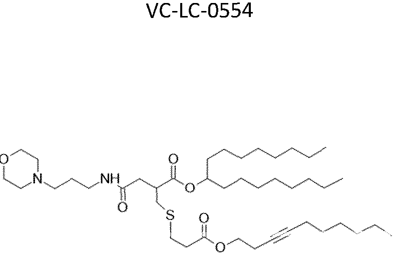 |
| | Total flux: 5,22E+07 | Total flux: 4,96E+07 | Total flux: 1,4E+08 |

**Table 7C**

| Chain 1 (R1) | LINEAR UNSATURATED | | |
|---|---|---|---|
| Chain 2 (R2) | Saturated | Branched | Unsaturated |
| (R3) morpholine or -N(EtOH)₂ | 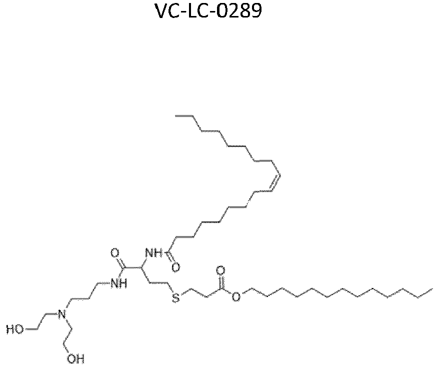 | 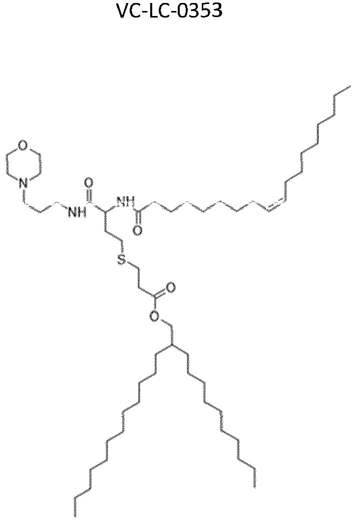 | 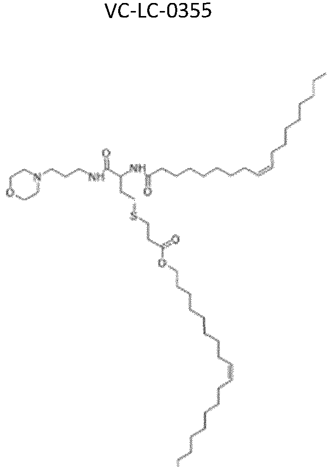 |
| | Total flux: 2,3E+07 | Total flux: 5,20E+07 | Total flux: 2,39E+07 |

Table 8 shows selected examples of LNPs comprising ionizable lipids of Formula (I) comprising a variety of R3 substituents (polar head) as well as different combinations of R2 and R3 in order to prove that the general structure of Formula (I) produces the desired effect independently of substituents R1, R2 and the selected polar head (R3).

**Table 8**

| REF. | Total Flux | Structure | Encaps. efficiency | Mean Ø (nm) |
|---|---|---|---|---|
| VC-LC-0258 | 5.5E+07 | 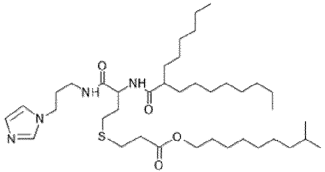 | 73.31 | - |
| VC-LC-0163 | 5.2E+07 | 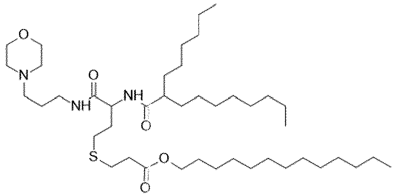 | 77.59 | - |
| VC-LC-0194 | 6.2E+07 | 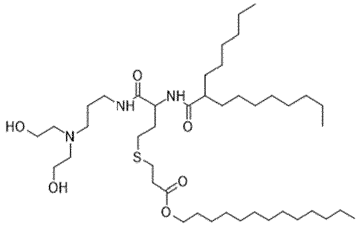 | 80.78 | - |
| VC-LC-0298 | 7.6E+07 | 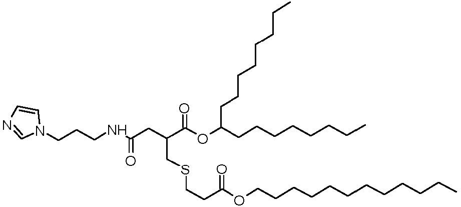 | 59.92 | 190.5 |
| VC-LC-0353 | 5.2E+07 | 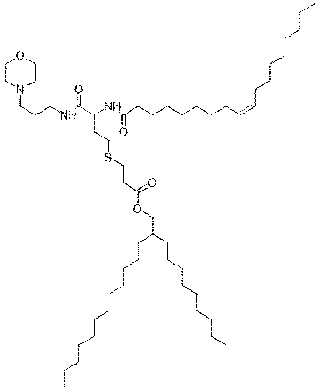 | 57.26 | 175.8 |
| VC-LC-443 | 8.5E+07 | 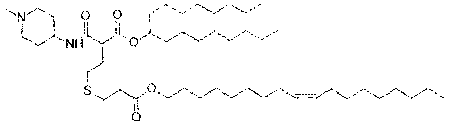 | 86.01 | 181.3 |
| VC-LC-0487 | 6.9E+07 | 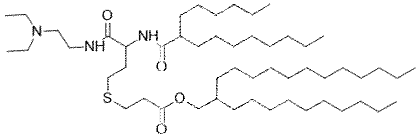 | 62.43 | 156.3 |
| VC-LC-0296 | 4.7E+07 | 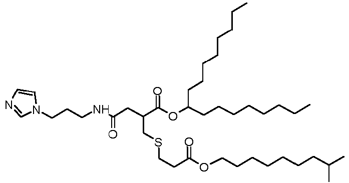 | 48.5 | 175.8 |
| VC-LC-0307 | 1.47E+08 | 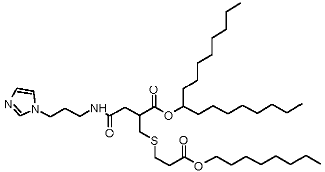 | 43.9 | 178 |
| VC-LC-0362 | 3.31E+07 | 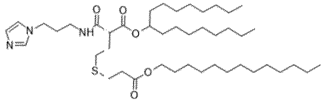 | 73.5 | 121.1 |
| VC-LC-0367 | 3.16E+07 | 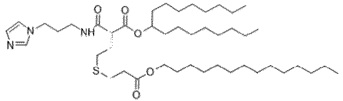 | 47.5 | 134.6 |
| VC-LC-302 | 7.9E+07 | 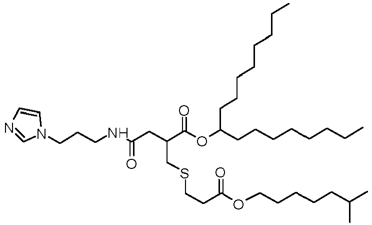 | 40.6 | 112.3 |
| VC-LC-0297 | 2.3E+07 | 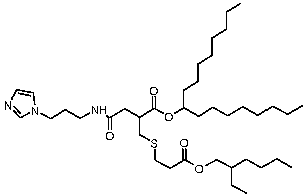 | 46.9 | 193.3 |
| VC-LC-0262 | 7.5E+07 | 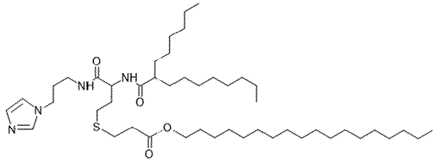 | 33.79 | 143.8 |
| VC-LC-0241 | 1.928E+07 | 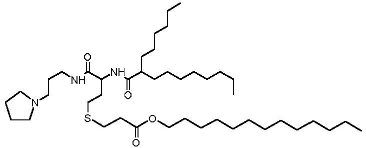 | 62.9 | - |
| VC-LC-0557 | 4.3E+07 | 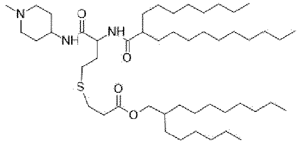 | 77.2 | 142.5 |
| VC-LC-0215 | 4.23E+07 | 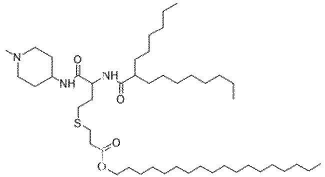 | 58.3 | 143.5 |
| VC-LC-0558 | 7.9E07 | 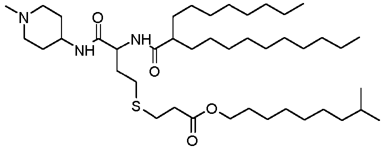 | 50.6 | 155.2 |
| VC-LC-0356 | 3.83E+07 | 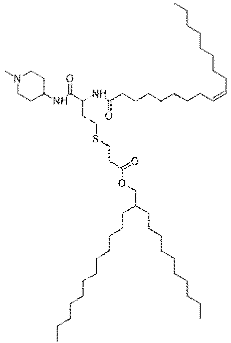 | 68.3 | 218.9 |
| VC-LC-0539 | 3.6E+07 | 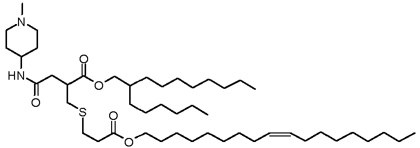 | 85.3 | 176.7 |
| VC-LC-0442 | 2.95E+07 | 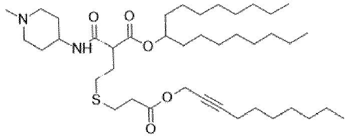 | 87.8 | 171.8 |
| VC-LC-0525 | 2.62E+07 | 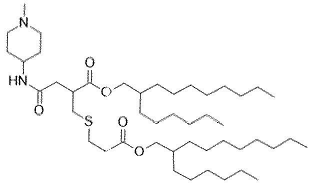 | 59.9 | 151 |
| VC-LC-0431 | 9.92E+07 | 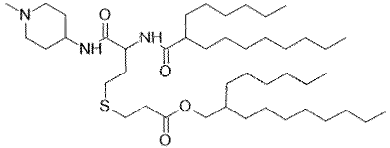 | 60.4 | 196.6 |
| VC-LC-0440 | 2.42E+07 | 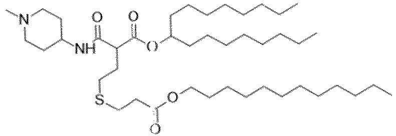 | 76.5 | 167.4 |
| VC-LC-0439 | 2.39E+07 | 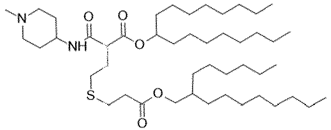 | 91.1 | 144.8 |
| VC-LC-0491 | 2.37E+07 | 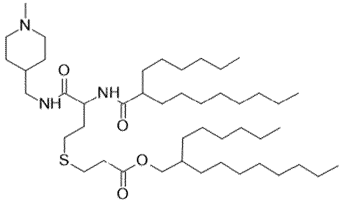 | 72.1 | 160.8 |
| VC-LC-0507 | 2.29E+07 | 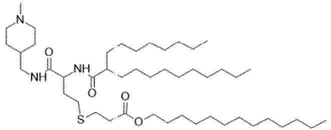 | 95.2 | 151.1 |
| VC-LC-0490 | 1.7E+07 | 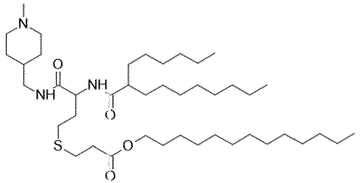 | 97.2 | 136.1 |
| VC-LC-0441 | 2.2E+07 | 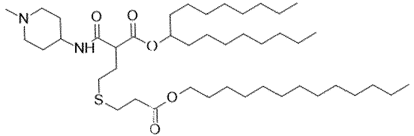 | 78.3 | 186.5 |
| VC-LC-0178 | 8.1E+07 | 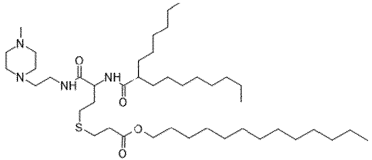 | 65,4 | 196.3 |
| VC-LC-0515 | 3.0E+07 | 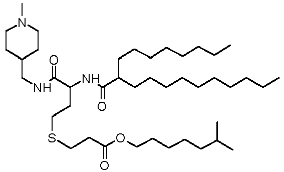 | 98.61 | 141.7 |
| VC-LC-0525 | 2.69E+07 | 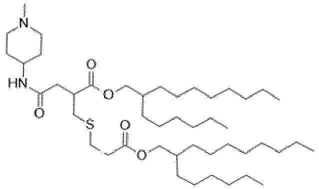 | 95.6 | 151 |
| VC-LC-0546 | 2.54E+07 | 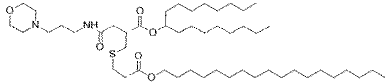 | 39.6 | 164.2 |
| VC-LC-0549 | 2.5E+07 | 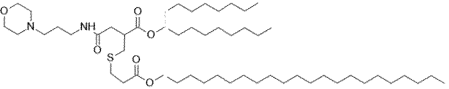 | 36.38 | 194.9 |
| VC-LC-0547 | 1.1E+08 | 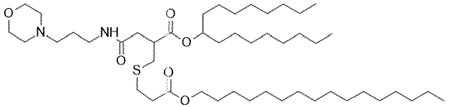 | 31.52 | 168.0 |
| VC-LC-0548 | 1.2E+08 | 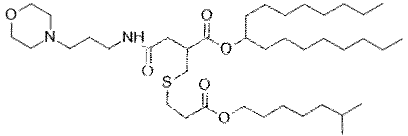 | 30.22 | 145.5 |
| VC-LC-0551 | 1.1E+08 | 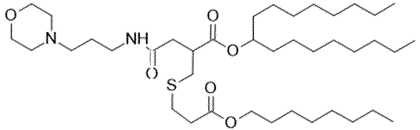 | 29.06 | 155.3 |
| VC-LC-0554 | 1.4E+08 | 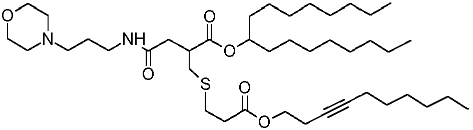 | 29.19 | 158.4 |
| VC-LC-0355 | 2.39E+07 | 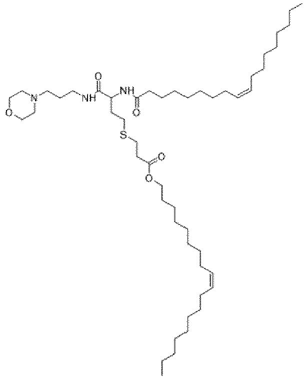 | 81.6 | 242.2 |
| VC-LC-0169 | 7.6E+07 | 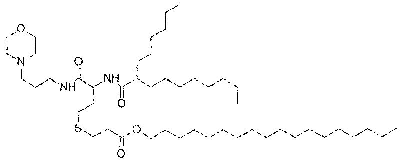 | 33.8 | 158.2 |
| VC-LC-0289 | 2.329E+07 | 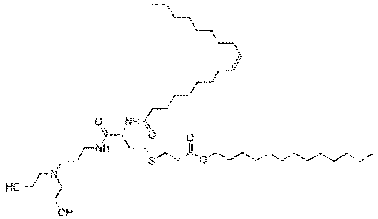 | 89.3 | 207.1 |
| VC-LC-0199 | 1.7E+07 | 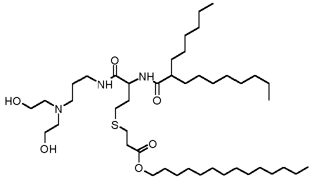 | 96.0 | 194.1 |
| VC-LC-0198 | 1.46E+07 | 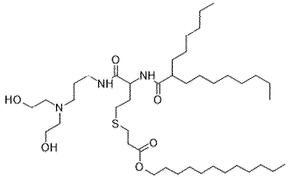 | 90.2 | 227.4 |
| VC-LC-0101 | 1.05E+07 | 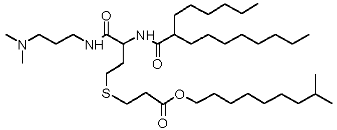 | 96.0 | - |
| VC-LC-0540 | 1.3E+08 | 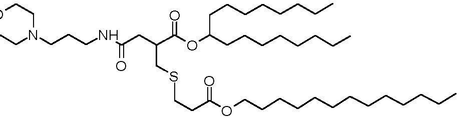 | 43.65 | 140.3 |
| VC-LC-0521 | 1.1E+08 | 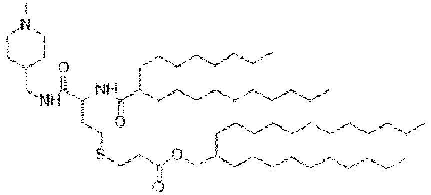 | 52.01 | 153.1 |
| VC-LC-0508 | 1.7E+08 | 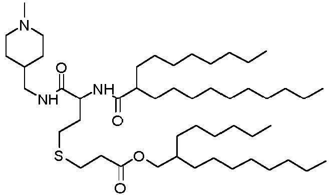 | 91.49 | 152.6 |

Table 7A-C and Table 8 demonstrate that LNPs comprising ionizable lipids of Formula I effectively encapsulate polynucleotides and produce high levels of cellular transfection *in vivo* after administration.

### Example 6: Comparative example.

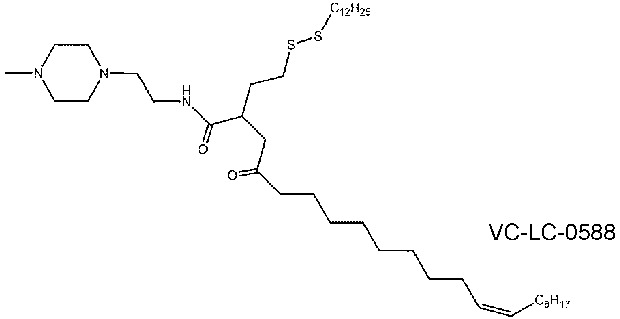

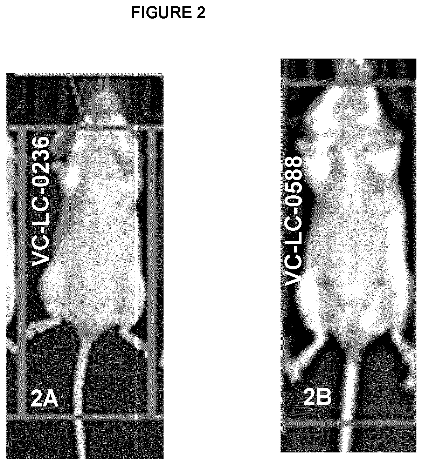

Compound VC-LC-0588 as depicted herein was synthesized following the same synthesis previously reported by Molla et al. (Bioconjugate Chem. 2018, 29, 4, 992-999, referenced as T18U-PY12-A) in a combinatorial library of 288 lipidoids for gene delivery applications via a thiolactone ring opening reaction, obtaining compounds containing unsaturated bonds and reducible disulfide bonds. The study shows the results of the compound tested in vitro using HEK293T cells without addition of helper lipids. The authors reported highly stable liposomes with low toxicity and about 95% transfection efficiency. The study, however, did not report *in vivo* experiments in mammals. Hence, VC-LC-0588 was also studied *in vivo* herein in order to compare the differences between the compounds of general Formula (I) comprising a thioether instead of a disulfide group and the impact of at least one ester group nearby the S atom in the thioether. As depicted in Figure 2B, no transfection (luminescence) was observed in vivo after intra muscular injection of LNPs comprising VC-LC-0588 as ionizable lipid in the following formulation: ionizable lipid: helper lipid: sterol: pegylated lipid → 50:10:38.5:1.5, and encapsulating mRNA coding for for luciferase.

Compounds depicted in Table 9 were also synthesized, characterized and tested *in vivo.* These compounds are partially similar to a dimer of Formula (I), but introduce a disulfide moiety, hence showing a general formula as the one depicted here: 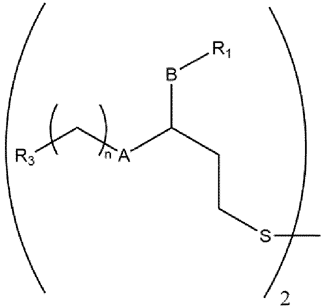 wherein A, B, n, R1 and R3 are the same as defined in general Formula (I).

**Table 9**

| **Ref. No.** | **Structure** | **Total Flux** | **Retention time** | **Theoretical/ experimental MS** |
|---|---|---|---|---|
| VC-LC-0230 | 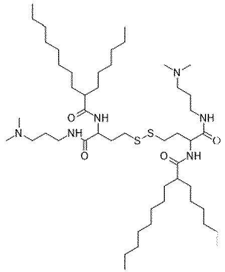 | 4.47E+05 | 13.65 | 913.73/ 913.73 |
| VC-LC-0236 | 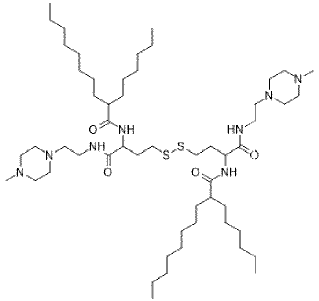 | 5.20E+05 | 13.27 | 995.78/ 996.05 |
| VC-LC-0237 | 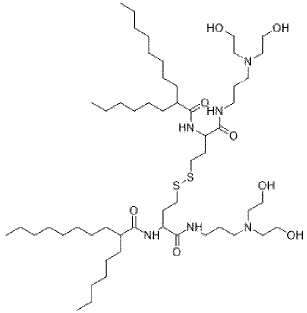 | 3.16E+05 | 13.29 | 1033.7/ 1034.01 |

As depicted in Figure 2A, no transfection (luminescence) was observable *in vivo* after intramuscular injection of LNPs comprising VC-LC-0236 as ionizable lipid in the following formulation: ionizable lipid: helper lipid: sterol: pegylated lipid → 50:10:38.5:1.5, and encapsulating mRNA encoding for luciferase.

### Example 7

Compound VC-LC-0431 was selected to test different compositions ratios.

As previously showed in Tables 7 and 8 (Example 5), a standard formulation having the following composition ratios was tested *in vivo* for the ionizable lipids of Formula (I) of the present invention: ionizable lipid: helper lipid: sterol: pegylated lipid → 50:10:38.5:1.5.

Wherein, as previously described, the ionizable lipid is VC-LC-0431, the helper lipid is DOPE, the sterol is cholesterol and the pegylated lipid is DMG-PEG2000.

Table 10 below shows the results obtained for said standard composition:

**Table 10**

| Particle size/ Ø (nm) | P.D.I. | Z Potential (± D.E.) [mV] | Encapsulation efficiency (%) | Total flux (p·s⁻¹) |
|---|---|---|---|---|
| 196,6 | 0.114 | -0.281 | 60.4 | 9.92E+07 |
| VC-LC-0431 : DOPE: Cholesterol : DMG-PEG2000 | 50:10:38.5:1.5 | | | |

### Optimization of the LNP formulation.

Different ratios of the components comprised in the mixture conforming the LNPs were tested to obtain an optimized formulation, in order to improve the transfection rate *in vivo.* The tested compositions, depicted in Table 11, were injected in mice (intraperitoneal injection) observing a significant improvement in some of them.

**Table 11**

| **Composition** | **Particle size / Ø (nm)** | **PDI** | **Z potential (± D.E.) [mV]** | **Encapsulation efficiency (%)** | **Total flux (p·s⁻¹)** | **Ionizable lipid: DOPE: Cholesterol: DMG-PEG2000** |
|---|---|---|---|---|---|---|
| 1 | 157 | 0.142 | -1.266 | 93.9 | 5.09E+07 | 52.1:19:28.4:0.5 |
| 2 | 203 | 0.085 | -5.572 | 59.2 | 5.68E+07 | 54.5:9.9:34.7:1 |
| 3 | 206.1 | 0.168 | -1.567 | 98.5 | 1.19E+08 | 26:41.7:31.3:1 |
| 4 | 167.1 | 0.101 | -9.236 | 98.7 | 1.25E+08 | 34.8:39.8:24.9:0.5 |
| 5 | 145.6 | 0.112 | -5.879 | 60.5 | 1.33E+08 | 43.8:12.5:37.5:6.3 |
| 6 | 176.8 | 0.118 | -6.896 | 69.5 | 1.53E+08 | 49.5:11:38.5:1.1 |
| 7 | 171.2 | 0.120 | -7.327 | 91.2 | 1.63E+08 | 41.9:37.2:18.6:2.3 |
| 8 | 161.8 | 0.087 | -7.371 | 90.0 | 1.88E+08 | 46.8:34:17:2.1 |

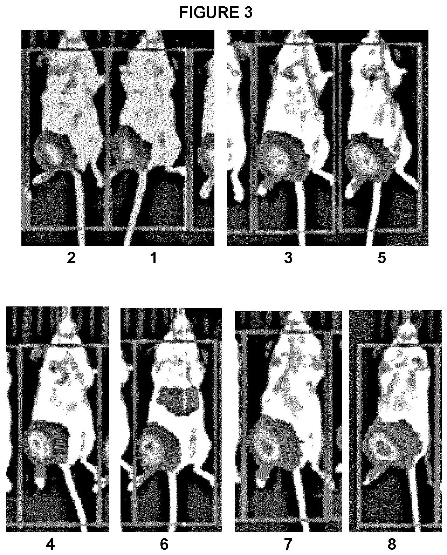

The results depicted in Table 11 demonstrate that it is possible to improve the transfection rate *in vivo* by modifying the composition rates of the components in the formulation of the LNPs comprising the ionizable lipids of Formula (I) of the present invention. The LNP of compositions 3-8 achieved higher transfection rates in when compared to the previous LNP comprising a standard composition of 50:10:38,5:1,5 for ionizable lipid: DOPE: cholesterol: DMG-PEG2000. Figure 3 depicts in vivo transfection in mice after administration of the 8 compositions defined in Table 11.

### Example 8 - LNPs stability overtime

Two LNPs comprising:
ionizable lipid (Formula I):non-cationic lipid:sterol: PEG-modified lipid

in the ratios 50:10:38.5:1.5 were tested in order to determine their stability. Formulation A comprising VC-LC-0163 as the ionizable lipid was administered to mice and transfection efficiency measured with a fresh preparation in a first experiment, and with a preparation that had been stored for 83 days at T = 4 °C.

Formulation B comprising VC-LC-0263 as the ionizable lipid was administered to mice and transfection efficiency measured with a fresh preparation in a first experiment, and with a preparation that had been stored for 61 days at T = 4 °C.

The results are summarized in Table 12 below:

**Table 12**

| **Ionizable lipid: DOPE: Cholesterol: DMG-PEG2000** | **Total flux (p·s⁻¹)** | **Total flux (p·s⁻¹)** | **Total flux (p·s⁻¹)** |
|---|---|---|---|
| | **t = 0** | **t = 83 days** | **t = 61 days** |
| VC-LC-0163 | 5.22E+07 | 1.04E+07 | |
| VC-LC-0263 | 1.19E+08 | | 5.07E+07 |

The results obtained demonstrate that LNPs comprising the ionizable lipids of Formula (I) of the present disclosure are still able to produce *in vivo* transfection after long periods of storage in normal refrigeration conditions of 4 °C.

### Citation List

1. Molla MR, et al. (cf. Molla MR, et al. "One-Pot Parallel Synthesis of Lipid Library via Thiolactone Ring Opening and Screening for Gene Delivery". Bioconjug Chem. 2018, vol. 29(4), pp. 992-999. doi: 10.1021/acs.bioconjchem.8b00007
2. Garbiras, B.J.; Marburg, S. "Preparation of Carboxythiolactones and Their Active Derivatives". Synthesis, 1999, vol. 2, pp. 270-274; doi:10.1055/s-1999-3377;
3. Hassett, K. J. et al., "Optimization of Lipid Nanoparticles for Intramuscular Administration of mRNA Vaccines", 2019, Mol. Ther. Nucleic Acid, vol. 15, pp. 1-11, DOI:10.1016/j.omtn.2019.01.013.
4. Wang X., Liu S., Sun Y., et al. "Preparation of selective organ-targeting (SORT) lipid nanaoparticles (LNPs) using multiple technical methods for tissue-specific mRNA delivery", Nat. Protoc., 2022, doi:10.1038/s41596-022-00755-x

## Claims

1. An ionizable lipid of formula (I): 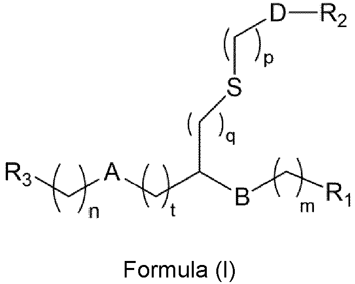
or a pharmaceutically acceptable salt thereof, or a stereoisomer of any one of them,
wherein
A is 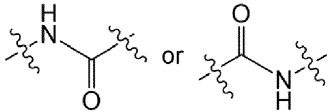
B is 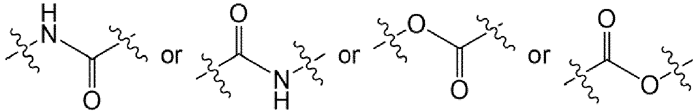
D is 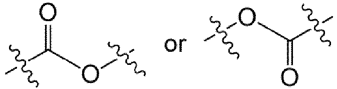
n, m and p are independently 0, 1, 2, 3, 4, 5, or 6;
q is selected from 1 and 2,
t is selected from 0 and 1,
and wherein q + t = 2;
R₁ and R₂ are independently a linear or branched C1-C30 alkyl, C2-C30 alkenyl, C2-C30 alkynyl, wherein R1 and R2 are optionally substituted with one or more substituents selected from the group consisting of -OH, -COOR₄, and (-C(=O)SR₄), wherein R₄ is C1-C6 alkyl, C2-C6 alkenyl or C2-C6 alkynyl,
and wherein
R₃ is a heterocycle comprising at least one N atom, or alternatively R₃ is: 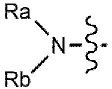
wherein Ra and Rb are independently linear or branched C1-C6 alkyl, optionally substituted with a hydroxyl group;
provided that the compounds methyl-S-(2-methoxy-2-oxoethyl)-N-(pyridin-2-ylcarbonyl)-homocysteinate 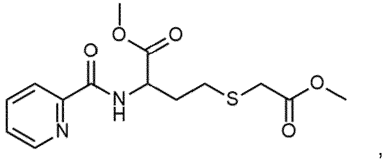 and
methyl N-[(4-chloropyridin-2-yl)carbonyl]-S-(2-methoxy-2-oxoethyl)homocysteinate 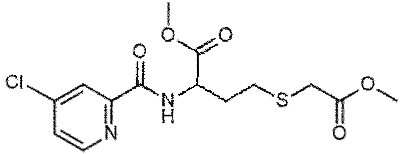 are excluded.

2. The ionizable lipid of claim 1, wherein R₃ is a 5-membered ring or a 6 membered ring comprising one N atom and, optionally, a second heteroatom selected preferably from N and O.

3. The ionizable lipid of any one of the claims 1 to 2, wherein R3 is selected from the following structures:

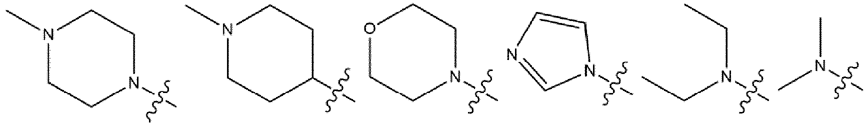

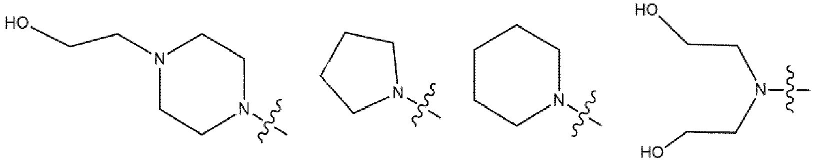

4. The ionizable lipid according to any of the preceding claims, wherein n is selected from 0, 1, 2 or 3; p is selected from 0, 1, 2, or 3; m is selected from 0, 1, 2 or 3; t is 0 or 1; q is 1 or 2, and t + q = 2.

5. The compound according to claim 4, wherein n is 2 or 3, m = t = 0, p = q = 2,
A=B= 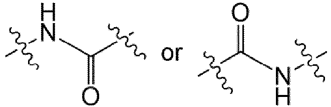
wherein
R₁ is a C10-C20 branched alkyl;
R₂ is a C2-C30 linear or branched alkyl or a C6-C24 linear alkenyl or alkynyl; and
R₃ is: 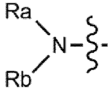
wherein Ra = Rb = C1-C4 alkyl;
or, alternatively wherein n = 2, and R₂ is C2-C30 linear or branched alkyl, and wherein R3 is: 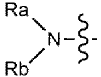 Wherein Ra = Rb = C1-C4 alcohol terminated alkyl;
or, alternatively
wherein n is 2 or 3, m = t = 0, p = q = 2,
A is 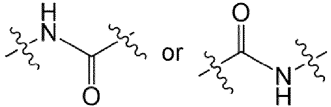
wherein R₁ is C10-C20 branched alkyl, and R₂ is C3-C30 linear or branched alkyl, and wherein R₃ is an imidazole ring;
or, alternatively
wherein n = 3, m = t = 0, p = q = 2,
A is 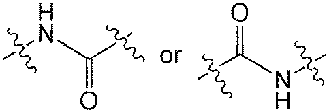
wherein R₁ is C10-C20 branched alkyl, and R₂ is C4-C20 linear or branched alkyl, and wherein R₃ is a pyrrolidine ring;
or, alternatively
wherein n = 2, m = t = 0, p = q = 2,
A is 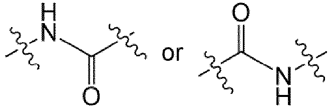 wherein R1 is C10-C20 linear or branched alkyl, and R₂ is C4-C20 linear or branched alkyl, and wherein R₃ is N-4-methylpiperazine;
or, alternatively
wherein n is selected from 2 or 3,
m = t = 0, and p = q = 2,
A=B= 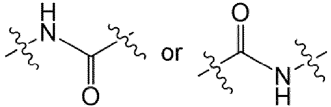
R₁ is C10-C22 linear or branched alkyl, alkenyl or alkynyl; R₂ is C3-C30 linear or branched alkyl or C3-C30 linear alkenyl or alkynyl; and wherein R₃ is a morpholino ring;
or, alternatively
wherein n is selected from 2 or 3, m = 0, p = 2,
q = t = 1,
A is 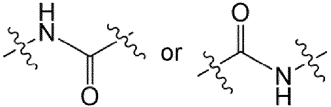
B = D, R₁ is C10-C22 linear branched alkyl; R₂ is C3-C30 linear or branched alkyl or C3-C30 linear alkenyl or alkynyl, and R₃ is a morpholino ring.

6. The ionizable lipid of claim 1, which is a compound selected from the group consisting of:
| | |
|---|---|
| VC-LC-0099 | 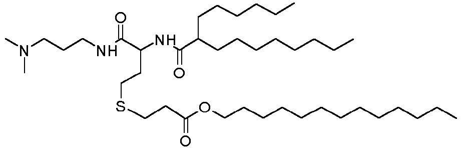 |
| VC-LC-0101 | 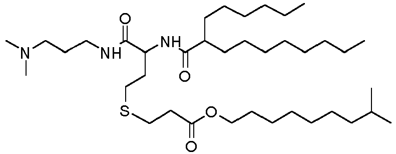 |
| VC-LC-0163 | 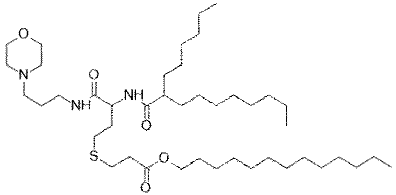 |
| VC-LC-0168 | 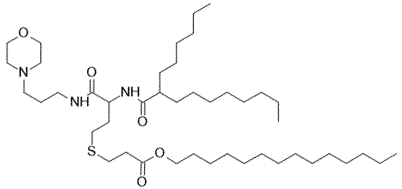 |
| VC-LC-0169 | 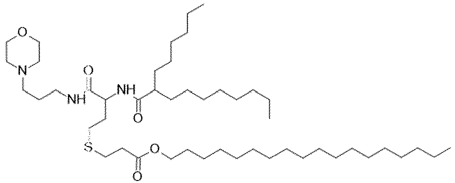 |
| VC-LC-0170 | 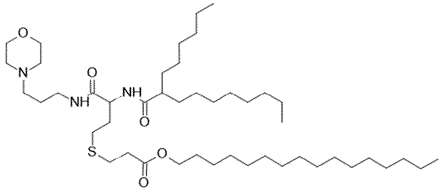 |
| VC-LC-0178 | 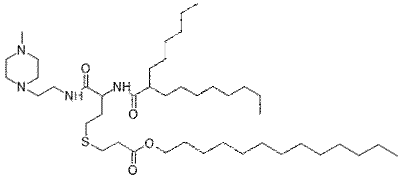 |
| VC-LC-0183 | 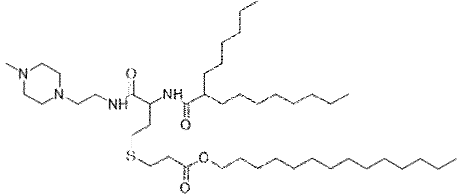 |
| VC-LC-0194 | 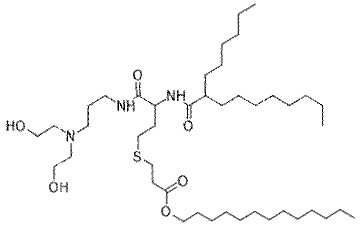 |
| VC-LC-0196 | 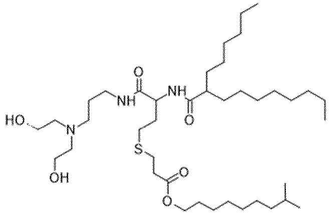 |
| VC-LC-0198 | 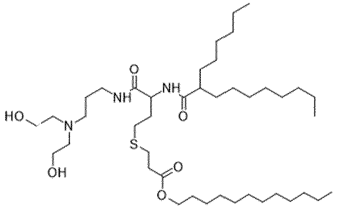 |
| VC-LC-0199 | 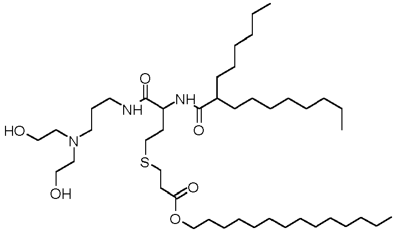 |
| VC-LC-0200 | 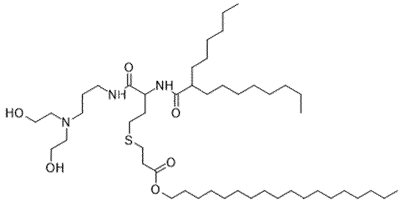 |
| VC-LC-0201 | 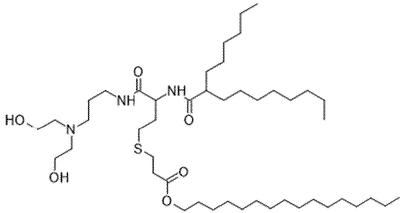 |
| VC-LC-0202 | 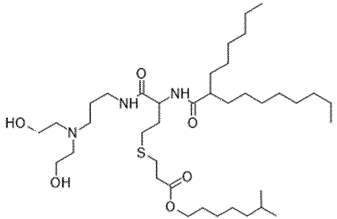 |
| VC-LC-0207 | 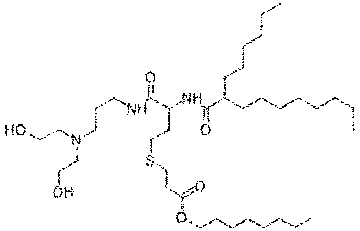 |
| VC-LC-0209 | 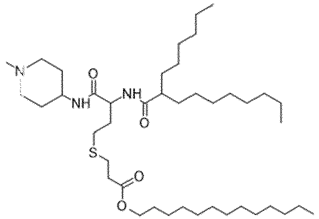 |
| VC-LC-0211 | 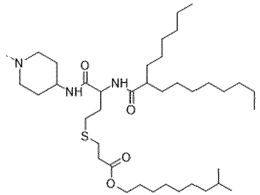 |
| VC-LC-0213 | 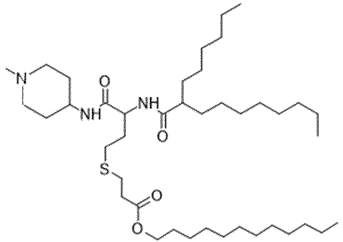 |
| VC-LC-0214 | 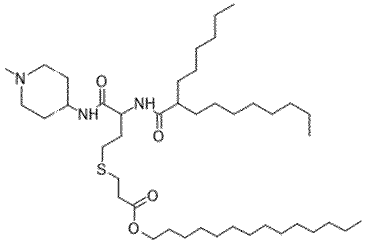 |
| VC-LC-0215 | 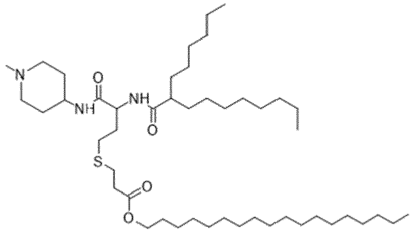 |
| VC-LC-0216 | 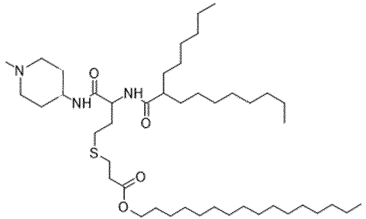 |
| VC-LC-0241 | 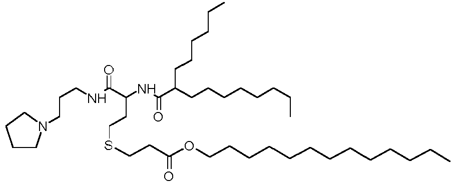 |
| VC-LC-0256 | 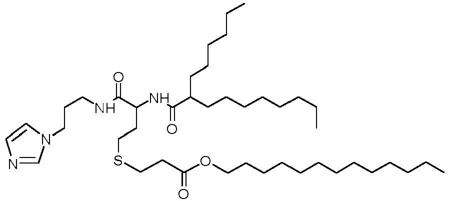 |
| VC-LC-0258 | 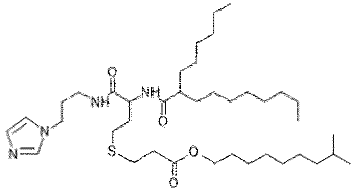 |
| VC-LC-0261 | 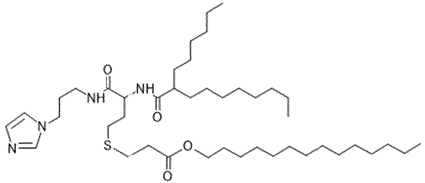 |
| VC-LC-0262 | 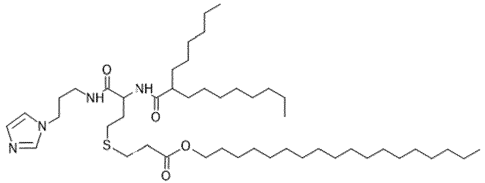 |
| VC-LC-0263 | 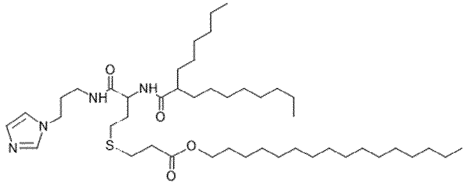 |
| VC-LC-0268 | 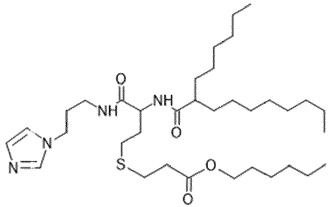 |
| VC-LC-0269 | 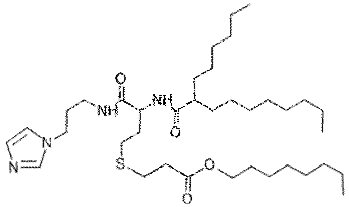 |
| VC-LC-0289 | 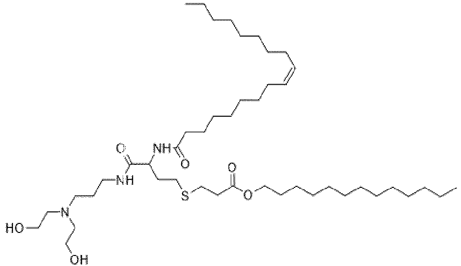 |
| VC-LC-0294 | 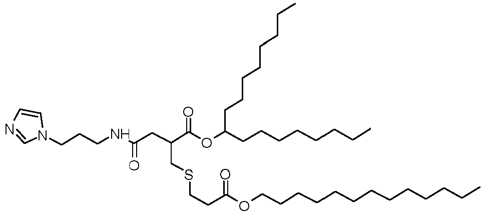 |
| VC-LC-0296 | 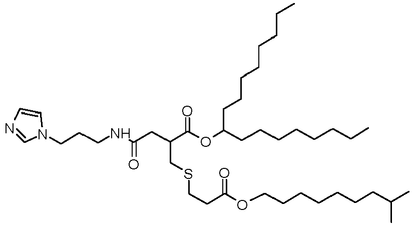 |
| VC-LC-0297 | 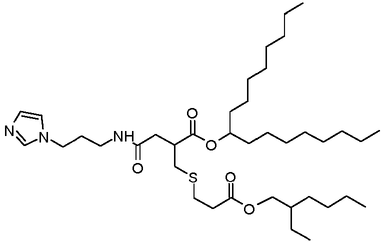 |
| VC-LC-0298 | 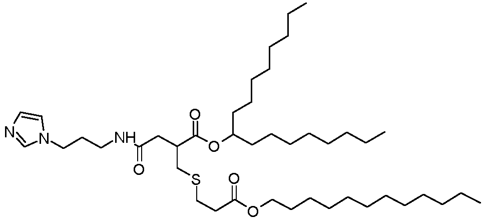 |
| VC-LC-0299 | 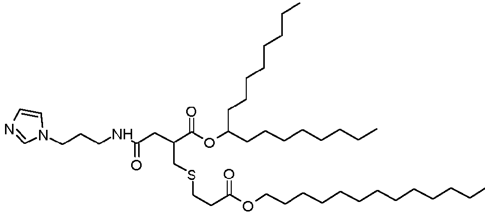 |
| VC-LC-0300 | 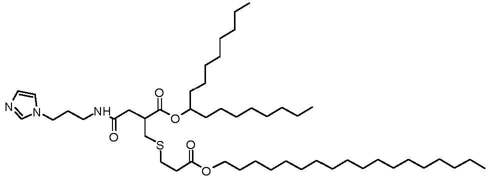 |
| VC-LC-0301 | 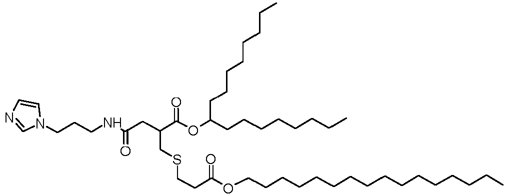 |
| VC-LC-0302 | 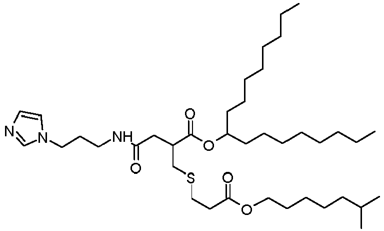 |
| VC-LC-0304 | 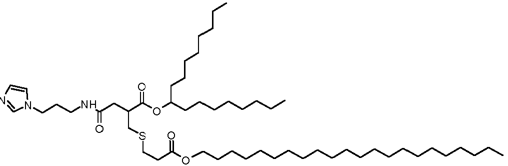 |
| VC-LC-0306 | 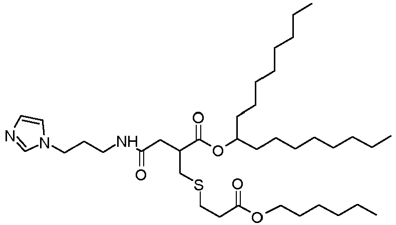 |
| VC-LC-0307 | 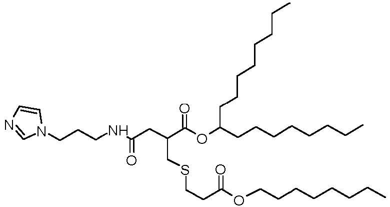 |
| VC-LC-0353 | 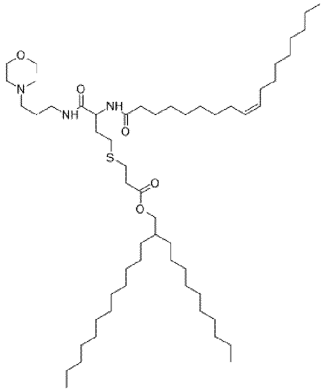 |
| VC-LC-0355 | 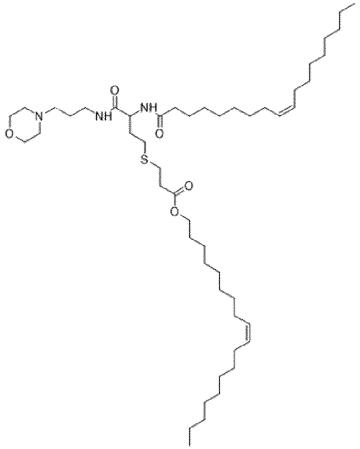 |
| VC-LC-0356 | 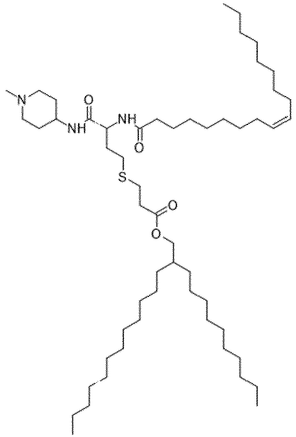 |
| VC-LC-0362 | 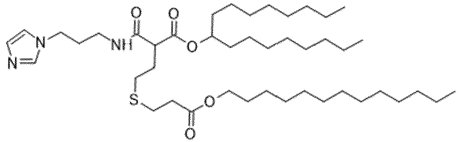 |
| VC-LC-0366 | 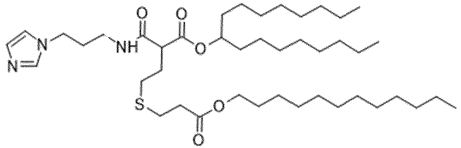 |
| VC-LC-0367 | 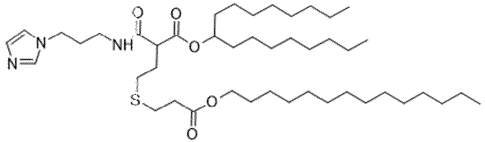 |
| VC-LC-0369 | 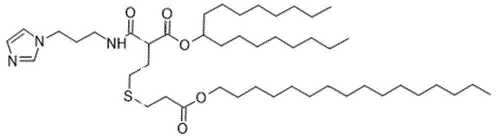 |
| VC-LC-0370 | 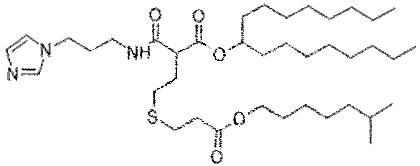 |
| VC-LC-0389 | 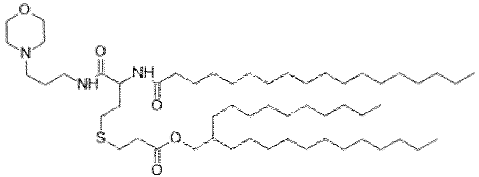 |
| VC-LC-0428 | 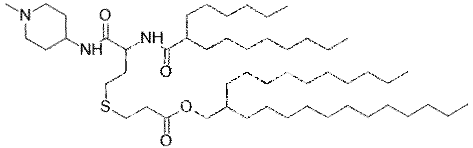 |
| VC-LC-0429 | 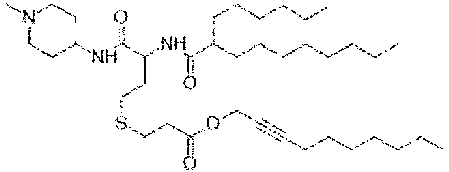 |
| VC-LC-0430 | 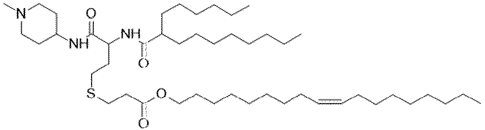 |
| VC-LC-0431 | 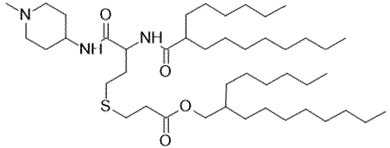 |
| VC-LC-0439 | 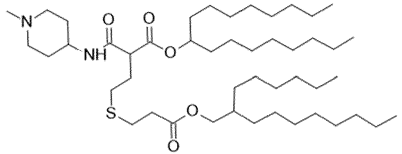 |
| VC-LC-0440 | 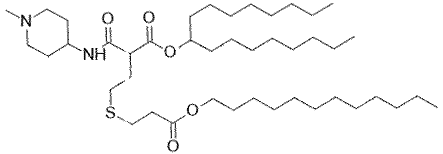 |
| VC-LC-0441 | 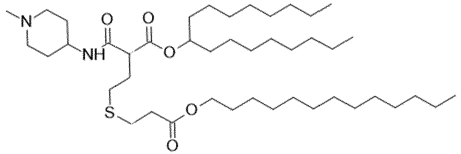 |
| VC-LC-0442 | 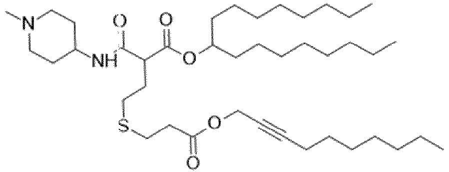 |
| VC-LC-0443 | 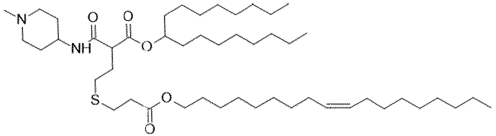 |
| VC-LC-0444 | 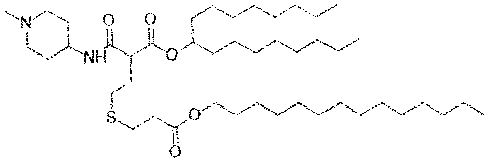 |
| VC-LC-0473 | 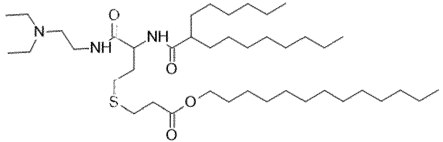 |
| VC-LC-0474 | 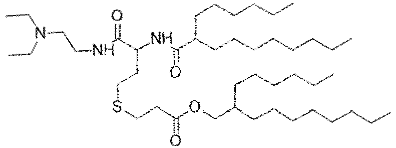 |
| VC-LC-0475 | 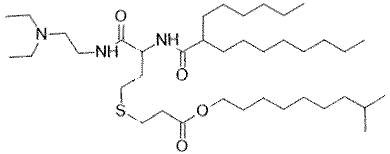 |
| VC-LC-0477 | 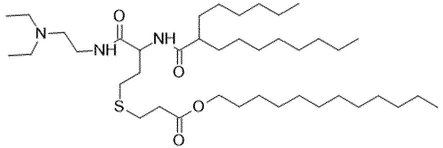 |
| VC-LC-0478 | 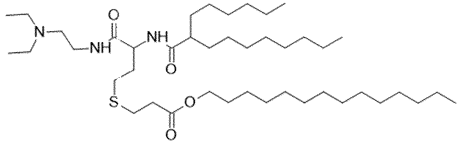 |
| VC-LC-0480 | 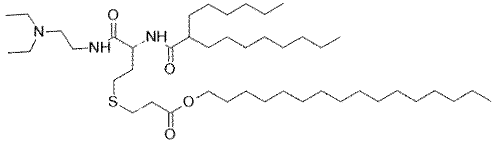 |
| VC-LC-0487 | 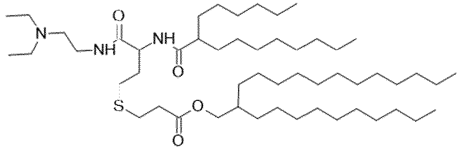 |
| VC-LC-0488 | 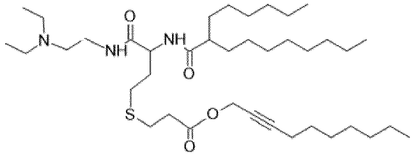 |
| VC-LC-0489 | 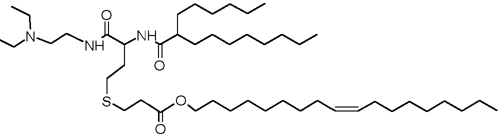 |
| VC-LC-0490 | 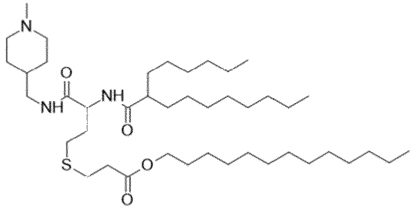 |
| VC-LC-0491 | 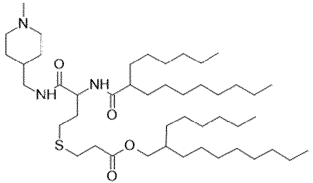 |
| VC-LC-0492 | 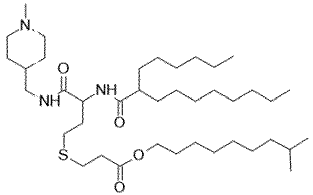 |
| VC-LC-0504 | 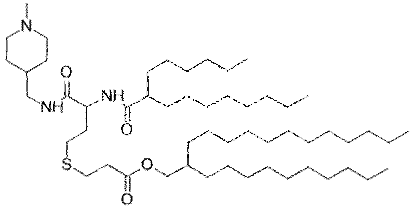 |
| VC-LC-0505 | 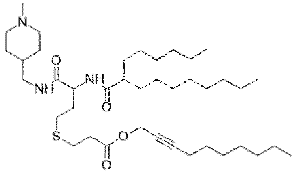 |
| VC-LC-0507 | 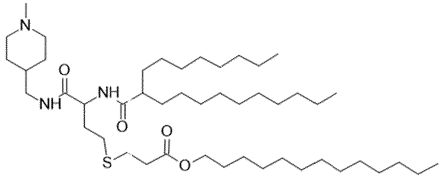 |
| VC-LC-0508 | 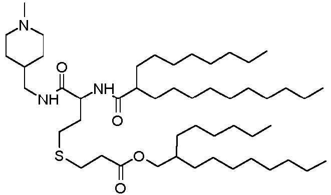 |
| VC-LC-0509 | 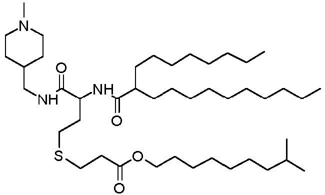 |
| VC-LC-0510 | 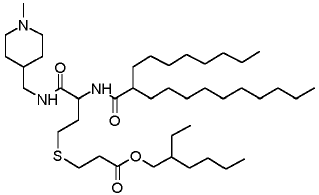 |
| VC-LC-0515 | 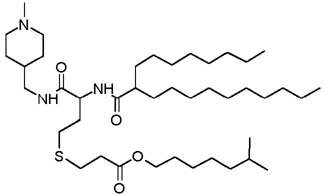 |
| VC-LC-0521 | 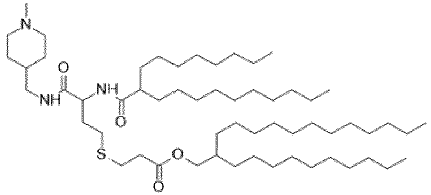 |
| VC-LC-0524 | 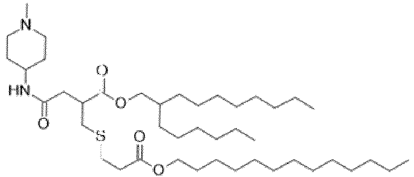 |
| VC-LC-0525 | 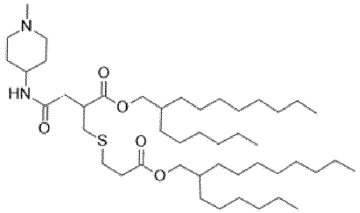 |
| VC-LC-0531 | 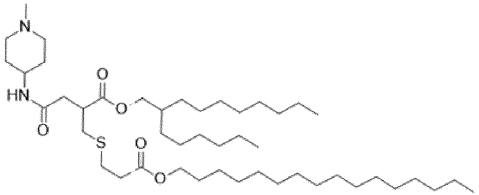 |
| VC-LC-0539 | 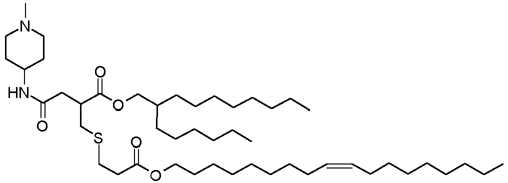 |
| VC-LC-0540 | 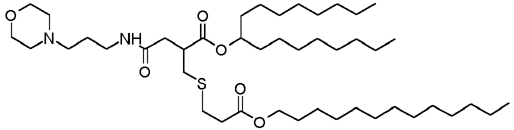 |
| VC-LC-0541 | 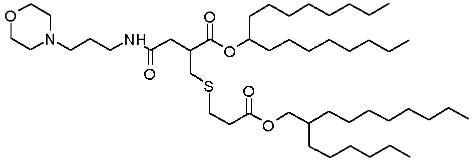 |
| VC-LC-0542 | 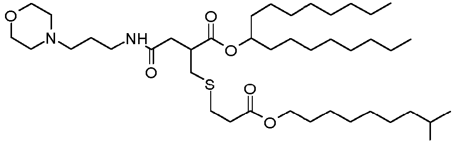 |
| VC-LC-0543 | 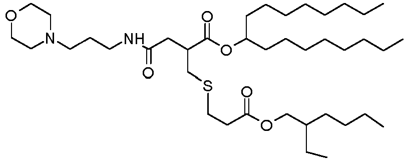 |
| VC-LC-0544 | 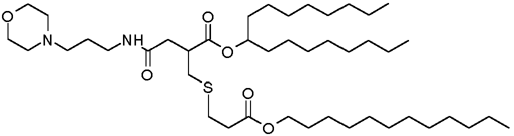 |
| VC-LC-0546 | 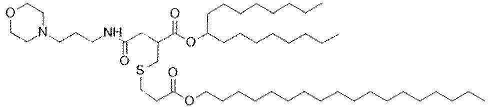 |
| VC-LC-0547 | 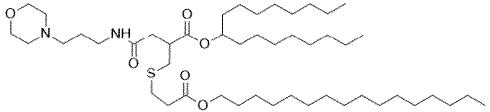 |
| VC-LC-0548 | 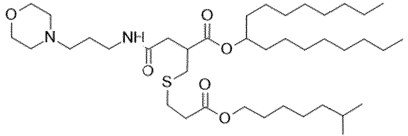 |
| VC-LC-0549 | 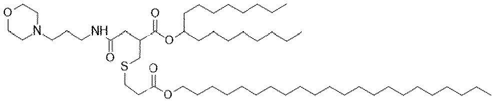 |
| VC-LC-0550 | 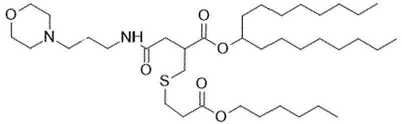 |
| VC-LC-0551 | 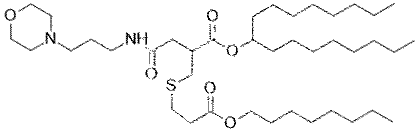 |
| VC-LC-0553 | 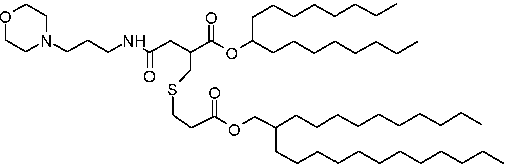 |
| VC-LC-0554 | 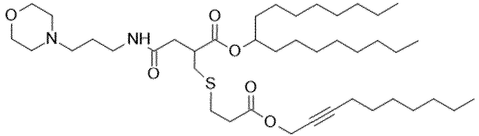 |
| VC-LC-0556 | 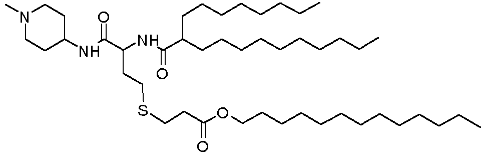 |
| VC-LC-0557 | 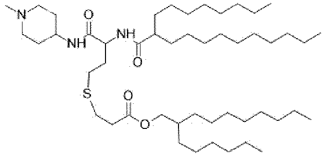 |
| VC-LC-0558 | 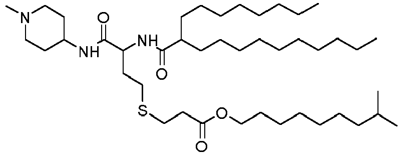 |
| VC-LC-0559 | 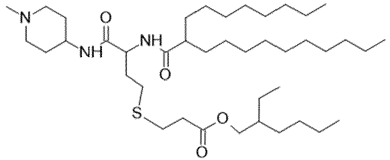 |
| VC-LC-0606 | 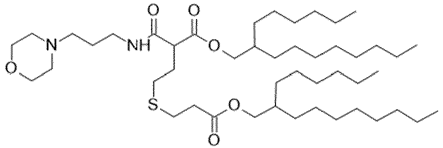 |
| VC-LC-0607 | 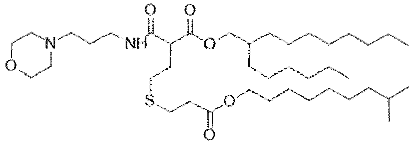 |
| VC-LC-0608 | 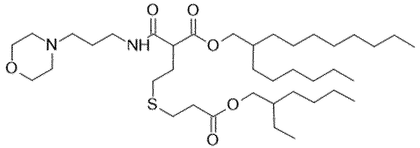 |
| VC-LC-0609 | 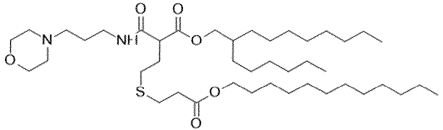 |
| VC-LC-0610 | 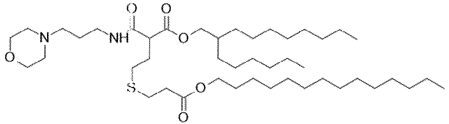 |
or a pharmaceutically acceptable salt thereof, or a stereoisomer of any one of them.

7. A lipid nanoparticle comprising an ionizable lipid of formula (I): 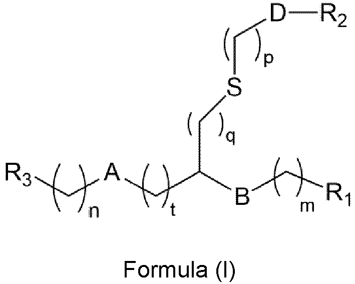
or a pharmaceutically acceptable salt thereof, or a stereoisomer of any one of them,
wherein
A is 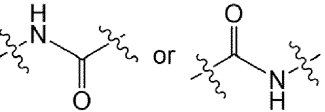
B is 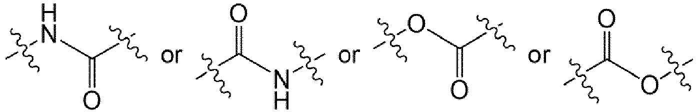
D is 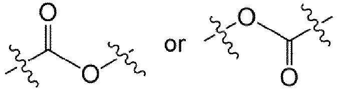
n, m and p are independently 0, 1, 2, 3, 4, 5, or 6;
q is selected from 1 and 2,
t is selected from 0 and 1,
and wherein q + t = 2;
R₁ and R₂ are independently a linear or branched C1-C30 alkyl, C2-C30 alkenyl, C2-C30 alkynyl, wherein R1 and R2 are optionally substituted with one or more substituents selected from the group consisting of -OH, -COOR₄, and (-C(=O)SR₄), wherein R₄ is C1-C6 alkyl, C2-C6 alkenyl or C2-C6 alkynyl,
and wherein
R₃ is a heterocycle comprising at least one N atom, or alternatively R₃ is: 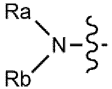
wherein Ra and Rb are independently linear or branched C1-C6 alkyl, optionally substituted with a hydroxyl group.

8. The lipid nanoparticle according to claim 7, further comprising:
i) a non-cationic lipid;
optionally, wherein the non-cationic lipid is selected from the group consisting of: 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1-stearoyl-2-oleoyl-*sn*-glycero-3-phosphocoline (SOPC), 1,2-dipalmitoyl-sn-glycero-3-phosphocholine (DPPC), 1,2-distearoyl-sn-glycero-3 -phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl- sn -glycero-3-phosphocholine (18:0 Diether PC), 1-oleoyl-2-cholesterylhemisuccinoyl- sn -glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine,1,2-dioleoyl-sn-glycero-3-phosphoetha nolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), sphingomyelin, and mixtures thereof;
optionally, wherein the non-cationic lipid is DOPE; and/or wherein the non-cationic lipid is DSPC;
ii) a sterol;
optionally, wherein the sterol is selected from the group consisting of cholesterol, fecosterol, sitosterol, ergosterol, campesterol, stigmasterol, brassicasterol, tomatidine, ursolic acid, alpha-tocopherol, and mixtures thereof;
further optionally, wherein the sterol is cholesterol; and
iii) a PEG-modified lipid;
optionally, wherein the PEG-modified lipid is selected from the group consisting of a PEG-modified phosphatidylethanolamine, a PEG-modified phosphatidic acid, a PEG-modified phosphatidylcholine, a PEG-modified ceramide, a PEG-modified dialkylamine, a PEG-modified diacylglycerol, a PEG-modified dialkylglycerol, and mixtures thereof.

9. The lipid nanoparticle according to any one of claims 7 to 8, further comprising a non-cationic lipid, a sterol, and a PEG-modified lipid, wherein the ionizable lipid is in an amount from 25 to 60 mol%, the PEG-modified lipid is in an amount from 0.1 to 10 mol%; the non-cationic lipid is in an amount from 10 to 45 mol%; and the sterol is in an amount from 10 to 40 mol%.

10. A lipid nanoparticle according to any one of claims 7 to 9, further comprising a pharmaceutically active agent.

11. The lipid nanoparticle according to claim 10, wherein the pharmaceutically active agent is selected from the group consisting of a polynucleotide, a DNA construct comprising a promoter operatively linked to a sequence encoding a polynucleotide, and an expression vector comprising a DNA construct comprising a promoter operatively linked to a sequence encoding a polynucleotide.

12. The lipid nanoparticle according to claim 11, wherein the polynucleotide is a natural or an artificial deoxyribonucleic acid (DNA) or a natural or artificial ribonucleic acid (RNA).

13. A pharmaceutical composition comprising the lipid nanoparticle as defined in any one of claims 10 to 12 and a pharmaceutically acceptable excipient or carrier.

14. A lipid nanoparticle as defined in any one of claims 10 to 12, or a pharmaceutical composition as defined in claim 13, for use in a method for treating a disease or disorder in a subject in need thereof, the method comprising administering to the subject a therapeutically effective amount of the nanoparticle composition or of the pharmaceutical composition.

15. A lipid nanoparticle of any one of claims 7-9 as defined herein for use as an encapsulation agent.

## Patentansprüche

1. Ein ionisierbares Lipid der Formel (I): 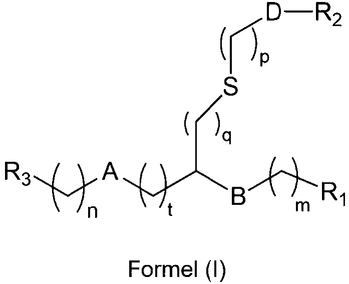
oder ein pharmazeutisch akzeptables Salz davon, oder ein Stereoisomer von einem von ihnen, wobei
A 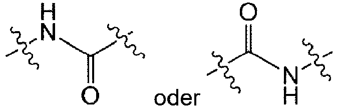 ist
B 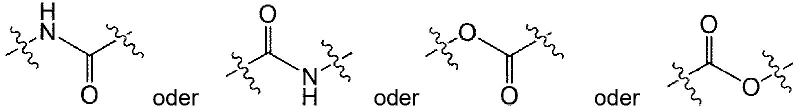 ist
D 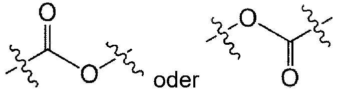 ist
n, m und p unabhängig voneinander 0, 1, 2, 3, 4, 5 oder 6 sind;
q ausgewählt aus 1 und 2 ist,
t ausgewählt aus 0 und 1 ist,
und wobei q + t = 2 ist;
R₁ und R₂ unabhängig voneinander ein lineares oder verzweigtes C1-C30-Alkyl, C2-C30-Alkenyl, C2-C30-Alkinyl sind, wobei R1 und R2 wahlweise mit einem oder mehreren Substituenten substituiert sind, die aus der Gruppe ausgewählt sind, die aus -OH, - COOR₄ und (-C(=O)SR₄) besteht, wobei R₄ = C1-C6-Alkyl, C2-C6-Alkenyl oder C2-C6-Alkinyl ist,
und wobei
R₃ ein Heterocyclus ist, der mindestens ein N-Atom umfasst, oder ersatzweise R₃ ist: 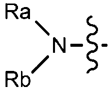
wobei Ra und Rb unabhängig voneinander lineares oder verzweigtes C1-C6-Alkyl sind, wahlweise substituiert durch eine Hydroxylgruppe;
unter der Voraussetzung, dass die Verbindungen Methyl-S-(2-methoxy-2-oxoethyl)-N-(pyridin-2-ylcarbonyl)-homocysteinat 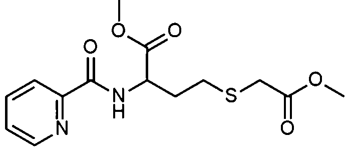 und
Methyl-N-[(4-Chlorpyridin-2-yl)carbonyl]-S-(2-methoxy-2-oxoethyl)homocysteinat 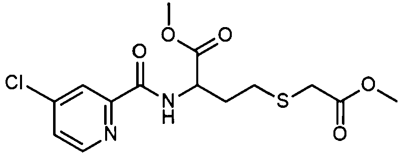 ausgeschlossen sind.

2. Das ionisierbare Lipid von Anspruch 1, wobei R₃ ein 5-gliedriger Ring oder ein 6-gliedriger Ring ist, der ein N-Atom und wahlweise ein zweites Heteroatom umfasst, das vorzugsweise aus N und O ausgewählt ist.

3. Das ionisierbare Lipid von einem der Ansprüche 1 bis 2, wobei R3 ausgewählt ist aus den folgenden Strukturen:

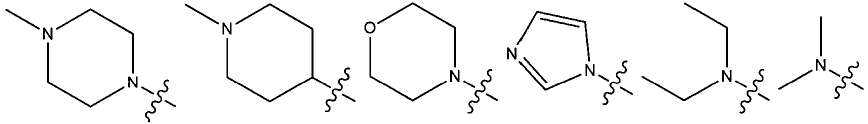

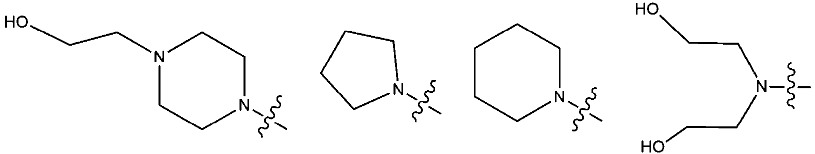

4. Das ionisierbare Lipid nach einem der vorhergehenden Ansprüche, wobei n ausgewählt ist aus 0, 1, 2 oder 3; p ausgewählt ist aus 0, 1, 2 oder 3; m ausgewählt ist aus 0, 1, 2 oder 3; t = 0 oder 1; q = 1 oder 2 und t + q = 2 ist.

5. Die Verbindung nach Anspruch 4, wobei n = 2 oder 3 ist, m = t = 0, p = q = 2,
A=B= 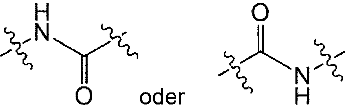 ist
wobei
R₁ ein verzweigtes C10-C20-Alkyl ist;
R₂ ein lineares oder verzweigtes C2-C30-Alkyl oder ein lineares C6-C24-Alkenyl oder - Alkinyl ist; und
R₃ ist: 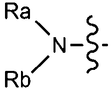
wobei Ra = Rb = C1-C4-Alkyl ist;
oder, ersatzweise, wobei n = 2 ist und R₂ lineares oder verzweigtes C2-C30-Alkyl ist und wobei R3 ist: 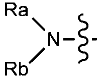
wobei Ra = Rb = C1-C4-Alkohol-terminiertes Alkyl ist;
oder, ersatzweise,
wobei n= 2 oder 3, m = t = 0, p = q = 2 ist,
A 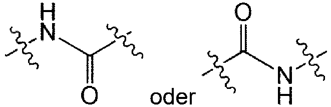 ist
wobei R₁ verzweigtes C10-C20-Alkyl ist und R₂ lineares oder verzweigtes C3-C30-Alkyl ist und wobei R₃ ein Imidazolring ist;
oder, ersatzweise,
wobei n = 3, m = t = 0, p = q = 2 ist,
A 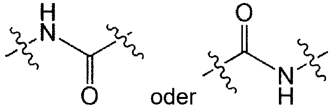 ist
wobei R₁ verzweigtes C10-C20-Alkyl ist und R₂ lineares oder verzweigtes C4-C20-Alkyl ist und wobei R₃ ein Pyrrolidinring ist;
oder, ersatzweise,
wobei n = 2, m = t = 0, p = q = 2 ist,
A 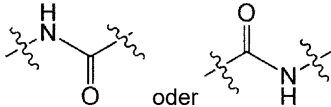 ist
wobei R1 lineares oder verzweigtes C10-C20-Alkyl ist und R₂ lineares oder verzweigtes C4-C20-Alkyl ist und wobei R₃ = N-4-Methylpiperazin ist;
oder, ersatzweise,
wobei n ausgewählt aus 2 oder 3 ist,
m = t = 0 und p = q = 2 ist,
A=B= 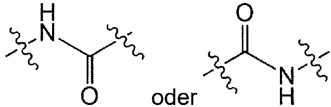 ist
R₁ lineares oder verzweigtes C10-C22-Alkyl, -Alkenyl oder -Alkinyl ist; R₂ lineares oder verzweigtes C3-C30-Alkyl oder lineares C3-C30-Alkenyl oder -Alkinyl ist; und wobei R₃ ein Morpholinring ist;
oder, ersatzweise,
wobei n ausgewählt ist aus 2 oder 3, m = 0, p = 2,
q = t = 1,
A 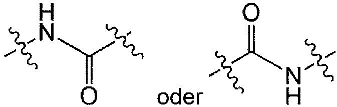 ist
B = D, R₁ lineares verzweigtes C10-C22-Alkyl ist; R₂ lineares oder verzweigtes C3-C30-Alkyl oder lineares C3-C30-Alkenyl oder -Alkinyl ist und R₃ ein Morpholinring ist.

6. Das ionisierbare Lipid von Anspruch 1, das eine Verbindung ist, die ausgewählt ist aus der Gruppe bestehend aus:
| | |
|---|---|
| VC-LC-0099 | 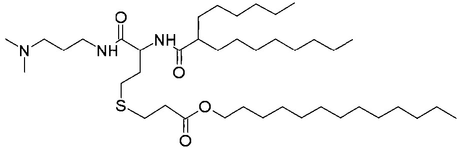 |
| VC-LC-0101 | 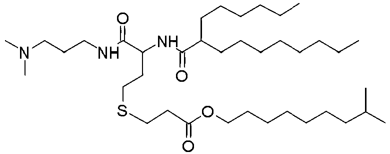 |
| VC-LC-0163 | 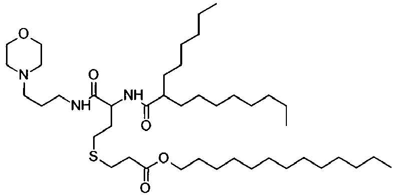 |
| VC-LC-0168 | 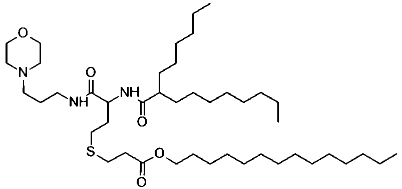 |
| VC-LC-0169 | 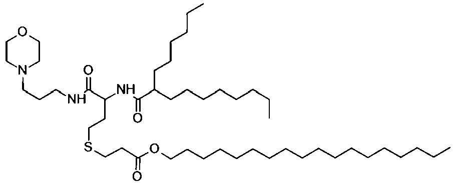 |
| VC-LC-0170 | 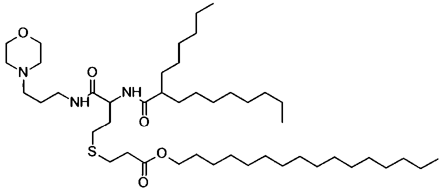 |
| VC-LC-0178 | 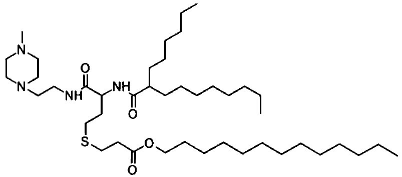 |
| VC-LC-0183 | 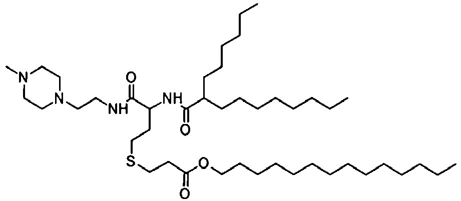 |
| VC-LC-0194 | 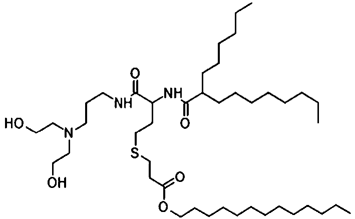 |
| VC-LC-0196 | 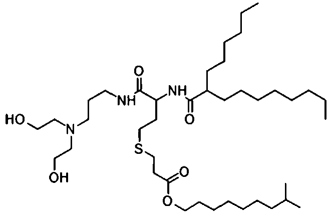 |
| VC-LC-0198 | 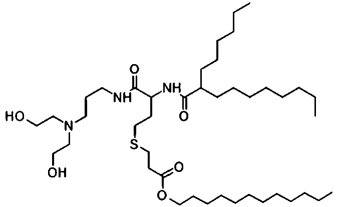 |
| VC-LC-0199 | 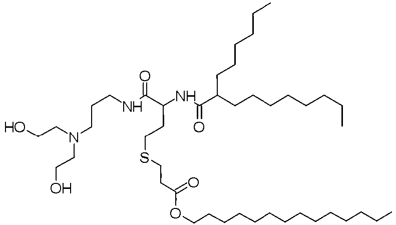 |
| VC-LC-0200 | 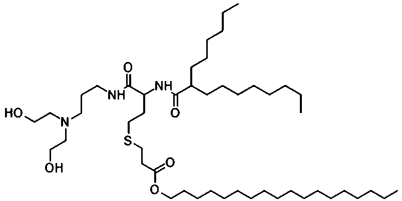 |
| VC-LC-0201 | 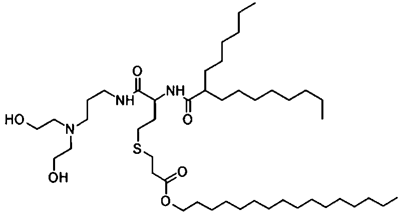 |
| VC-LC-0202 | 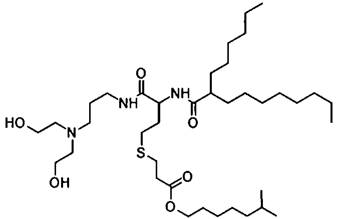 |
| VC-LC-0207 | 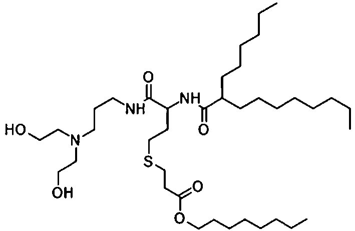 |
| VC-LC-0209 | 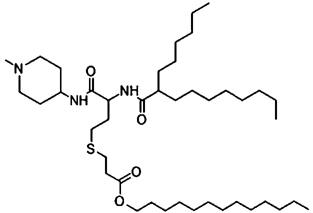 |
| VC-LC-0211 | 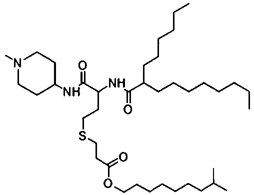 |
| VC-LC-0213 | 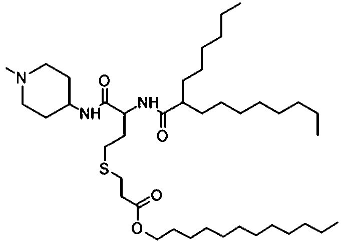 |
| VC-LC-0214 | 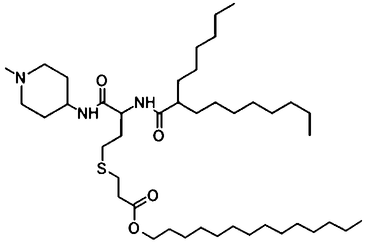 |
| VC-LC-0215 | 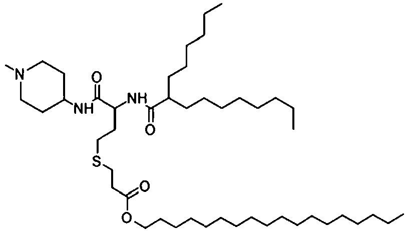 |
| VC-LC-0216 | 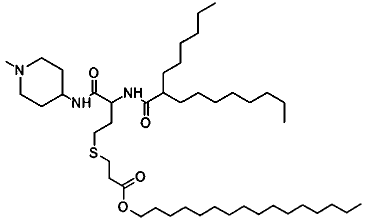 |
| VC-LC-0241 | 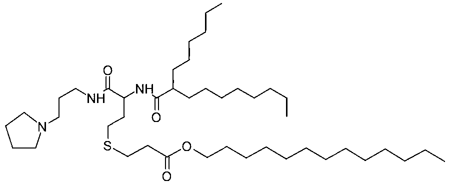 |
| VC-LC-0256 | 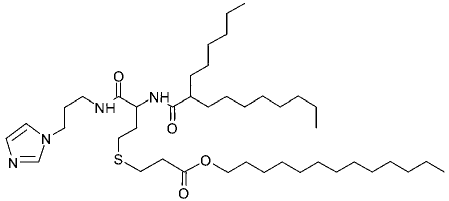 |
| VC-LC-0258 | 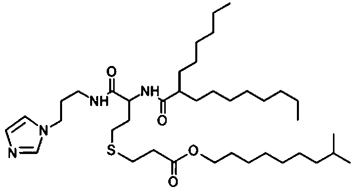 |
| VC-LC-0261 | 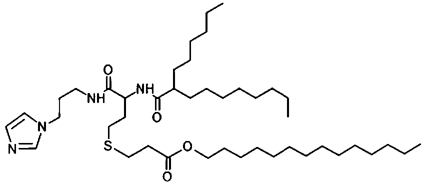 |
| VC-LC-0262 | 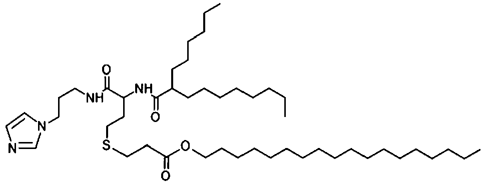 |
| VC-LC-0263 | 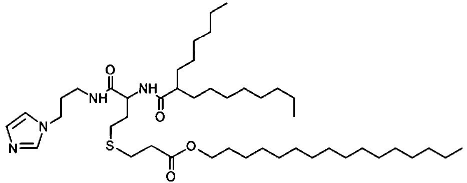 |
| VC-LC-0268 | 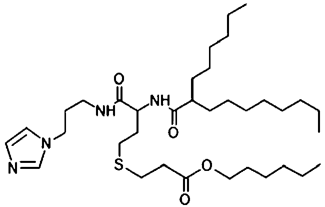 |
| VC-LC-0269 | 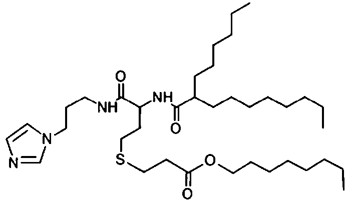 |
| VC-LC-0289 | 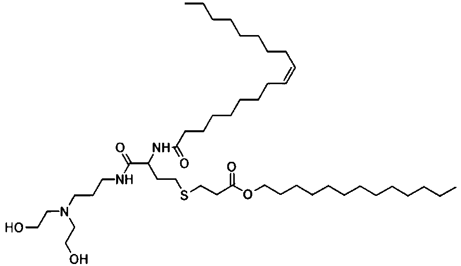 |
| VC-LC-0294 | 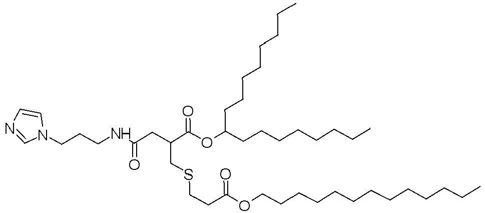 |
| VC-LC-0296 | 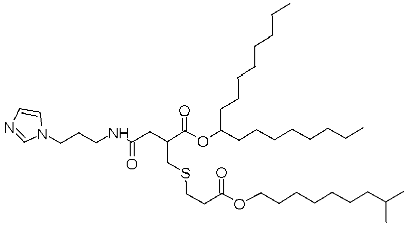 |
| VC-LC-0297 | 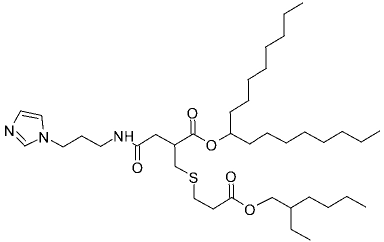 |
| VC-LC-0298 | 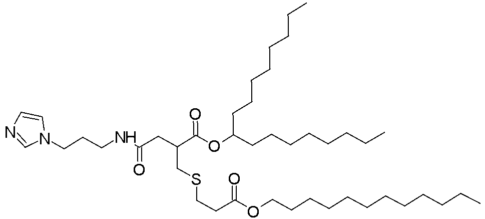 |
| VC-LC-0299 | 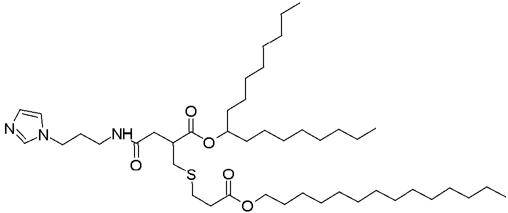 |
| VC-LC-0300 | 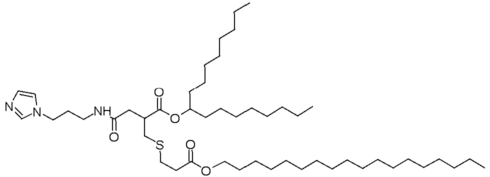 |
| VC-LC-0301 | 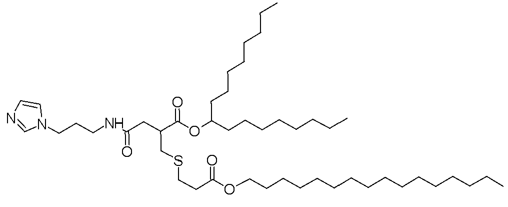 |
| VC-LC-0302 | 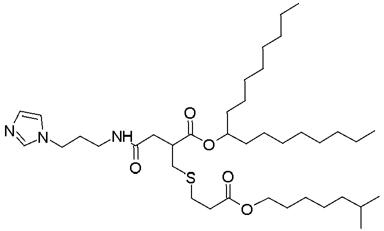 |
| VC-LC-0304 | 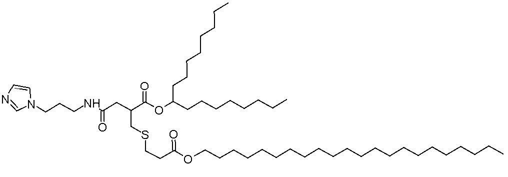 |
| VC-LC-0306 | 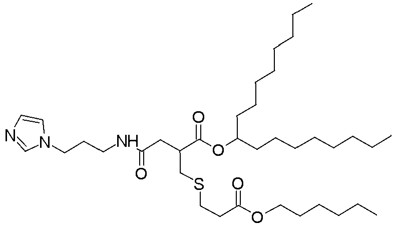 |
| VC-LC-0307 | 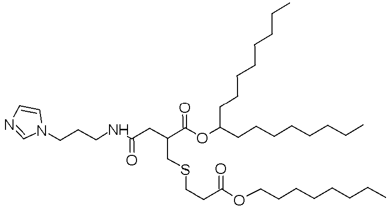 |
| VC-LC-0353 | 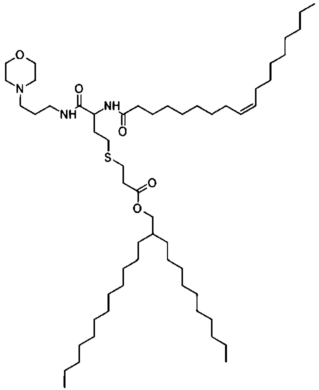 |
| VC-LC-0355 | 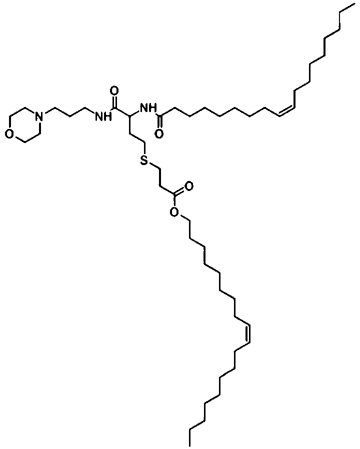 |
| VC-LC-0356 | 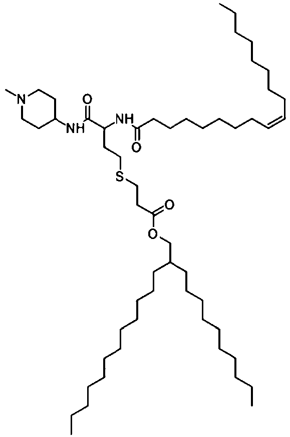 |
| VC-LC-0362 | 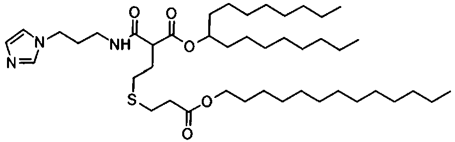 |
| VC-LC-0366 | 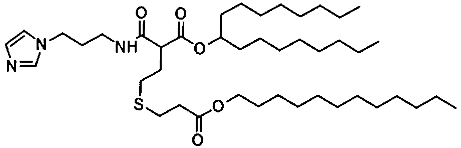 |
| VC-LC-0367 | 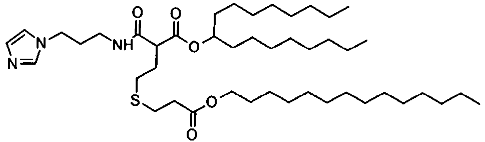 |
| VC-LC-0369 | 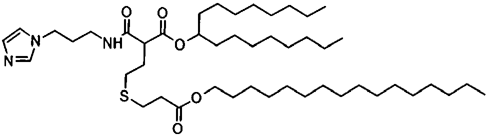 |
| VC-LC-0370 | 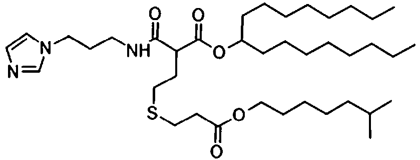 |
| VC-LC-0389 | 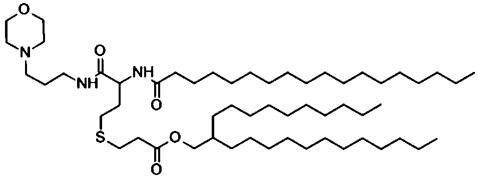 |
| VC-LC-0428 | 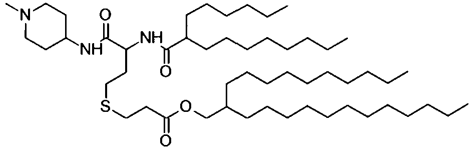 |
| VC-LC-0429 | 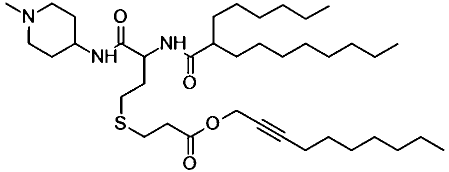 |
| VC-LC-0430 | 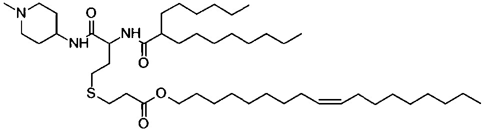 |
| VC-LC-0431 | 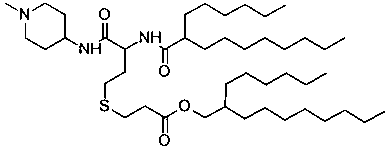 |
| VC-LC-0439 | 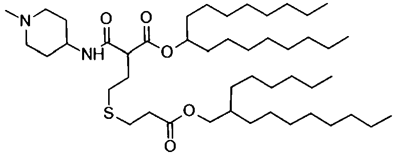 |
| VC-LC-0440 | 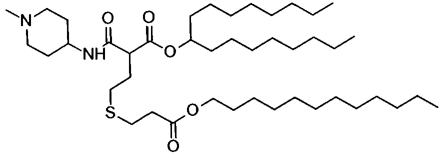 |
| VC-LC-0441 | 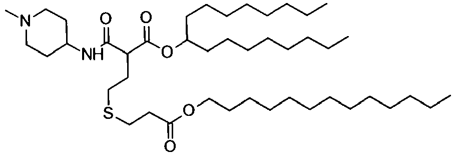 |
| VC-LC-0442 | 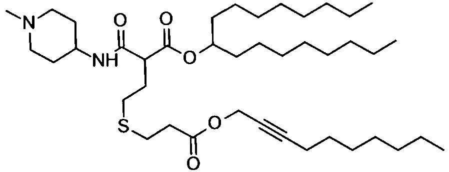 |
| VC-LC-0443 | 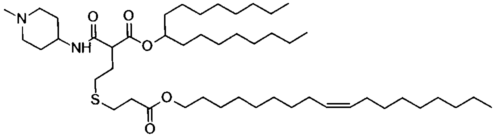 |
| VC-LC-0444 | 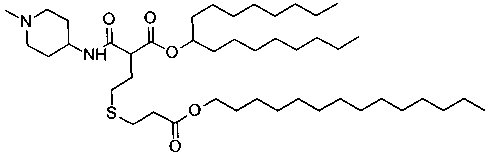 |
| VC-LC-0473 | 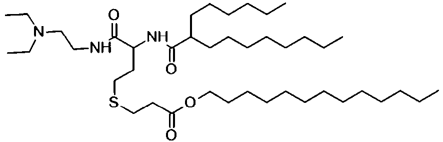 |
| VC-LC-0474 | 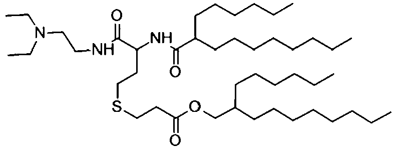 |
| VC-LC-0475 | 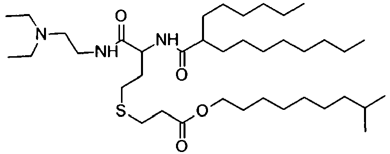 |
| VC-LC-0477 | 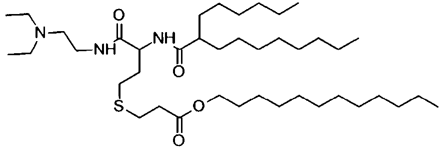 |
| VC-LC-0478 | 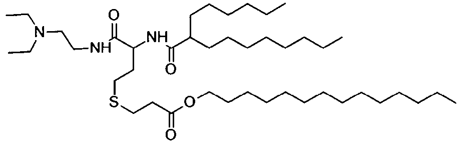 |
| VC-LC-0480 | 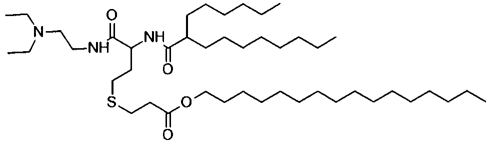 |
| VC-LC-0487 | 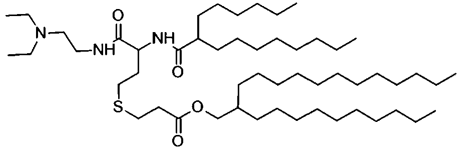 |
| VC-LC-0488 | 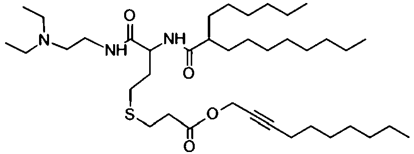 |
| VC-LC-0489 | 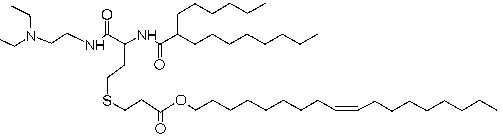 |
| VC-LC-0490 | 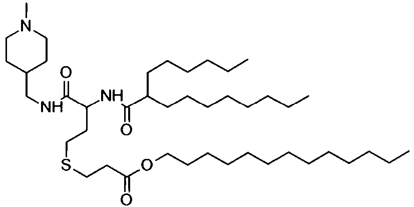 |
| VC-LC-0491 | 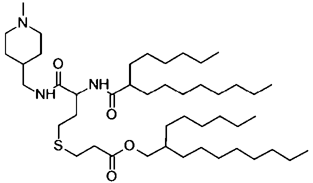 |
| VC-LC-0492 | 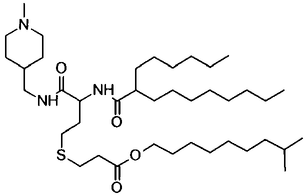 |
| VC-LC-0504 | 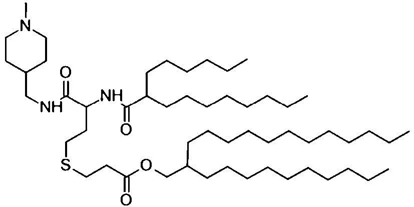 |
| VC-LC-0505 | 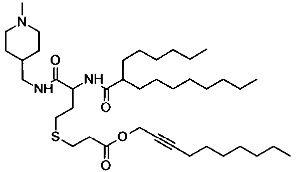 |
| VC-LC-0507 | 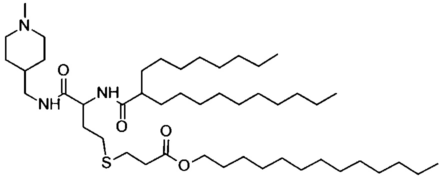 |
| VC-LC-0508 | 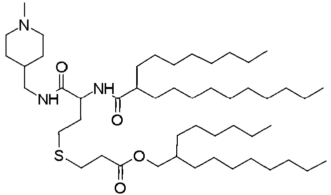 |
| VC-LC-0509 | 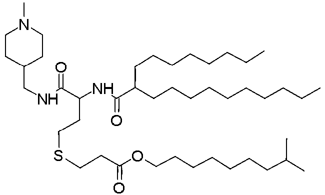 |
| VC-LC-0510 | 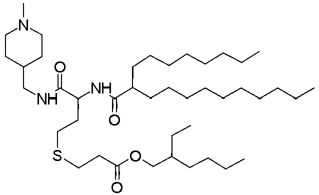 |
| VC-LC-0515 | 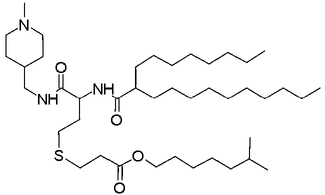 |
| VC-LC-0521 | 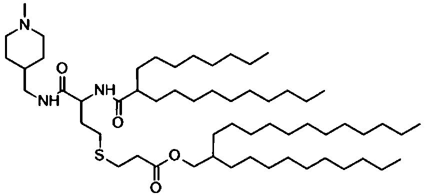 |
| VC-LC-0524 | 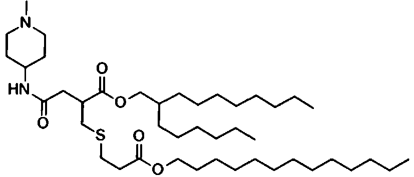 |
| VC-LC-0525 | 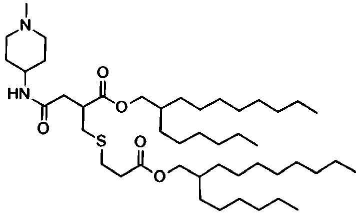 |
| VC-LC-0531 | 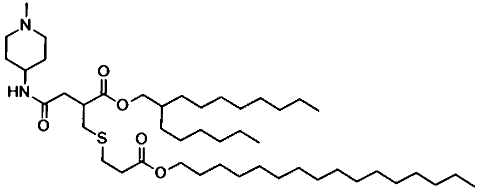 |
| VC-LC-0539 | 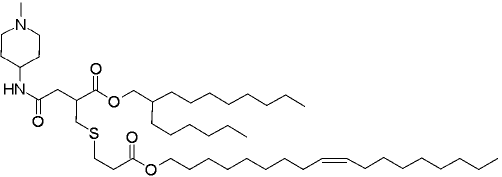 |
| VC-LC-0540 | 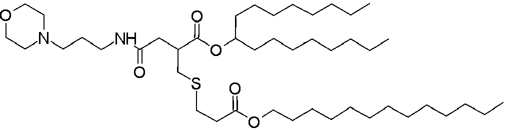 |
| VC-LC-0541 | 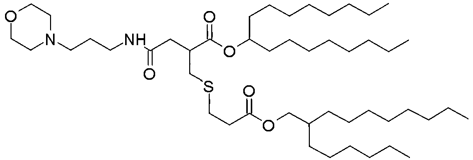 |
| VC-LC-0542 | 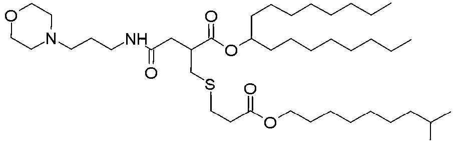 |
| VC-LC-0543 | 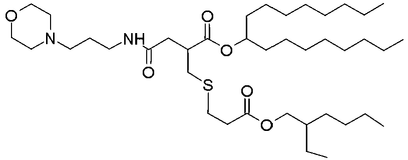 |
| VC-LC-0544 | 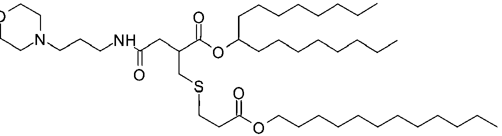 |
| VC-LC-0546 | 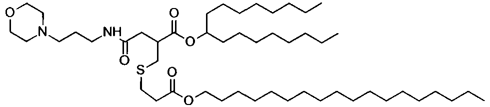 |
| VC-LC-0547 | 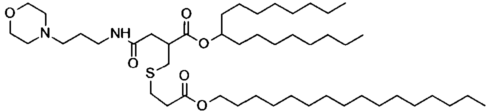 |
| VC-LC-0548 | 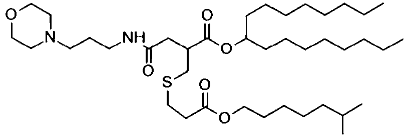 |
| VC-LC-0549 | 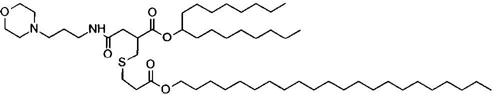 |
| VC-LC-0550 | 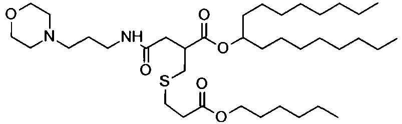 |
| VC-LC-0551 | 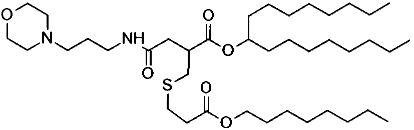 |
| VC-LC-0553 | 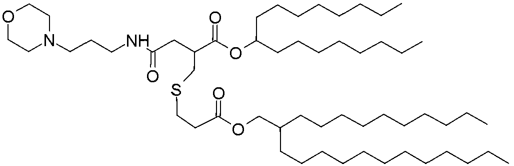 |
| VC-LC-0554 | 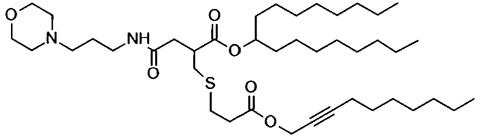 |
| VC-LC-0556 | 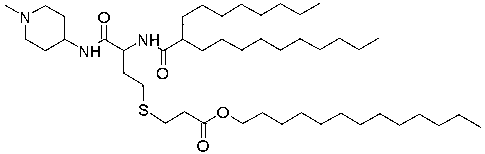 |
| VC-LC-0557 | 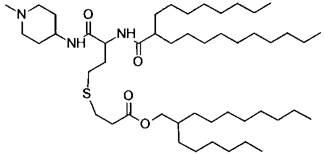 |
| VC-LC-0558 | 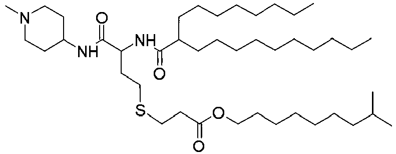 |
| VC-LC-0559 | 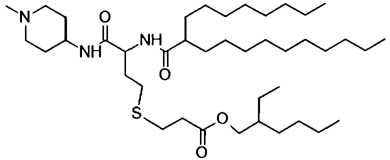 |
| VC-LC-0606 | 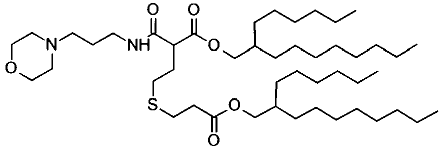 |
| VC-LC-0607 | 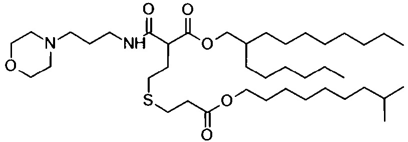 |
| VC-LC-0608 | 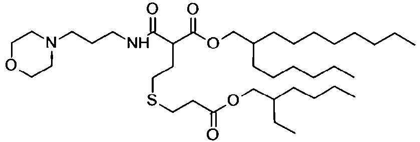 |
| VC-LC-0609 | 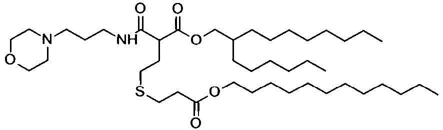 |
| VC-LC-0610 | 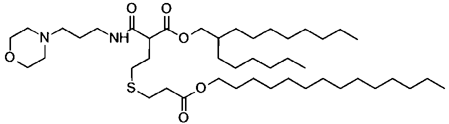 |
oder ein pharmazeutisch akzeptables Salz davon, oder ein Stereoisomer von einem von ihnen.

7. Ein Lipidnanopartikel umfassend ein ionisierbares Lipid der Formel (I): 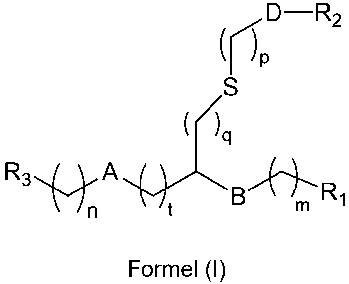
oder ein pharmazeutisch akzeptables Salz davon, oder ein Stereoisomer von einem von ihnen, wobei
A 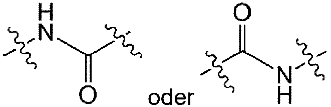 ist
B 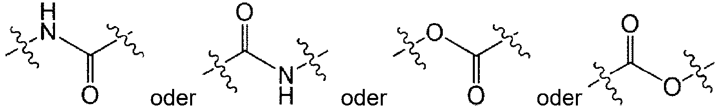 ist
D 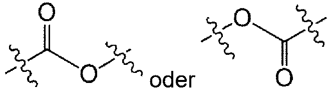 ist
n, m und p unabhängig voneinander 0, 1, 2, 3, 4, 5 oder 6 sind;
q ausgewählt aus 1 und 2 ist,
t ausgewählt aus 0 und 1 ist,
und wobei q + t = 2 ist;
R₁ und R₂ unabhängig voneinander ein lineares oder verzweigtes C1-C30-Alkyl, C2-C30-Alkenyl, C2-C30-Alkinyl sind, wobei R1 und R2 wahlweise mit einem oder mehreren Substituenten substituiert sind, die aus der Gruppe ausgewählt sind, die aus -OH, - COOR₄ und (-C(=O)SR₄) besteht, wobei R₄ = C1-C6-Alkyl, C2-C6-Alkenyl oder C2-C6-Alkinyl ist,
und wobei
R₃ ein Heterocyclus ist, der mindestens ein N-Atom umfasst, oder ersatzweise R₃ ist: 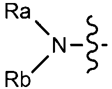
wobei Ra und Rb unabhängig voneinander lineares oder verzweigtes C1-C6-Alkyl sind, das wahlweise mit einer Hydroxylgruppe substituiert ist.

8. Das Lipidnanopartikel nach Anspruch 7, ferner umfassend:
i) ein nicht-kationisches Lipid;
wahlweise, wobei das nicht-kationische Lipid ausgewählt ist aus der Gruppe bestehend aus: 1,2-Dilinoleoyl-sn-glycero-3-phosphocholin (DLPC), 1,2-Dimyristoyl-sn-glycerophosphocholin (DMPC), 1,2-Dioleoyl-sn-glycero-3-phosphocholin (DOPC), 1-Stearoyl-2-oleoyl-*sn*-glycero-3-phosphocholin (SOPC), 1,2-Dipalmitoyl-sn-glycero-3-phosphocholin (DPPC), 1,2-Distearoyl-sn-glycero-3-phosphocholin (DSPC), 1,2-Diundecanoyl-snglycero-phosphocholin (DUPC), 1-Palmitoyl-2-oleoyl-sn-glycero-3-phosphocholin (POPC), 1,2-Di-O-octadecenyl-sn-glycero-3-phosphocholin (18:0 Diether PC), 1-Oleoyl-2-cholesterylhemisuccinoyl-sn-glycero-3-phosphocholin (OChemsPC), 1-Hexadecyl-sn-glycero-3-phosphocholin (C16 Lyso PC), 1,2-Dilinolenoyl-sn-glycero-3-phosphocholin, 1,2-Diarachidonoyl-sn-glycero-3-phosphocholin, 1,2-Didocosahexaenoyl-sn-glycero-3-phosphocholin, 1,2-Dioleoyl-sn-glycero-3-phosphoethanolamin (DOPE), 1,2-Diphytanoyl-sn-glycero-3-phosphoethanolamin (ME 16,0 PE), 1,2-Distearoyl-sn-glycero-3-phosphoethanolamin, 1,2-Dilinoleoyl-sn-glycero-3-phosphoethanolamin, 1,2-Dilinolenoyl-sn-glycero-3-phosphoethanolamin, 1,2-Diarachidonoyl-sn-glycero-3-phosphoethanolamin, 1,2-Didocosahexaenoyl-sn-glycero-3-phosphoethanolamin, 1,2-Dioleoyl-sn-glycero-3-phospho-rac-(1-glycerol)-Natriumsalz (DOPG), Sphingomyelin und Mischungen davon;
wahlweise, wobei das nicht-kationische Lipid DOPE ist; und/oder wobei das nicht-kationische Lipid DSPC ist;
ii) ein Sterol;
wahlweise, wobei das Sterol ausgewählt ist aus der Gruppe bestehend aus Cholesterin, Fecosterol, Sitosterol, Ergosterol, Campesterol, Stigmasterol, Brassicasterol, Tomatidin, Ursolsäure, Alpha-Tocopherol und Mischungen davon;
ferner wahlweise, wobei das Sterol Cholesterin ist; und
iii) ein PEG-modifiziertes Lipid;
wahlweise, wobei das PEG-modifizierte Lipid ausgewählt ist aus der Gruppe bestehend aus einem PEG-modifizierten Phosphatidylethanolamin, einer PEG-modifizierten Phosphatidsäure, einem PEG-modifizierten Phosphatidylcholin, einem PEG-modifizierten Ceramid, einem PEG-modifizierten Dialkylamin, einem PEG-modifizierten Diacylglycerin, einem PEG-modifizierten Dialkylglycerin und Mischungen davon.

9. Das Lipidnanopartikel nach einem der Ansprüche 7 bis 8, ferner umfassend ein nicht-kationisches Lipid, ein Sterol und ein PEG-modifiziertes Lipid, wobei das ionisierbare Lipid in einer Menge von 25 bis 60 Mol-% vorliegt, das PEG-modifizierte Lipid in einer Menge von 0,1 bis 10 Mol-% vorliegt; das nicht-kationische Lipid in einer Menge von 10 bis 45 Mol-% vorliegt; und das Sterol in einer Menge von 10 bis 40 Mol-% vorliegt.

10. Ein Lipidnanopartikel nach einem der Ansprüche 7 bis 9, ferner umfassend einen pharmazeutischen Wirkstoff.

11. Das Lipidnanopartikel nach Anspruch 10, wobei der pharmazeutische Wirkstoff ausgewählt ist aus der Gruppe bestehend aus einem Polynukleotid, einem DNA-Konstrukt, das einen Promotor umfasst, der operativ mit einer Sequenz verbunden ist, die ein Polynukleotid kodiert, und einem Expressionsvektor, der ein DNA-Konstrukt umfasst, das einen Promotor umfasst, der operativ mit einer Sequenz verbunden ist, die ein Polynukleotid kodiert.

12. Das Lipidnanopartikel nach Anspruch 11, wobei das Polynukleotid eine natürliche oder eine künstliche Desoxyribonukleinsäure (DNA) oder eine natürliche oder künstliche Ribonukleinsäure (RNA) ist.

13. Eine pharmazeutische Zusammensetzung umfassend das Lipidnanopartikel wie in einem der Ansprüche 10 bis 12 definiert und einen pharmazeutischen Wirkstoff oder Träger.

14. Ein Lipidnanopartikel wie in einem der Ansprüche 10 bis 12 definiert oder eine pharmazeutische Zusammensetzung wie in Anspruch 13 definiert zur Verwendung in einem Verfahren zur Behandlung einer Krankheit oder Störung bei einem Patienten, der dies benötigt, wobei das Verfahren die Verabreichung einer therapeutisch wirksamen Menge der Nanopartikelzusammensetzung oder der pharmazeutischen Zusammensetzung an den Patienten umfasst.

15. Ein Lipidnanopartikel von einem der Ansprüche 7 bis 9, wie hierin definiert, zur Verwendung als Verkapselungsmittel.

## Revendications

1. Un lipide ionisable de formule (I) : 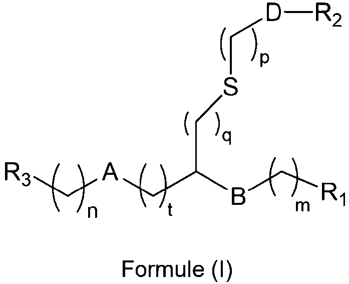
ou un sel pharmaceutiquement acceptable de celui-ci, ou un stéréoisomère de l'un quelconque d'entre eux, dans lequel
A est 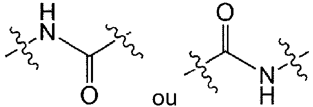
B est 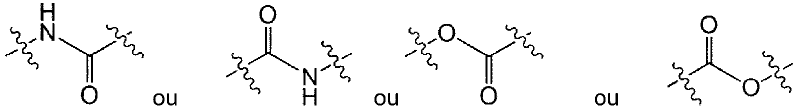
D est 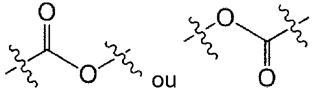
n, m et p sont indépendamment 0, 1, 2, 3, 4, 5 ou 6 ;
q est choisi entre 1 et 2,
t est choisi entre 0 et 1,
et où q + t = 2 ;
R₁ et R₂ sont indépendamment un alkyle en C1-C30 linéaire ou ramifié, un alcényle en C2-C30, un alcynyle en C2-C30, où R1 et R2 sont facultativement substitués par un ou plusieurs substituants choisis dans le groupe constitué de -OH, -COOR₄ et (-C(=O)SR₄), où R₄ est un alkyle en C1-C6, un alcényle en C2-C6 ou un alcynyle en C2-C6,
et où
R₃ est un hétérocycle comprenant au moins un atome de N, ou en variante R₃ est : 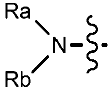
où Ra et Rb sont indépendamment un alkyle en C1-C6 linéaire ou ramifié, facultativement substitué par un groupe hydroxyle ;
à condition que les composés S-(2-méthoxy-2-oxoéthyl)-N-(pyridin-2-ylcarbonyl)-homocystéinate de méthyle 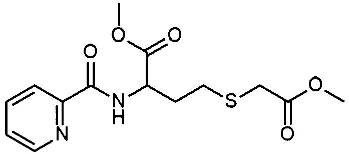 et
N-[(4-chloropyridin-2-yl)carbonyl]-S-(2-méthoxy-2-oxoéthyl)homocystéinate de méthyle 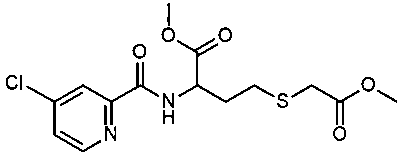 soient exclus.

2. Le lipide ionisable de la revendication 1, dans lequel R₃ est un cycle à 5 chaînons ou un cycle à 6 chaînons comprenant un atome de N et, facultativement, un second hétéroatome choisi de préférence parmi N et O.

3. Le lipide ionisable de l'une quelconque des revendications 1 à 2, dans lequel R3 est choisi parmi les structures suivantes :

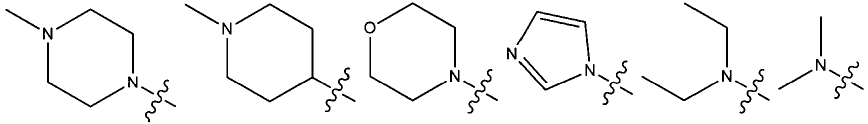

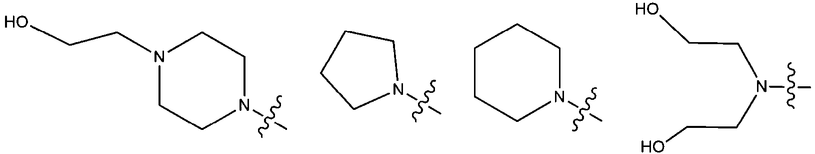

4. Le lipide ionisable selon l'une quelconque des revendications précédentes, dans lequel n est choisi parmi 0, 1, 2 ou 3 ; p est choisi parmi 0, 1, 2 ou 3 ; m est choisi parmi 0, 1, 2 ou 3 ; test 0 ou 1 ; q est 1 ou 2, et t + q = 2.

5. Le composé selon la revendication 4, dans lequel n est 2 ou 3, m = t = 0, p = q = 2,
A=B= 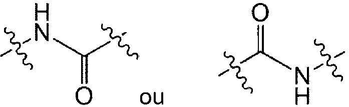 où
R₁ est un alkyle ramifié en C10-C20 ;
R₂ est un alkyle linéaire ou ramifié en C2-C30 ou un alcényle ou alcynyle linéaire en C6-C24 ; et
R₃ est : 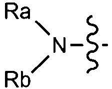
dans laquelle Ra = Rb = alkyle en C1-C4 ;
ou, en variante, où n = 2, et R₂ est un alkyle linéaire ou ramifié en C2-C30, et où R3 est : 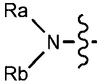
dans laquelle Ra = Rb = alkyle à terminaison alcool en C1-C4 ;
ou, en variante,
où n est 2 ou 3, m = t = 0, p = q = 2,
A est 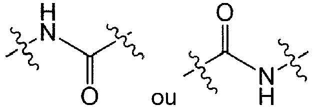
où R₁ est un alkyle ramifié en C10 à C20, et R₂ est un alkyle linéaire ou ramifié en C3-C30, et où R₃ est un cycle imidazole ;
ou, en variante,
où n = 3, m = t = 0, p = q = 2,
A est 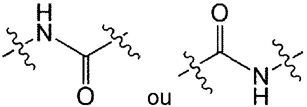
où R₁ est un alkyle ramifié en C10-C20, et R₂ est un alkyle linéaire ou ramifié en C4-C20, et où R₃ est un cycle pyrrolidine ;
ou, en variante,
où n = 2, m = t = 0, p = q = 2,
A est 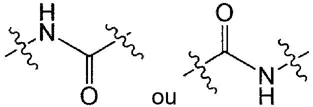
où R1 est un alkyle linéaire ou ramifié en C10-C20, et R₂ est un alkyle linéaire ou ramifié en C4-C20, et où R₃ est la N-4-méthylpipérazine ;
ou, en variante,
où n est choisi parmi 2 ou 3,
m = t = 0, et p = q = 2,
A=B= 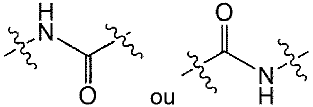
R₁ est alkyle, alcényle ou alcynyle linéaire ou ramifié en C10-C22 ; R₂ est alkyle linéaire ou ramifié en C3-C30 ou alcényle ou alcynyle linéaire en C3-C30 ; et où R₃ est un cycle morpholino ;
ou, en variante,
dans laquelle n est choisi parmi 2 ou 3, m = 0, p = 2,
q = t = 1,
A est 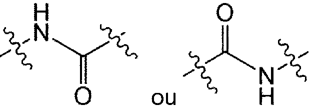
B = D, R₁ est un alkyle ramifié linéaire en C10-C22 ; R₂ est un alkyle linéaire ou ramifié en C3-C30 ou un alcényle ou alcynyle linéaire en C3-C30, et R₃ est un cycle morpholino.

6. Le lipide ionisable de la revendication 1, qui est un composé choisi dans le groupe constitué par :
| | |
|---|---|
| VC-LC-0099 | 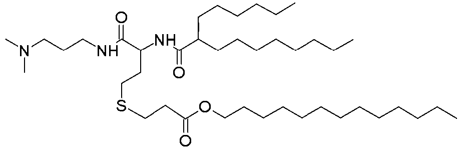 |
| VC-LC-0101 | 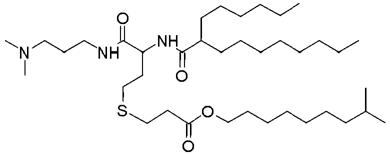 |
| VC-LC-0163 | 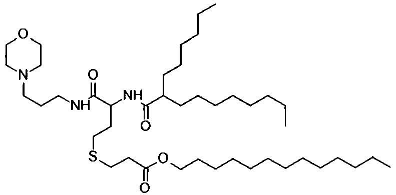 |
| VC-LC-0168 | 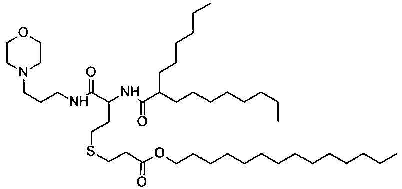 |
| VC-LC-0169 | 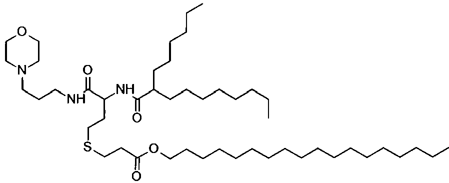 |
| VC-LC-0170 | 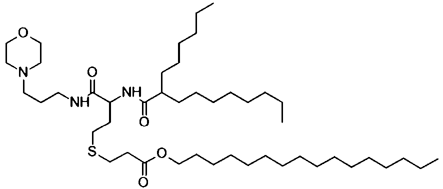 |
| VC-LC-0178 | 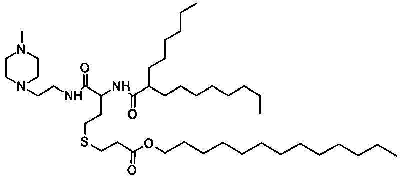 |
| VC-LC-0183 | 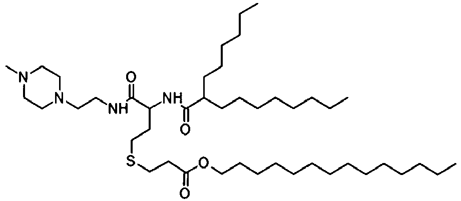 |
| VC-LC-0194 | 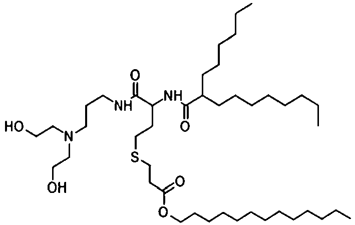 |
| VC-LC-0196 | 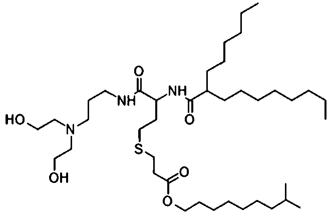 |
| VC-LC-0198 | 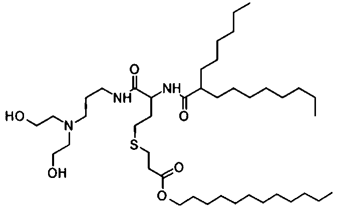 |
| VC-LC-0199 | 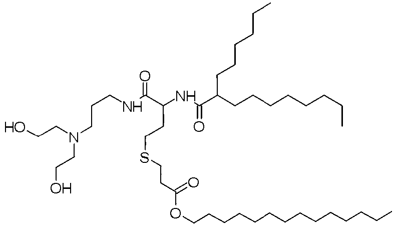 |
| VC-LC-0200 | 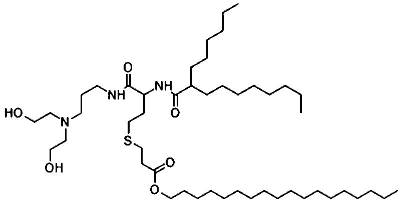 |
| VC-LC-0201 | 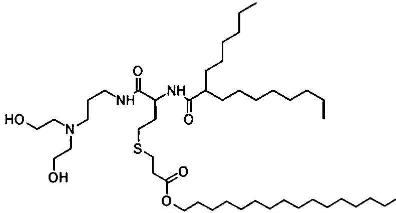 |
| VC-LC-0202 | 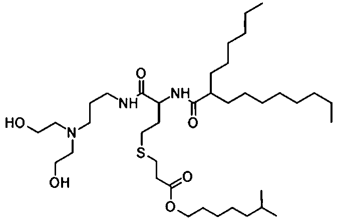 |
| VC-LC-0207 | 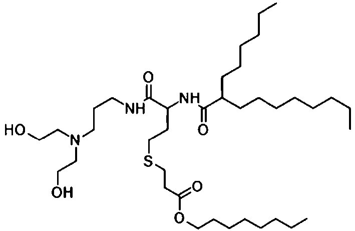 |
| VC-LC-0209 | 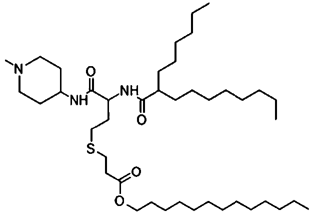 |
| VC-LC-0211 | 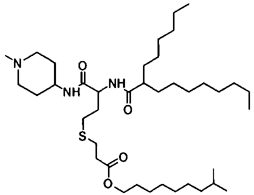 |
| VC-LC-0213 | 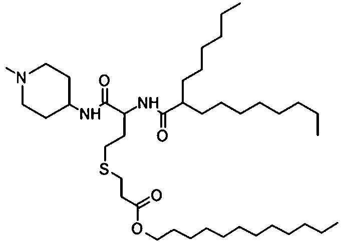 |
| VC-LC-0214 | 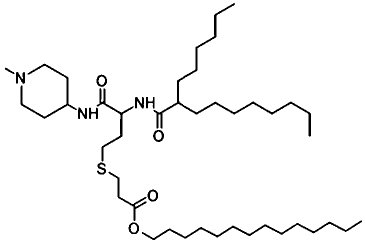 |
| VC-LC-0215 | 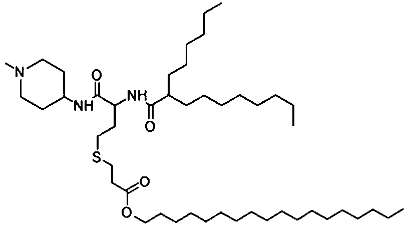 |
| VC-LC-0216 | 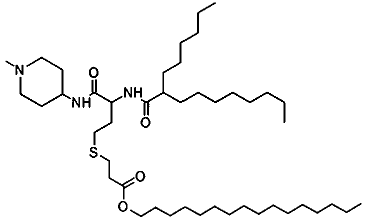 |
| VC-LC-0241 | 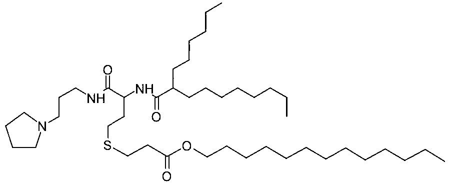 |
| VC-LC-0256 | 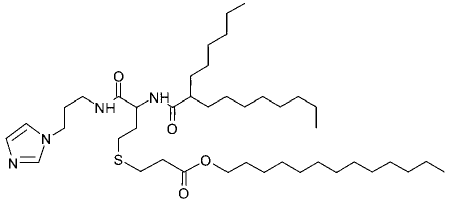 |
| VC-LC-0258 | 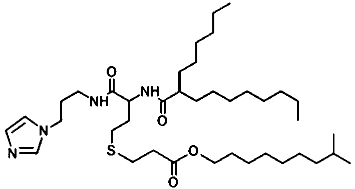 |
| VC-LC-0261 | 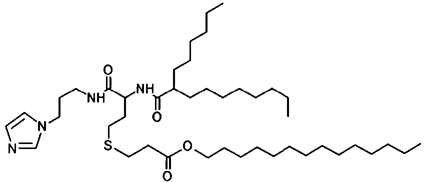 |
| VC-LC-0262 | 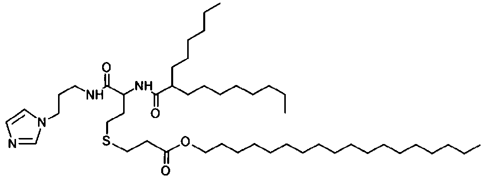 |
| VC-LC-0263 | 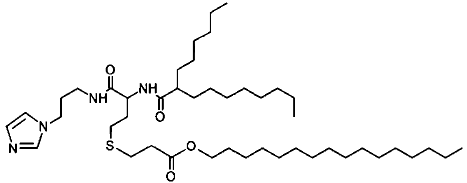 |
| VC-LC-0268 | 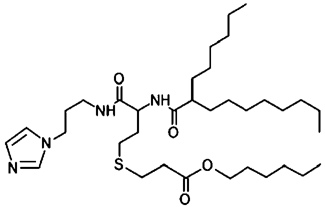 |
| VC-LC-0269 | 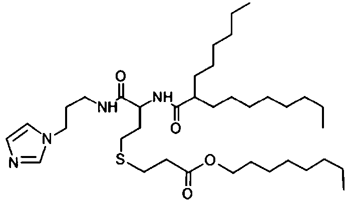 |
| VC-LC-0289 | 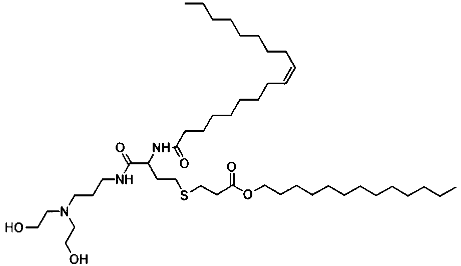 |
| VC-LC-0294 | 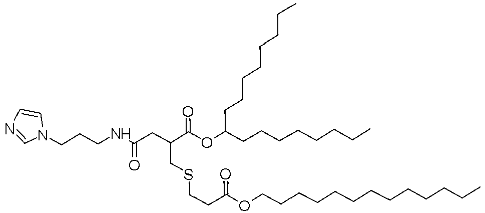 |
| VC-LC-0296 | 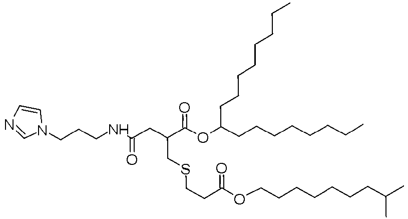 |
| VC-LC-0297 | 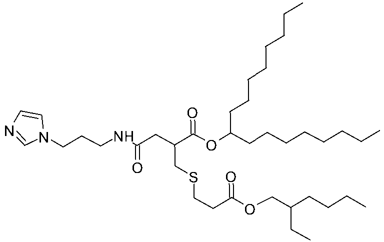 |
| VC-LC-0298 | 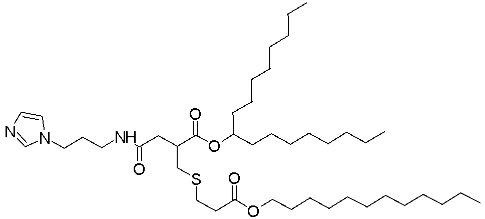 |
| VC-LC-0299 | 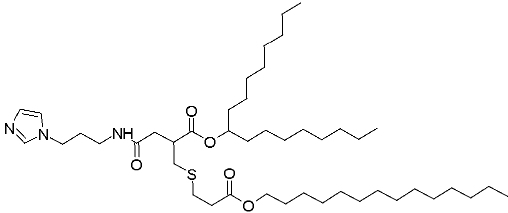 |
| VC-LC-0300 | 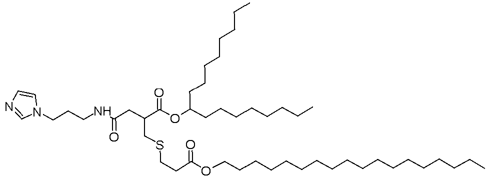 |
| VC-LC-0301 | 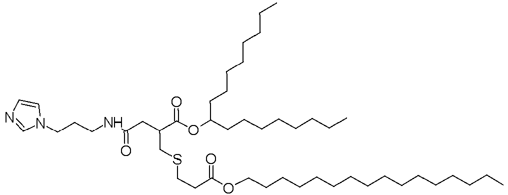 |
| VC-LC-0302 | 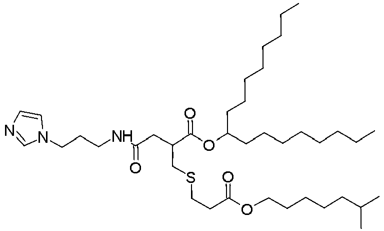 |
| VC-LC-0304 | 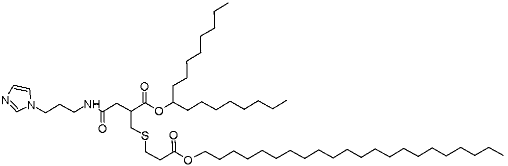 |
| VC-LC-0306 | 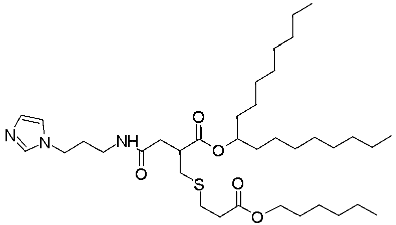 |
| VC-LC-0307 | 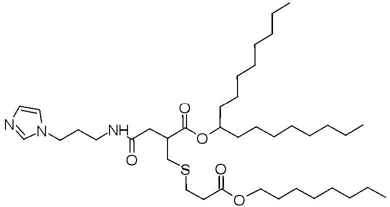 |
| VC-LC-0353 | 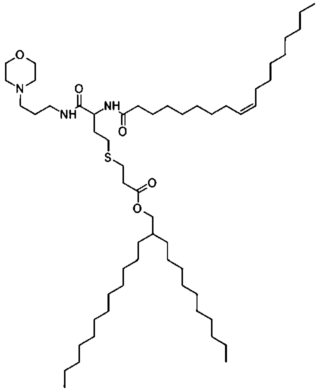 |
| VC-LC-0355 | 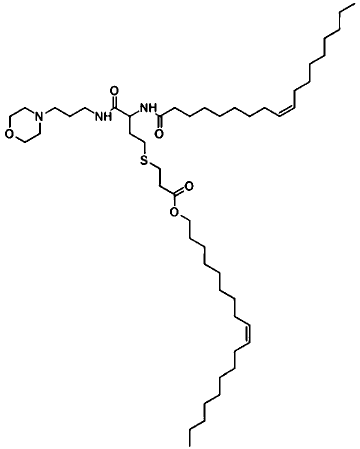 |
| VC-LC-0356 | 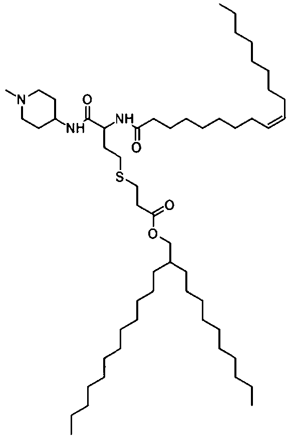 |
| VC-LC-0362 | 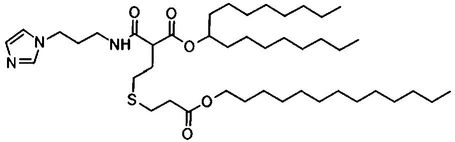 |
| VC-LC-0366 | 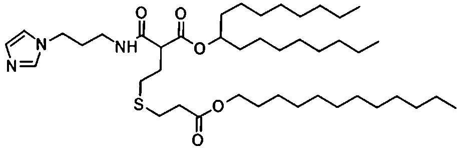 |
| VC-LC-0367 | 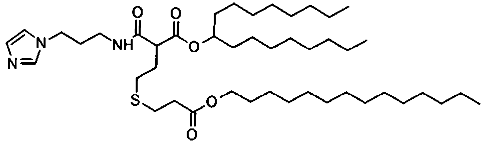 |
| VC-LC-0369 | 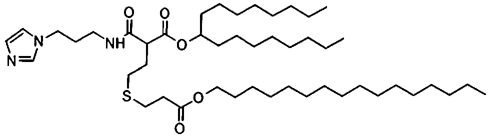 |
| VC-LC-0370 | 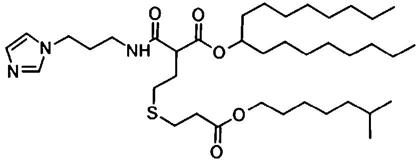 |
| VC-LC-0389 | 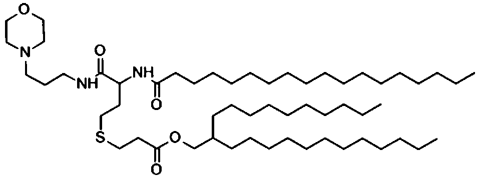 |
| VC-LC-0428 | 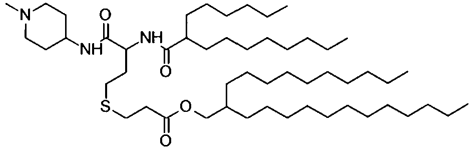 |
| VC-LC-0429 | 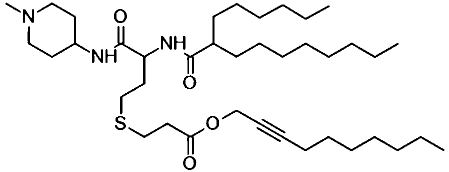 |
| VC-LC-0430 | 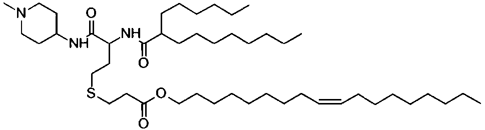 |
| VC-LC-0431 | 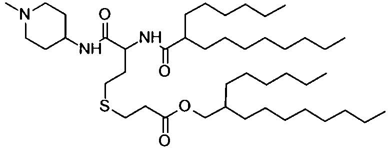 |
| VC-LC-0439 | 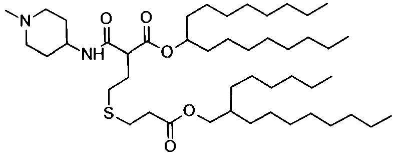 |
| VC-LC-0440 | 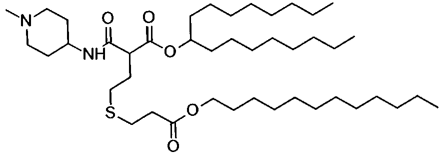 |
| VC-LC-0441 | 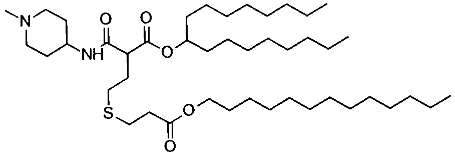 |
| VC-LC-0442 | 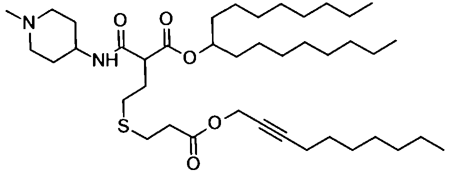 |
| VC-LC-0443 | 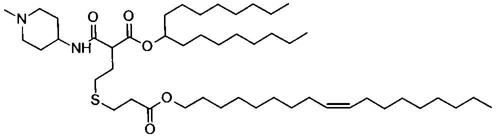 |
| VC-LC-0444 | 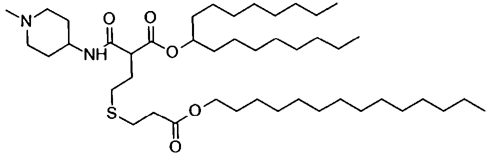 |
| VC-LC-0473 | 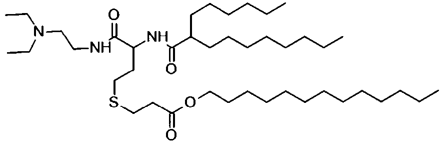 |
| VC-LC-0474 | 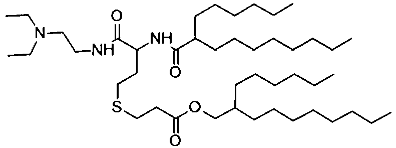 |
| VC-LC-0475 | 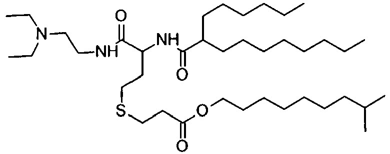 |
| VC-LC-0477 | 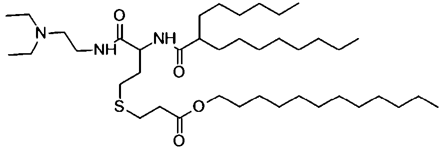 |
| VC-LC-0478 | 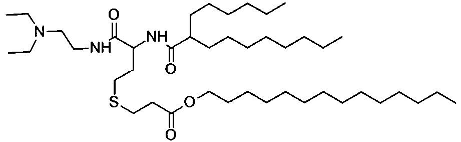 |
| VC-LC-0480 | 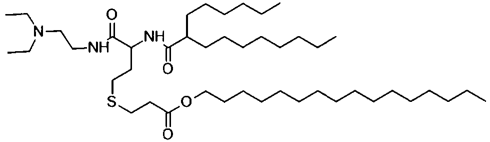 |
| VC-LC-0487 | 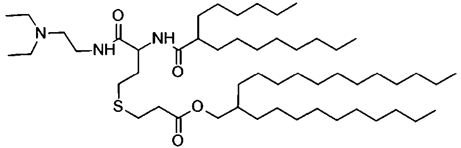 |
| VC-LC-0488 | 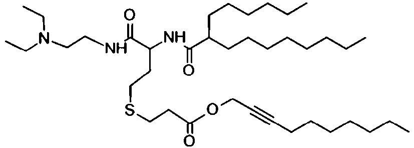 |
| VC-LC-0489 | 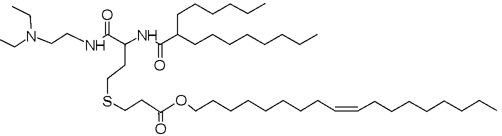 |
| VC-LC-0490 | 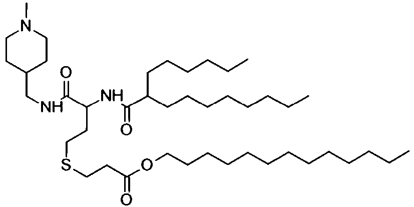 |
| VC-LC-0491 | 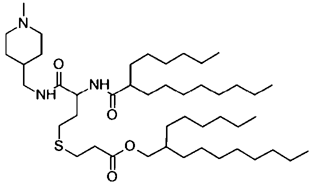 |
| VC-LC-0492 | 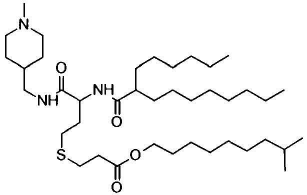 |
| VC-LC-0504 | 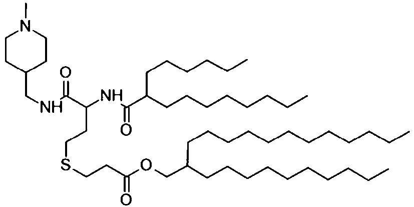 |
| VC-LC-0505 | 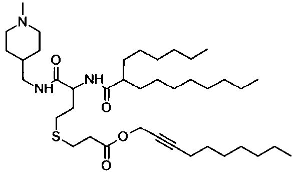 |
| VC-LC-0507 | 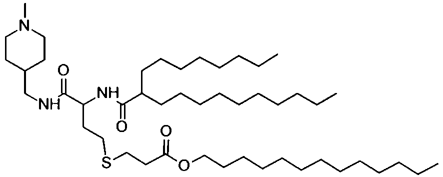 |
| VC-LC-0508 | 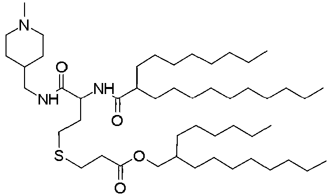 |
| VC-LC-0509 | 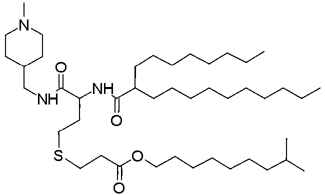 |
| VC-LC-0510 | 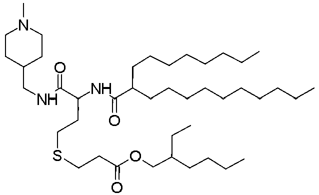 |
| VC-LC-0515 | 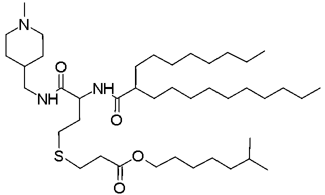 |
| VC-LC-0521 | 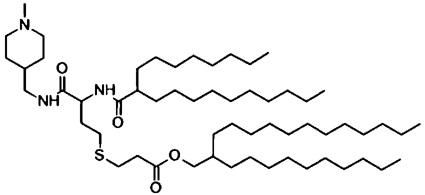 |
| VC-LC-0524 | 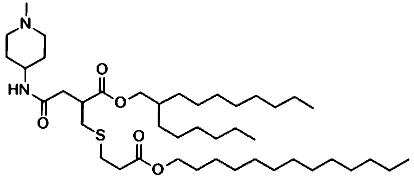 |
| VC-LC-0525 | 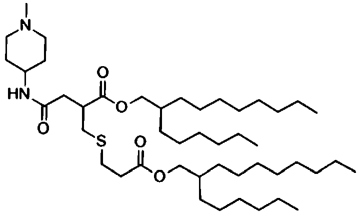 |
| VC-LC-0531 | 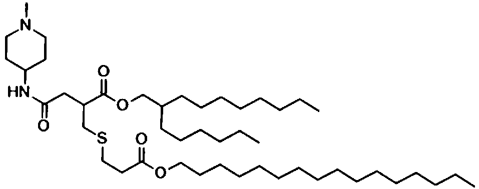 |
| VC-LC-0539 | 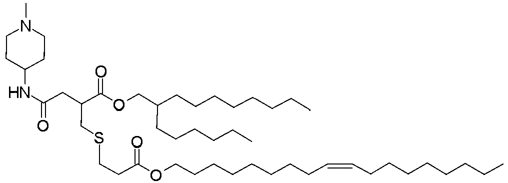 |
| VC-LC-0540 | 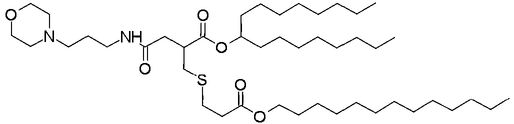 |
| VC-LC-0541 | 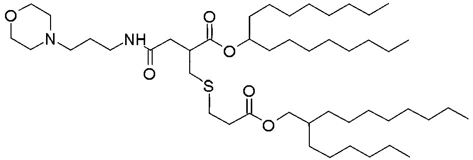 |
| VC-LC-0542 | 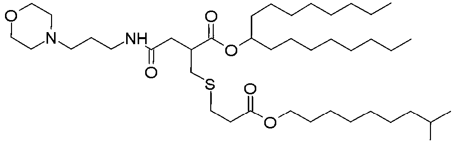 |
| VC-LC-0543 | 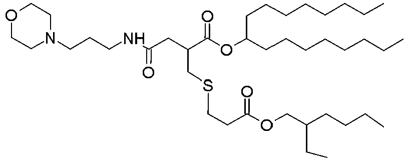 |
| VC-LC-0544 | 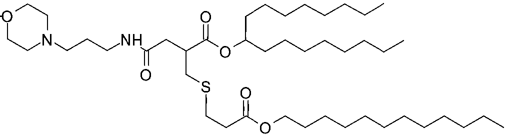 |
| VC-LC-0546 | 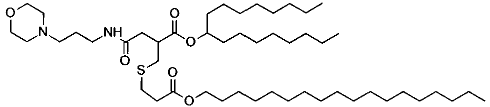 |
| VC-LC-0547 | 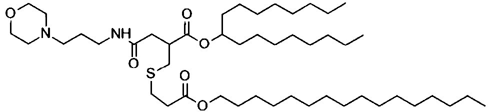 |
| VC-LC-0548 | 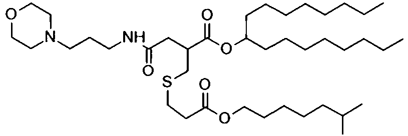 |
| VC-LC-0549 | 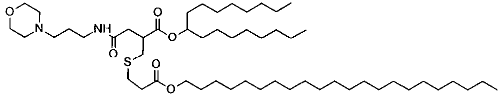 |
| VC-LC-0550 | 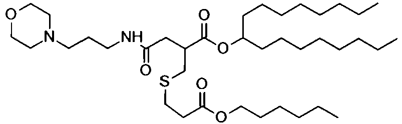 |
| VC-LC-0551 | 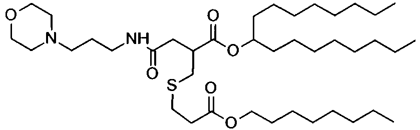 |
| VC-LC-0553 | 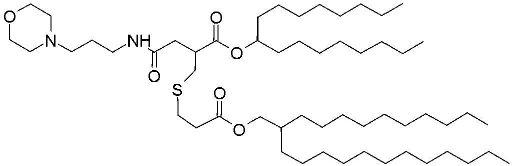 |
| VC-LC-0554 | 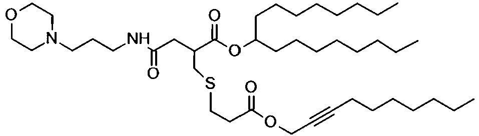 |
| VC-LC-0556 | 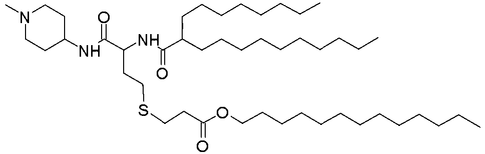 |
| VC-LC-0557 | 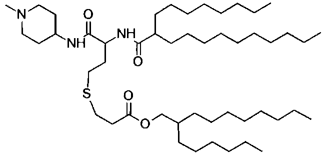 |
| VC-LC-0558 | 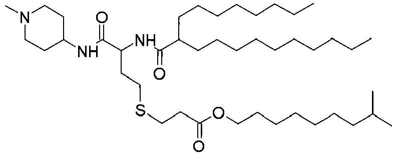 |
| VC-LC-0559 | 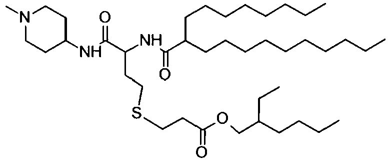 |
| VC-LC-0606 | 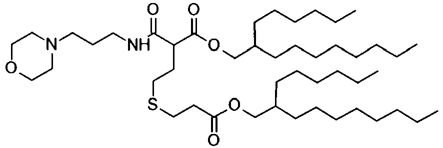 |
| VC-LC-0607 | 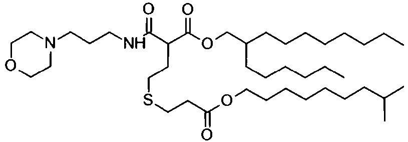 |
| VC-LC-0608 | 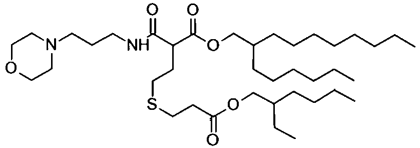 |
| VC-LC-0609 | 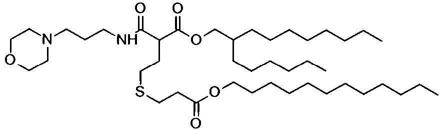 |
| VC-LC-0610 | 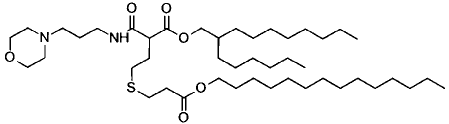 |
ou un sel pharmaceutiquement acceptable de celui-ci, ou un stéréoisomère de l'un quelconque d'entre eux.

7. Une nanoparticule lipidique comprenant un lipide ionisable de formule (I) : 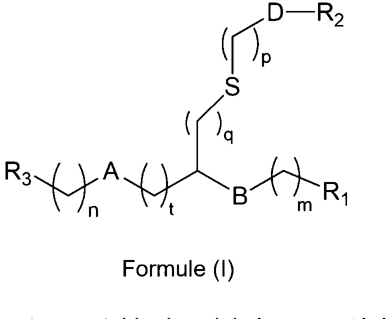
ou un sel pharmaceutiquement acceptable de celui-ci, ou un stéréoisomère de l'un quelconque d'entre eux, où
A est 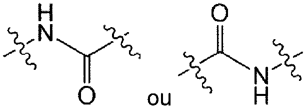
B est 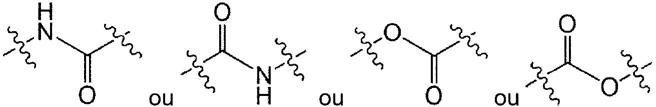
D est 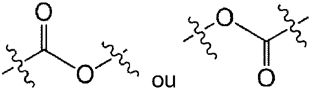
n, m et p sont indépendamment 0, 1, 2, 3, 4, 5 ou 6 ;
q est choisi entre 1 et 2,
t est choisi entre 0 et 1,
et où q + t = 2 ;
R₁ et R₂ sont indépendamment un alkyle en C1-C30 linéaire ou ramifié, un alcényle en C2-C30, un alcynyle en C2-C30, où R1 et R2 sont facultativement substitués par un ou
plusieurs substituants choisis dans le groupe constitué de -OH, -COOR₄ et (-C(=O)SR₄), où R₄ est un alkyle en C1-C6, un alcényle en C2-C6 ou un alcynyle en C2-C6,
et où
R₃ est un hétérocycle comprenant au moins un atome de N, ou en variante R₃ est : 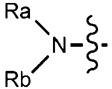
dans laquelle Ra et Rb sont indépendamment un alkyle en C1-C6 linéaire ou ramifié, facultativement substitué par un groupe hydroxyle.

8. La nanoparticule lipidique selon la revendication 7, comprenant en outre :
i) un lipide non cationique ;
facultativement, dans laquelle le lipide non cationique est choisi dans le groupe constitué par : 1,2-dilinoléoyl-sn-glycéro-3-phosphocholine (DLPC), 1,2-dimyristoyl-sn-glycéro-phosphocholine (DMPC), 1,2-dioléoyl-sn-glycéro-3-phosphocholine (DOPC), 1-stéaroyl-2-oléoyl-sn-glycéro-3-phosphocholine (SOPC), 1,2-dipalmitoyl-sn-glycéro-3-phosphocholine (DPPC), 1,2-distéaroyl-sn-glycéro-3-phosphocholine (DSPC), 1,2-diundécanoyl-sn-glycéro-phosphocholine (DUPC), 1-palmitoyl-2-oléoyl-sn-glycéro-3-phosphocholine (POPC), 1,2-di-O-octadécényl-sn-glycéro-3-phosphocholine (18:0 Diether PC), 1-oléoyl-2-cholestérylhémisuccinoyl-sn-glycéro-3-phosphocholine (OChemsPC), 1-hexadécyl-sn-glycéro-3-phosphocholine (C16 Lyso PC), 1,2-dilinolénoyl-sn-glycéro-3-phosphocholine, 1,2-diarachidonoyl-sn-glycéro-3-phosphocholine, 1,2-didocosahexaénoyl-sn-glycéro-3-phosphocholine, 1,2-dioléoyl-sn-glycéro-3-phosphoéthanolamine (DOPE), 1,2-diphytanoyl-sn-glycéro-3-phosphoéthanolamine (ME 16.0 PE), 1,2-distéaroyl-sn-glycéro-3-phosphoéthanolamine, 1,2-dilinoléoyl-sn-glycéro-3-phosphoéthanolamine, 1,2-dilinolénoyl-sn-glycéro-3-phosphoéthanolamine, 1,2-diarachidonoyl-sn-glycéro-3-phosphoéthanolamine, 1,2-didocosahexaénoyl-sn-glycéro-3-phosphoéthanolamine, sel de sodium de 1,2-dioléoyl-sn-glycéro-3-phospho-rac-(1-glycérol) (DOPG), sphingomyéline et leurs mélanges ;
facultativement, dans lequel le lipide non cationique est la DOPE ; et/ou dans lequel le lipide non cationique est la DSPC ;
ii) un stérol ;
facultativement, dans lequel le stérol est choisi dans le groupe constitué par le cholestérol, le fécostérol, le sitostérol, l'ergostérol, le campestérol, le stigmastérol, le brassicastérol, la tomatidine, l'acide ursolique, l'alpha-tocophérol et des mélanges de ceux-ci ;
en outre facultativement, dans lequel le stérol est le cholestérol ; et
iii) un lipide modifié par PEG ;
facultativement, dans lequel le lipide modifié par PEG est choisi dans le groupe constitué par une phosphatidyléthanolamine modifiée par PEG, un acide phosphatidique modifié par PEG, une phosphatidylcholine modifiée par PEG, un céramide modifié par PEG, une dialkylamine modifiée par PEG, un diacylglycérol modifié par PEG, un dialkylglycérol modifié par PEG et des mélanges de ceux-ci.

9. La nanoparticule lipidique selon l'une quelconque des revendications 7 à 8, comprenant en outre un lipide non cationique, un stérol et un lipide modifié par PEG, dans laquelle le lipide ionisable est en une quantité de 25 à 60 % en moles, le lipide modifié par PEG est en une quantité de 0,1 à 10 % en moles ; le lipide non cationique est en une quantité de 10 à 45 % en moles ; et le stérol est en une quantité de 10 à 40 % en moles.

10. Une nanoparticule lipidique selon l'une quelconque des revendications 7 à 9, comprenant en outre un agent pharmaceutiquement actif.

11. La nanoparticule lipidique selon la revendication 10, dans laquelle l'agent pharmaceutiquement actif est choisi dans le groupe constitué par un polynucléotide, une construction d'ADN comprenant un promoteur lié de manière opérationnelle à une séquence codant pour un polynucléotide, et un vecteur d'expression comprenant une construction d'ADN comprenant un promoteur lié de manière opérationnelle à une séquence codant pour un polynucléotide.

12. La nanoparticule lipidique selon la revendication 11, dans laquelle le polynucléotide est un acide désoxyribonucléique (ADN) naturel ou artificiel ou un acide ribonucléique (ARN) naturel ou artificiel.

13. Une composition pharmaceutique comprenant la nanoparticule lipidique telle que définie dans l'une quelconque des revendications 10 à 12 et un excipient ou un véhicule pharmaceutiquement acceptable.

14. Une nanoparticule lipidique telle que définie dans l'une quelconque des revendications 10 à 12, ou une composition pharmaceutique telle que définie dans la revendication 13, pour une utilisation dans un procédé de traitement d'une maladie ou d'un trouble chez un sujet en ayant besoin, le procédé comprenant l'administration au sujet d'une quantité thérapeutiquement efficace de la composition nanoparticulaire ou de la composition pharmaceutique.

15. Une nanoparticule lipidique de l'une quelconque des revendications 7 à 9 telle que définie ici pour une utilisation en tant qu'agent d'encapsulation.
